(19) Europäisches Patentamt
European Patent Office
Office européen des brevets

(11) Publication number: 0 475 206 A2

(12) EUROPEAN PATENT APPLICATION

(21) Application number: 91114542.3

(22) Date of filing: 29.08.91

(51) Int. Cl.[5]: C07D 403/12, A61K 31/505, C07D 239/42, C07D 403/14, C07D 401/12, C07D 257/04, A61K 31/44, A61K 31/495

(30) Priority: 10.09.90 US 580400
15.08.91 US 744241

(43) Date of publication of application:
18.03.92 Bulletin 92/12

(84) Designated Contracting States:
AT BE CH DE DK ES FR GB GR IT LI LU NL SE

(71) Applicant: ABBOTT LABORATORIES
One Abbott Park Road
Abbott Park, Illinois 60064-3500(US)

(72) Inventor: Winn, Martin
1263 Carlisle Place
Deerfield, Illinois 60015(US)
Inventor: De, Biswanath
101 Annapolis Drive
Vernon Hills, Illinois 60061(US)
Inventor: Zydowsky, Thomas M.
2630 Delaney
Waukegan, Illinois 60087(US)
Inventor: Kerkman, Daniel J.
21 Cremin Drive
Lake Villa, Illinois 60046(US)
Inventor: De Bernardis, John F.
2500 Colony Avenue
Lindenhurst, Illinois 60046(US)
Inventor: Rosenberg, Saul H.
1110 Fairlawn Drive
Libertyville, Illinois 60048(US)
Inventor: Shiosaki, Kazumi
1021 Mayfair
Libertyville, Illinois 60048(US)
Inventor: Basha, Fatima Z.
41 East Heron Drive
Lake Forest, Illinois 60045(US)
Inventor: Tasker, Andrew S.
220 Dittmer Lane
Lindenhurst, Illinois 60046(US)
Inventor: Von Geldern, Thomas W.
4209 W. Solon Road
Richmond, Illinois 60071(US)
Inventor: Kester, Jeffrey A.
680 Sampling
Deerfield, Illinois 60015(US)
Inventor: Holleman, William H.
39124 Cedar Crest Drive
Lake Villa, Illinois 60046(US)

(74) Representative: Modiano, Guido et al
MODIANO, JOSIF, PISANTY & STAUB
Modiano & Associati Via Meravigli, 16
I-20123 Milano(IT)

(54) **Angiotensin II receptor antagonists.**

(57) Novel pyrimidine, pyridine, pyridazine, pyrazine and triazine compounds are provided of the formula (I).

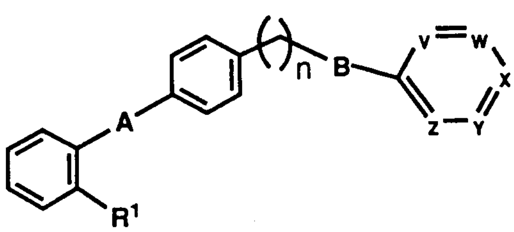

(I)

EP 0 475 206 A2

The compounds of the invention are useful for treating hypertension, edema, renal failure, congestive heart failure, psoriasis and glaucoma, and in the prevention and treatment of atherosclerosis or to treat gastrointestinal disorders associated with enhanced contractility and/or motility of intestinal smooth muscle or to treat contractile disorders of the uterus.

## TECHNICAL FIELD

This invention relates to compounds and compositions which block angiotensin II receptors, processes for making such compounds, synthetic intermediates employed in these processes and a method of treating hypertension, edema, renal failure, benign prostatic hypertrophy, diabetic nephropathy, Alzheimer's disease or congestive heart failure with such compounds. The present invention also relates to compositions and a method for treating glaucoma, and of preventing and treating atherosclerosis with such compounds.

## BACKGROUND OF THE INVENTION

Blood pressure is regulated by a multitude of interrelated factors involving neural, vascular and volume-related effects. The renin-angiotensin system (RAS) is one of the important blood pressure regulating systems.

The RAS functions as shown in the scheme below. Low renal perfusion pressure stimulates the juxtaglomerular cells of the kidney to produce the proteolytic enzyme renin. This enzyme acts on a circulating protein, angiotensinogen, cleaving off a decapeptide angiotensin I. Angiotensin I is then cleaved to the octapeptide angiotensin II by angiotensin converting enzyme (ACE). Angiotensin II is the most powerful pressor substance in the RAS. Angiotensin II binds to vascular smooth muscle receptors and induces vasoconstriction but has little or no stimulating action on the heart.

### Renin-Angiotensin System

Human Angiotensinogen: $H_2N$-Asp-Arg-Val-Tyr-Ile-His-Pro-Phe-His-Leu-Val-Ile-His-Protein

Renin

Angiotensin I: $H_2N$-Asp-Arg-Val-Tyr-Ile-His-Pro-Phe-His-Leu-OH

ACE

Angiotensin II: $H_2N$-Asp-Arg-Val-Tyr-Ile-His-Pro-Phe-OH

Aminopeptidase

Angiotensin III: $H_2N$-Arg-Val-Tyr-Ile-His-Pro-Phe-OH

Angiotensinases

Inactive Fragments

Inhibitors of two of the enzymes in the RAS, renin and ACE, have clinical efficacy in treating hypertension and congestive heart failure. Captopril and enalapril are both commercially available potent ACE inhibitors which are effective antihypertensive agents. ACE inhibitors, however, have reported side effects including dizziness, lightheadedness or fainting; skin rash and swelling of the face, mouth, hands or feet. The major reported side effect of ACE inhibitors is dry cough (*J. Hypertension,* **1989**, **7**:S308-309; D.M. Coulter and I.R. Edwards, *Br. Medical J.*, **1987**, *294*:1521-1523). Dipeptide renin inhibitors such as ABBOTT-64662 (enalkiren) are currently undergoing clinical evaluation. It has been demonstrated that enalkiren is effective in lowering systolic and diastolic blood pressure in patients with essential hypertension (P.W. Anderson, *et al.*, *Clinical Research*, **1989**, *37*:392A).

Angiotensin II receptor antagonists are known. Devynck and Meyer in "Advances in Pharmacology and Therapy, Volume 1 Receptors"; T. Jacobs, Ed, Pergamon Press, N.Y. (pp 279-289) give details of the

structure-activity relationships for some peptidic compounds. In particular, saralasin or [$Sar^1$,$Ala^8$] angiotensin II, was found to be a potent antagonist of the actions of angiotensin II. Saralasin, however, has several disadvantages. Because it is a peptide, saralasin has very poor oral bioavailability. The use of saralasin, therefore, is limited to administration to hospitalized patients by continuous intravenous infusion. Saralasin is also known to cause an initial increase in blood pressure after intravenous administration due to its activity as an angiotensin receptor agonist.

Non-peptide angiotensin II receptor antagonists are also known. For example, Herold, *et al.* , European Patent Application Number 424317 (published April 24, 1991) discloses 4-hydroxy substituted pyrimidine derivatives as angiotensin II receptor antagonists. Allen, *et al.*, European Patent Application Number 419048 (published March 27, 1991); Herold, *et al.* , European Patent Application Number 407342 (published January 9, 1991) and Herold, *et al.* , European Patent Application Number 435827 (published July 3, 1991) disclose pyrimidin-4-one derivatives as angiotensin II receptor antagonists.

## DISCLOSURE OF THE INVENTION

In accordance with the present invention there are provided angiotensin II receptor antagonists of formula (I):

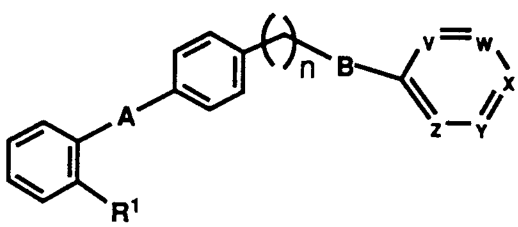

(I)

wherein n is 0 or 1;

A is selected from

(i) a covalent bond,

(ii) -O- and

(iii) -C(O)-;

B is selected from

(i) -N($R^4$)- wherein $R^4$ is hydrogen, lower alkyl, loweralkoxy-substituted lower alkyl, lower alkenyl, lower alkynyl, cycloalkyl or cycloalkylalkyl,

(ii) -O- and

(iii) -S-;

$R^1$ is selected from

(i) tetrazolyl,

(ii) $-COOR^5$ or $-CH_2COOR^5$ wherein $R^5$ is hydrogen or a carboxy-protecting group; and

(iii) $-NHS(O)_2R^6$ or $-CH_2NHS(O)_2R^6$ wherein $R^6$ is selected from lower alkyl and halo-substituted lower alkyl;

V, W, X, Y and Z are independently selected from N, -N(O)-, CH, $CR^2$ and $CR^3$, with the proviso that

(1) not more than one of V, W, X, Y and Z is $CR^2$,

(2) one of V, W, X, Y and Z is $CR^3$, and

(3) not more than three of V, W, X, Y and Z are N, wherein

$R^2$ is selected from

(i) lower alkyl,

(ii) halo,

(iii) halo-substituted loweralkyl,

(iv) lower alkylthio,

(v) loweralkoxy-substituted lower alkyl,

(vi) loweralkylthio-substituted lower alkyl,

(vii) arylalkyl,

(viii) $-OR^a$ wherein Ra is loweralkyl, halo-substituted loweralkyl or aryl and

(ix) $-NR^7R^8$ or $-CH_2NR^7R^8$ wherein $R^7$ and $R^8$ are independently selected from hydrogen and lower alkyl, or $R^7$ and $R^8$ taken together with the nitrogen atom to which they are attached form a 5- to 7-membered aliphatic heterocycle and

$R^3$ is selected from
(i) hydrogen,
(ii) loweralkyl,
(iii) halo-substituted loweralkyl,
(iv) -CN,
(v) $-NO_2$,
(vi) $-NH_2$,
(vii) -CHO,
(viii) tetrazolyl,
(ix) $-NHS(O)_2R^9$ or $-CH_2NHS(O)_2R^9$ or $-NHC(O)R^9$ or $-CH_2NHS(O)_2R9$ wherein $R^9$ is selected from lower alkyl and halo-substituted lower alkyl,
(x) $-COOR^{10}$ or $-CH_2COOR^{10}$ wherein $R^{10}$ is hydrogen or a carboxy- protecting group,
(xi) $-C(O)NR^{11}R^{12}$ or $-CH_2C(O)NR^{11}R^{12}$ or $-NHC(O)NR^{11}R^{12}$ or $-CH_2NHC(O)NR^{11}R^{12}$ wherein $R^{11}$ and $R^{12}$ are independently selected from hydrogen, lower alkyl, hydroxy, lower alkoxy, hydroxy- substituted lower alkyl, loweralkoxy-substituted lower alkyl and loweralkoxy-substituted loweralkoxy, or
$R^{11}$ and $R^{12}$ taken together with the nitrogen atom to which they are attached form a 5- to 7- membered aliphatic heterocycle;
(xii) $-CH_2OR^{13}$ wherein $R^{13}$ is selected from hydrogen, lower alkyl and $-C(O)R^{14}$ wherein $R^{14}$ is hydrogen, lower alkyl or aryl;
(xiii) $-CH_2NR^{15}R^{15*}$ wherein $R^{15}$ is selected from hydrogen, lower alkyl, $-C(O)R^{16}$, $-C(O)NR^{16}R^{16*}$ and $-S(O)_2R^{17}$ wherein
$R^{16}$ is selected from hydrogen, lower alkyl and aryl and
$R^{17}$ is selected from lower alkyl and halo-substituted lower alkyl and wherein $R^{15*}$ and $R^{16*}$ are independently selected from hydrogen, loweralkyl, hydroxy and lower alkoxy;
(xiv) $-SO_3H$ or $-CH_2SO_3H$ and
(xv) $-SO_2NR^{11}R^{12}$ or $-CH_2SO_2NR^{11}R^{12}$ wherein $R^{11}$ and $R^{12}$ are defined as above;
or a pharmaceutically acceptable salt or prodrug thereof.

In one preferred embodiment of the present invention represented by formula (I A), V and X are N, W is $CR^2$ and Z is $CR^3$.

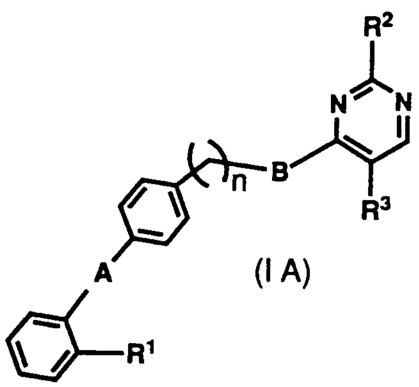

(I A)

In another preferred embodiment of the present invention represented by formula (I M), V and Z are N, W is $CR^2$ and Y is $CR^3$.

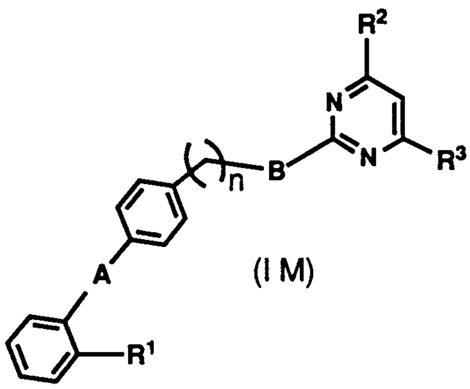

(I M)

In another preferred embodiment of the present invention represented by formula (IR), X is N, V is $CR^2$ and Z is $CR^3$.

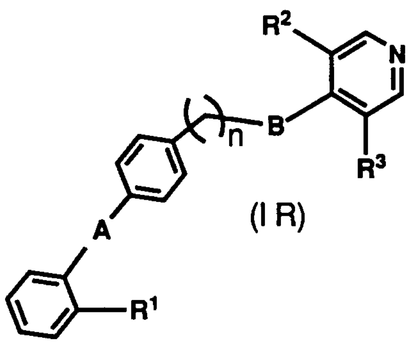

(I R)

In another preferred embodiment of the present invention represented by formula (I S), W and Z are N, X is $CR^2$ and V is $CR^3$.

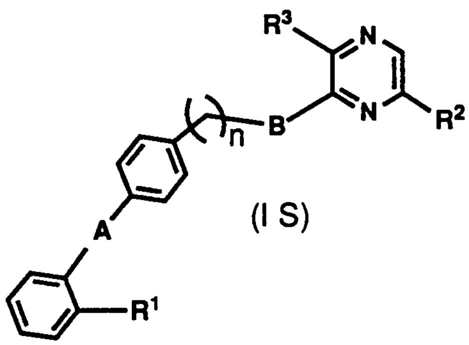

(I S)

In another preferred embodiment of the present invention represented by formula (I T), V and W are N, and Z is $CR^3$.

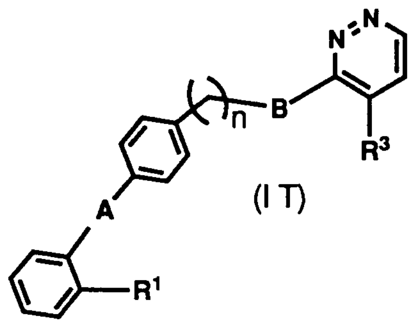

(I T)

In another preferred embodiment of the present invention represented by formula (I V), Z is N and V is $CR^3$.

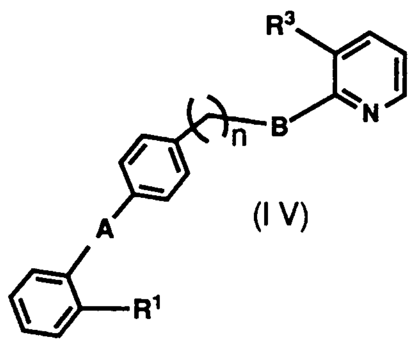

(I V)

In yet another preferred embodiment of the present invention represented by formula (I W), V is $CR^3$, W, X and Z are N and Y is $CR^2$.

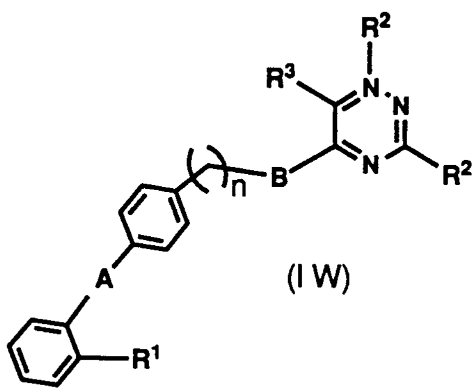

(I W)

More preferred compounds of the invention are compounds of the formula:

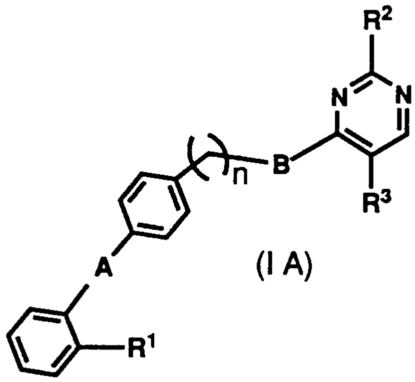

(I A)

wherein n is 1;
A is a covalent bond;
B is $-N(R^4)-$ wherein $R^4$ is defined as above;
$R^1$ is tetrazolyl;
$R^2$ hydrogen or $R^2$ is defined as above; and
$R^3$ is $-COOR^{10}$ wherein $R^{10}$ is hydrogen or a carboxy-protecting group or $-C(O)NR^{11}R^{12}$ wherein $R^{11}$ and $R^{12}$ are independently selected from hydrogen, lower alkyl, hydroxy, lower alkoxy, hydroxy-substituted lower alkyl, loweralkoxy-substituted lower alkyl and loweralkoxy-substituted loweralkoxy, or $R^{11}$ and $R^{12}$ taken together with the nitrogen atom to which they are attached form a 5- to 7- membered aliphatic heterocycle; or a pharmaceutically acceptable salt or prodrug thereof.

More preferred compounds of the invention also are compounds of the formula:

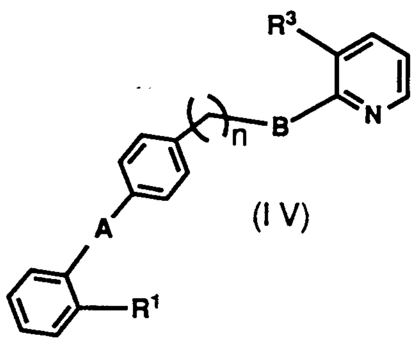

(I V)

wherein n is 1;
A is a covalent bond;
B is $-N(R^4)-$ wherein $R^4$ is defined as above;
$R^1$ is tetrazolyl; and
$R^3$ is $-COOR^{10}$ wherein $R^{10}$ is hydrogen or a carboxy-protecting group or $-C(O)NR^{11}R^{12}$ wherein $R^{11}$ and $R^{12}$ are independently selected from hydrogen, lower alkyl, hydroxy, lower alkoxy, hydroxy-substituted lower alkyl, loweralkoxy-substituted lower alkyl and loweralkoxy-substituted loweralkoxy, or $R^{11}$ and $R^{12}$ taken together with the nitrogen atom to which they are attached form a 5- to 7- membered aliphatic heterocycle; or a pharmaceutically acceptable salt or prodrug thereof.

The term "aliphatic heterocycle" as used herein refers to a saturated cyclic group containing 5 to 7 ring atoms and, in particular, at least 1 nitrogen atom in the ring and optionally 1 additional heteroatom selected from S, $S(O)_2$, O and N with the remaining ring atoms being carbon atoms. The ring may be substituted on a carbon atom or a heteroatom, for example, with a loweralkoxy or loweralkoxy-substituted loweralkoxy group. Representative aliphatic heterocycles include, pyrrolidine, piperidine, piperazine, morpholine, thiomorpholine and 4-methoxymethoxypiperidine and the like.

The term "aryl" as used herein refers to a $C_6$ monocyclic aromatic ring system or a $C_9$ or $C_{10}$ bicyclic carbocyclic ring system having one or more aromatic rings including, but not limited to, phenyl, naphthyl, tetrahydronaphthyl, indanyl, indenyl and the like. Aryl groups can be unsubstituted or substituted with one, two or three substituents independently selected from loweralkyl, halo-substituted loweralkyl, loweralkoxy,

loweralkylthio, loweralkoxycarbonyl, hydroxy, halo, mercapto, nitro, amino, loweralkylamino, carboxaldehyde, carboxy and carboxamide.

The term "arylalkyl" is used herein to mean a straight or branched chain radical of one to ten carbon atoms which is substituted with an aryl group as defined above. Representative arylalkyl groups include benzyl, phenylethyl groups, fluorobenzyl and fluorphenylethyl.

The term "alkanoyl" as used herein refers to R**C(O)- wherein R** is loweralkyl.

As used herein, the term "carboxy-protecting group" refers to a carboxy group which has been esterified with one of the commonly used carboxylic acid protecting ester groups employed to block or protect the carboxylic acid functionality while the reactions involving other functional sites of the compound are carried out. In addition, a carboxy-protecting group can be used as a prodrug whereby the carboxy-protecting group can be readily cleaved *in vivo* , for example by enzymatic hydrolysis, to release the biologically active parent. T. Higuchi and V. Stella provide a thorough discussion of the prodrug concept in "Pro-drugs as Novel Delivery Systems", Vol 14 of the A.C.S. Symposium Series, American Chemical Society (1975). Such carboxy-protecting groups are well known to those skilled in the art, having been extensively used in the protection of carboxyl groups in the penicillin and cephalosporin fields, as described in U.S. Pat. No. 3,840,556 and 3,719,667, the disclosures of which are incorporated herein by reference. Examples of esters useful as prodrugs for compounds containing carboxyl groups can be found on pages 14-21 of "Bioreversible Carriers in Drug Design: Theory and Application", edited by E.B. Roche, Pergamon Press:New York (1987). Representative protecting groups include $C_1$ to $C_8$ alkyl (e.g., methyl, ethyl or tertiary butyl and the like), benzyl and substituted derivatives thereof such as alkoxybenzyl or nitrobenzyl groups and the like, dialkylaminoalkyl (e.g., dimethylaminoethyl and the like), alkanoyloxyalkyl groups such as pivaloyloxymethyl or propionyloxymethyl and the like.

The term "halo-substituted lower alkyl" refers to a lower alkyl radical, as defined below, bearing at least one halogen substituent, for example, chloromethyl, fluoroethyl or trifluoromethyl and the like.

The term "halogen" or "halo" as used herein refers to I, Br, Cl or F.

The term "lower alkoxy" refers to R***O- wherein R*** is loweralkyl. Representative examples of lower alkoxy groups include methoxy, ethoxy, t-butoxy and the like.

The term "loweralkyl thio" as used herein refers to R*S- wherein R* is loweralkyl.

The term "lower alkoxy substituted loweralkyl" as used herein refers to a loweralkyl radical to which is appended a lower alkoxy group.

The term "hydroxy substituted loweralkyl" as used herein refers to a loweralkyl radical to which is appended one or two hydroxy (-OH) groups.

The term "lower alkyl" refers to branched or straight chain alkyl groups comprising one to ten carbon atoms, including, methyl, ethyl, propyl, isopropyl, n-butyl, t-butyl, neopentyl, and the like.

The term "lower alkenyl" as used herein refers to a branched or straight chain comprising two to ten carbon atoms which also comprises one or more carbon-carbon double bonds.

The term "lower alkynyl" as used herein refers to a branched or straight chain comprising two to ten carbon atoms which also comprises one or more carbon-carbon triple bonds.

The term "cycloalkyl" as used herein refers to an alicyclic group comprising from 3 to 7 carbon atoms including, but not limited to, cyclopropyl, cyclobutyl, cyclopentyl, cyclohexyl and the like.

The term "cycloalkylalkyl" as used herein refers to a loweralkyl radical to which is appended a cycloalkyl group.

The term "(loweralkyl)amino" refers to amino groups substituted with one or two lower alkyl groups, as defined above, including, methylamino, ethylamino, dimethylamino, propylamino or ethylmethylamino and the like.

As used herein the term "loweralkylthio-substituted lower alkyl" refers to a a loweralkyl radical to which is appended a lower alkyl thio group. Representative loweralkylthio-substituted loweralkyl groups include methylthiomethyl, methylthioethyl, ethylthioethyl, propylthiomethyl and the like.

The term "phenyl" refers to an unsubstituted benzene radical or a benzene radical substituted with from one to three substituents independently selected from halogen, hydroxy, lower alkyl, lower alkoxy, loweralkyl thio and halo-substituted lower alkyl.

By "pharmaceutically acceptable" it is meant those salts which are, within the scope of sound medical judgement, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M Berge, *et al.* describe pharmaceutically acceptable salts in detail in *J. Pharmaceutical Sciences*, 1977, ***66***::1 - 19 . The salts can be prepared *in situ* during the final isolation and purification of the compounds of formula (I), or separately by reacting the free base function with a suitable organic acid. Representative acid addition salts include

acetate, adipate, alginate, ascorbate, aspartate, benzenesulfonate, benzoate, bisulfate, borate, butyrate, camphorate, camphersulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, maleate, methanesulfonate, 2-naphthalenesulfonate, nicotinate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, calcium, potassium, magnesium salts and the like.

As used herein, the term "pharmaceutically acceptable carriers" means a non-toxic, inert solid, semi-solid or liquid filler, diluent, encapsulating material or formulation auxillary of any type. Some examples of the materials that can serve as pharmaceutically acceptable carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols, such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol and phosphate buffer solutions, as well as other non-toxic compatible substances used in pharmaceutical formulations. Wetting agents, emulsifiers and lubricants such as sodium lauryl sulfate and magnesium stearate, as well as coloring agents, releasing agents, coating agents, sweetening, flavoring and perfuming agents, preservatives and antioxidants can also be present in the composition, according to the judgement of the formulator.

By a "therapeutically effective amount" of the compound of the invention is meant a sufficient amount of the compound to treat hypertension, edema, renal failure, congestive heart failure, glaucoma, psoriaasis, benign prostatic hypertrophy, diabetic nephropathy, Alzheimer's disease or to prevent atherosclerosis or to treat gastrointestinal disorders associated with enhanced contractility and/or motility of intestinal smooth muscle or to treat contractile disorders of the uterus (including premature contractions, dysmenorrhea and the like) in a human or other mammal, at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The total daily dose of the compounds of this invention administered to a human or other mammal in single or in divided doses can be in amounts, for example, from 0.01 to 25 mg/kg body weight or more usually from 0.1 to 15 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of this invention per day in multiple doses or in a single dose of from 10 mg to 1000 mg.

Liquid dosage forms for oral administration may include pharmaceutically acceptable emulsions, microemulsions, solutions, suspensions, syrups and elixirs containing inert diluents commonly used in the art such as water. Such compositions may also comprise adjuvants, such as wetting agents; emulsifying and suspending agents; sweetening, flavoring and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulation can be sterilized, for example, by filtration through a bacteria-retaining filter, or by incorporating sterilizing agents in the form of sterile solid compositions which can be dissolved or dispersed in sterile water or other sterile injectable medium just prior to use.

In order to prolong the effect of a drug, it is often desirable to slow the absorption of a drug from subcutaneous or intramuscular injection. The most common way to accomplish this is to inject a suspension of crystalline or amorphous material with poor water solubility The rate of absorption of the drug becomes dependent on the rate of dissolution of the drug which is, in turn, dependent on the physical state of the drug, for example, the crystal size and the crystalline form. Another approach to delaying absorption of a drug is to administer the drug as a solution or suspension in oil. Injectable depot forms can also be made by forming microcapsule matrices of drugs and biodegradable polymers such as polylactide-polyglycolide. Depending on the ratio of drug to polymer and the composition of the polymer, the rate of drug release can be controlled. Examples of other biodegradable polymers include poly-orthoesters and polyanhydrides. The depot injectables can also be made by entrapping the drug in liposomes or microemulsions which are compatible with body tissues.

Suppositories for rectal administration of the drug can be prepared by mixing the drug with a suitable nonirritating excipient such as cocoa butter and polyethylene glycol which are solid at ordinary temperature but liquid at the rectal temperature and will therefore melt in the rectum and release the drug.

Solid dosage forms for oral administration may include capsules, tablets, pills, powders, prills and granules. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such as magnesium stearate and microcrystalline cellulose. In the case of capsules, tablets and pills, the dosage forms may also comprise buffering agents. Tablets and pills can additionally be prepared with enteric coatings and other release-controlling coatings.

Solid compositions of a similar type may also be employed as fillers in soft and hard-filled gelatin capsules using such exipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferably, in a certain part of the intestinal tract, optionally in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulations, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispersing the compound in the proper medium. Absorption enhancers can also be used to increase the flux of the compound across the skin. The rate can be controlled by either providing a rate controlling membrane or by dispersing the compound in a polymer matrix or gel.

The compounds of the present invention may be administered alone or in combination or in concurrent therapy with other cardiovascular agents independently selected from diuretics, adrenergic blocking agents, vasodilators, calcium channel blockers, angiotensin converting enzyme (ACE) inhibitors, potassium channel activators, antiserotoninergic agents, thromboxane synthetase inhibitors, renin inhibitors and other agents useful for treating (in a human or other mammal) hypertension, edema or congestive heart failure.

Representative diuretics include hydrochlorothiazide, chlorothiazide, acetazolamide, amiloride, bumetanide, benzthiazide, ethacrynic acid, furosemide, indacrinone, metolazone, spironolactone, triamterene, chlorthalidone and the like or a pharmaceutically acceptable salt thereof.

Representative adrenergic blocking agents include phentolamine, phenoxybenzamine, prazosin, terazosin, tolazine, atenolol, metoprolol, nadolol, propranolol, timolol, carteolol and the like or a pharmaceutically acceptable salt thereof.

Representative vasodilators include hydralazine, minoxidil, diazoxide, nitroprusside, flosequinan and the like or a pharmaceutically acceptable salt thereof.

Representative calcium channel blockers include amrinone, bencyclane, diltiazem, fendiline, flunarizine, nicardipine, nimodipine, perhexilene, verapamil, gallopamil, nifedipine and the like or a pharmaceutically acceptable salt thereof.

Representative ACE inhibitors include captopril, enalapril, lisinopril and the like or a pharmaceutically acceptable salt thereof.

Representative potassium channel activators include pinacidil and the like or a pharmaceutically acceptable salt thereof.

Representative antiserotoninergic agents include ketanserin and the like or a pharmaceutically acceptable salt thereof.

Representative renin inhibitiors include enalkiren, A-72517, PD-134672 or Ro 42-5892 and the like.

Other representative cardiovascular agents include sympatholytic agents such as methyldopa, clonidine, guanabenz, reserpine and the like or a pharmaceutically acceptable salt thereof.

The compound of formula I and the other cardiovascular agent can be administered at the recommended maximum clinical dosage or at lower doses. Dosage levels of the active compounds in the compositions of the invention may be varied so as to obtain a desired therapeutic response depending on the route of administration, severity of the disease and the response of the patient. The combination can be administered as separate compositions or as a single dosage form containing both agents.

In general, the compounds of this invention can be prepared by the processes illustrated in Schemes I through X. It should be understood that A, B, V, W, X, Y, Z, n, $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$ and $R^{17}$ as used herein correspond to the groups identified by formula (I). P is a protecting group. In the course of synthesis, certain groups present in the molecule, particulary carboxylic acid and tetrazole groups, are protected and deprotected as necessary. The term "protecting group" is well known in the art and refers to substituents on functional groups of compounds undergoing chemical transformation which prevent undesired reactions and degradations during a synthesis; see, for example, T.H. Greene, "Protective Groups in Organic Synthesis", John Wiley & Sons, New York (1981) for methods of introducing and removing appropriate protecting groups. Suitable carboxy-protecting groups include t-butyl and benzyl groups. Suitable tetrazole nitrogen-protecting groups include triphenylmethyl (Tr), p-nitrobenzyl and 1-ethoxyethyl.

The compounds of formula (I) may be prepared using the reactions and techniques described in this section. The reactions are performed in a solvent appropriate to the reagents and materials employed and suitable for the transformation being effected. It is understood by those skilled in the art of organic synthesis that the functionality present on the heterocycle and other portions of the molecule must be consistent with the chemical transformation proposed. This will frequently necessitate judgment as to the order of synthetic steps, protecting groups required and deprotection conditions. Throughout the following section, not all compounds of formula (I) falling into a given class may necessarily be prepared by all methods described for that class. Substituents on the starting materials may be incompatible with some of the reaction conditions required in some of the methods described. Such restrictions to the substituents which are compatible with the reaction conditions will be readily apparent to one skilled in the art and alternative methods described must then be used.

Scheme I A

According to reaction scheme I A , an intermediate of Formula 1 (prepared as shown in reaction schemes II, III, IV, V, VI VII and VIII), is condensed with an intermediate of Formula 2 (prepared as shown in reaction schemes IX and X) to afford a compound of Formula 3. In the case wherein $R^1$P is COOR or a protected tetrazole ring, the R carboxy-protecting group or the tetrazole N-protecting group can then be removed to afford a compound of Formula (I A). One suitable method for removing triphenylmethyl N-protecting groups is mild acid treatment, for example using ethanolic hydrogen chloride followed by aqueous work-up.

Alternately, in the case wherein $R^3$ is an ester group of Formula $COOR^{10}$, the ester may be hydrolyzed to give a compound of Formula 3a and the $R^1$ protecting group, if present, is removed to afford a compound of Formula (I B).

Alternately, the compound of Formula 3 (in which $R^3$ is an ester group of Formula $COOR^{10}$) may be treated with a suitable reagent for reducing the ester group to a hydroxyl group, for example lithium aluminum hydride. The $R^1$ protecting group, if present, is removed to afford a compound of Formula (I C). The compounds of Formula 3b may also be oxidized with a suitable oxidizing agent such as manganese

dioxide or pyridinium chlorochhromate (and the $R^1$ protecting group, if present removed) to afford the compounds of Formula (I D). The compounds of Formula (I C) also may be further converted by activation (with for example methanesulfonyl chloride) or replacement (with, for example bromine) of the hydroxy group, followed by nucleophilic displacement with an alkoxide anion (e.g. sodium methoxide in methanol) and removal of the $R^1$ protecting groups, if present, to afford an ether of Formula (I E).

Scheme I B

According to reaction scheme I B, a compound of Formula 3a is treated with an amine in the presence of a coupling reagent such as 3-(dimethylaminopropyl)-3-ethyl-carbodiimide hydrochloride and a suitable base, preferably N-methylmorpholine, followed by removal of the $R^1$ protecting groups, if present, to afford an amide of Formula (I F). Suitable amines include pyrrolidine, morpholine, 4-methoxymethoxypiperidine, dimethylamine, methylamine, 2-(hydroxyethyl)amine, 2-(methoxymethyl)amine, and 2-(2-methoxyethoxy)-amine.

Scheme I C

According to reaction scheme I C, a compound of Formula 3b is treated with an acid chloride, for example benzoyl chloride or acetyl chloride, followed by removal of the $R^1$ protecting groups, if present, to afford an ester of Formula (I G).

Scheme I D

According to reaction scheme I D, a compound of Formula 3 (in which $R^3$ is a cyano group) is hydrolyzed by standard methods, for example using aqueous potassium hydroxide, followed by removal of the $R^1$ protecting groups, if present, to afford an amide of Formula (I H).

Alternately. a compound of Formula 3 is treated with a suitable reducing agent, for example lithium aluminum hydride, to afford the aminomethyl compound of Formula 3c. In the case wherein $R^1$ is COOR or a protected tetrazole ring, the R carboxy-protecting group or the tetrazole N-protecting group can then removed to afford a compound of Formula (I J). Alternately, a compound of Formula 3c is converted to the corresponding amides, carbamates and sulfonylamines by standard methods. Treatment of a compound of Formula 3c with a suitable isocyanate such as methyl isocyanate affords a compound of Formula 3d in which Y is $C(O)NHR^{16}$ (a urea derivative). Treatment of a compound of Formula 3c with a suitable acid chloride such as acetyl chloride or benzoyl chloride, in the presence of a base, affords a compound of Formula 3d in which Y is $C(O)R^{16}$. Treatment of a compound of Formula 3c with a sulfonic acid chloride, for example methanesulfonyl chloride, in the presence of a suitable base (e.g. triethylamine) affords a compound of Formula 3d in which Y is $S(O)2R^{17}$. In the case wherein $R^1$ is COOR or a protected tetrazole ring, the R carboxy-protecting group or the tetrazole N-protecting group can then be removed to afford a compound of Formula (I K).

Scheme I E

According to reaction scheme I E, an intermediate of Formula 1 (prepared as shown in reaction schemes II, III, IV, V, VI VII and VIII), is condensed with an intermediate of Formula 2 (prepared as shown in reaction schemes IX and X) to afford a compound of Formula 3. A compound of Formula 3 is, in turn, reduced to an amino compound of Formula 4 using standard methods. A compound of Formula 4 is then treated with a suitable sulfonyl chloride, for example trifluoromethylsulfonyl chloride, to afford a compound of Formula (I L).

Scheme I F

According to reaction scheme I F , an intermediate of Formula 1 (prepared as shown in reaction schemes II, III, IV, V, VI VII and VIII), is condensed with an intermediate of Formula 5 (prepared as shown in reaction scheme XII) to afford a compound of Formula 6. In the case wherein $R^1P$ is COOR or a protected tetrazole ring, the R carboxy-protecting group or the tetrazole N-protecting group can then removed to afford a compound of Formula (I M). One suitable method for removing triphenylmethyl N-protecting groups is mild acid treatment, for example using ethanolic hydrogen chloride followed by aqueous work-up.

Alternately, in the case wherein $R^3$ is an ester group of Formula $COOR^{10}$, the ester may be hydrolyzed

to give a compound of Formula 6a and the $R^1$ protecting group, if present, is removed to afford a compound of Formula (I N).

Alternately, the compound of Formula 6 (in which $R^3$ is an ester group of Formula $COOR^{10}$) may be treated with a suitable reagent for reducing the ester group to a hydroxyl group, for example lithium aluminum hydride to afford the compounds of Formula 6b. The $R^1$ protecting group, if present, is removed to afford a compound of Formula (I O). The compounds of Formula 6b may also be oxidized with a suitable oxidizing agent such as manganese dioxide or pyridinium chlorochhromate (and the $R^1$ protecting group, if present removed) to afford the compounds of Formula (I P). The compounds of Formula (I O) also may be further converted by activation (with for example methanesulfonyl chloride) or replacement (with, for example bromine) of the hydroxy group, followed by nucleophilic displacement with an alkoxide anion (e.g. sodium methoxide in methanol) and removal of the $R^1$ protecting groups, if present, to afford an ether of Formula (I Q).

Scheme I G

According to reaction scheme I G, an intermediate of Formula 1 (prepared as shown in reaction schemes II through VIII), is condensed with an intermediate of Formula 7 (prepared as shown in reaction scheme XIII) and the $R^1$ protecting group, if present, is removed to afford a compound of Formula (I R).

Scheme I H

According to reaction scheme I H, an intermediate of Formula 1 (prepared as shown in reaction schemes II through VIII), is condensed with an intermediate of Formula 8 (prepared as shown in reaction scheme XIV) and the $R^1$ protecting group, if present, is removed to afford a compound of Formula (I S).

Scheme I J

According to reaction scheme I J, an intermediate of Formula 1 (prepared as shown in reaction schemes II through VIII), is condensed with an intermediate of Formula 9 and the $R^1$ protecting group, if present, is removed to afford a compound of Formula (I T).

Alternately, an intermediate of Formula 1 (prepared as shown in reaction schemes II through VIII), is condensed with an intermediate of Formula 10 (prepared as shown in reaction scheme XI) to give a compound of Formula 11 which is treated with a suitable reagent for reducing the nitro group (for example, catalytic hydrogenation) to give the a compound of Formula 12. A compound of Formula 12 is, in turn, reacted with a sulfonyl chloride such as methanesulfonyl chloride or trifluoromethanesulfonyl chloride and the $R^1$ protecting group, if present, is removed to afford a compound of Formula (I U).

Alternately, an intermediate of Formula 1 (prepared as shown in reaction schemes II through VIII), is condensed with an intermediate of Formula 13 and the $R^1$ protecting group, if present, is removed to afford a compound of Formula (I V).

Scheme I K

According to reaction scheme I J, an intermediate of Formula 1 (prepared as shown in reaction schemes II through VIII), is condensed with an intermediate of Formula 56 and the $R^1$ protecting group, if present, is removed to afford a compound of Formula (I T).

Scheme I A

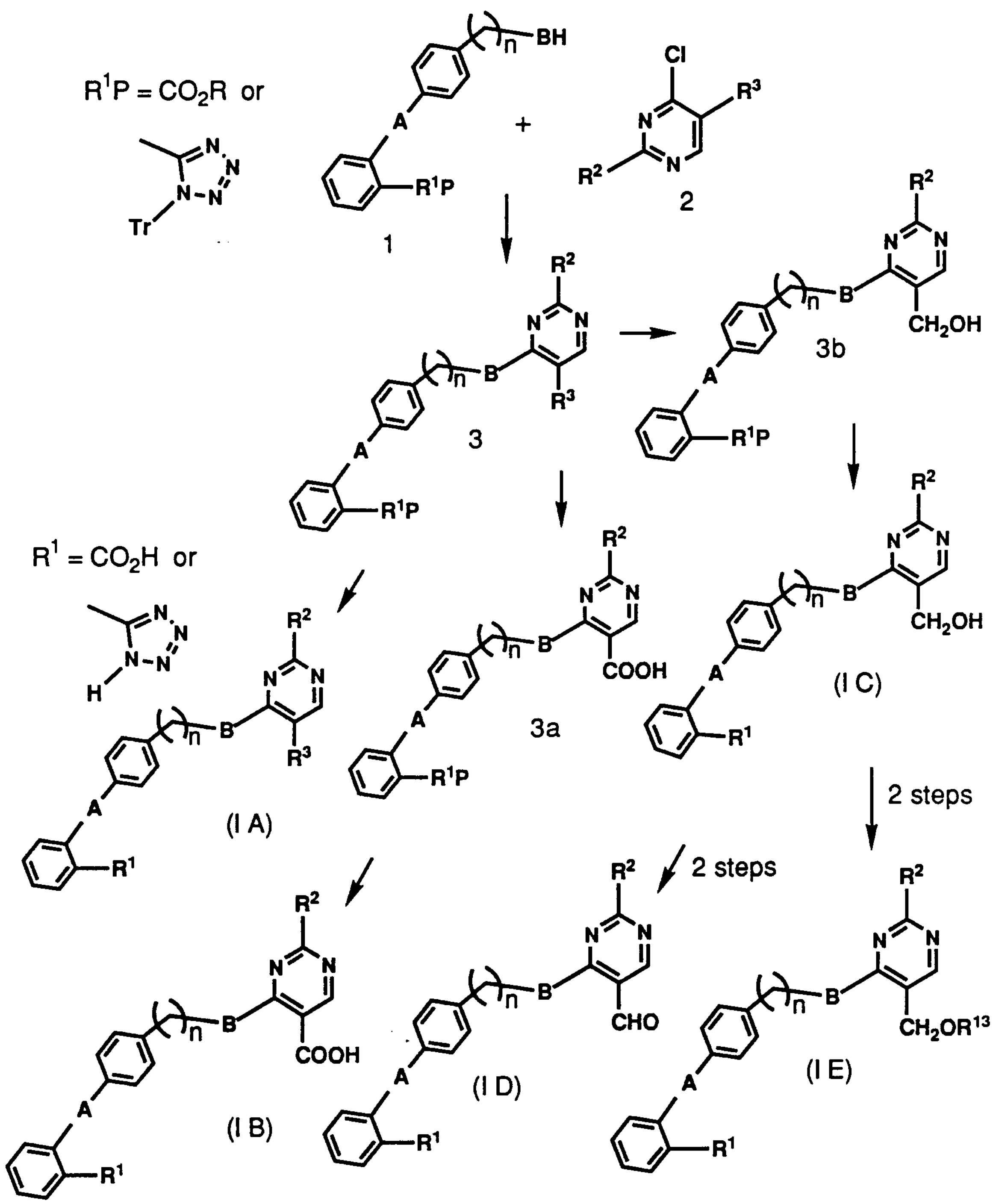


1, 2, 3, 3a, 3b, (I A), (I B), (I C), (I D), (I E)

Scheme I B

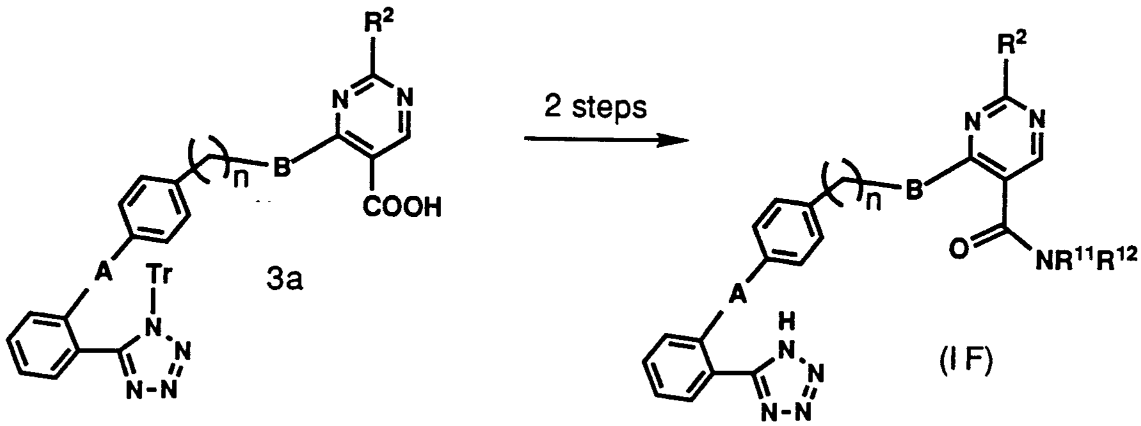


3a

(I F)

Scheme I C

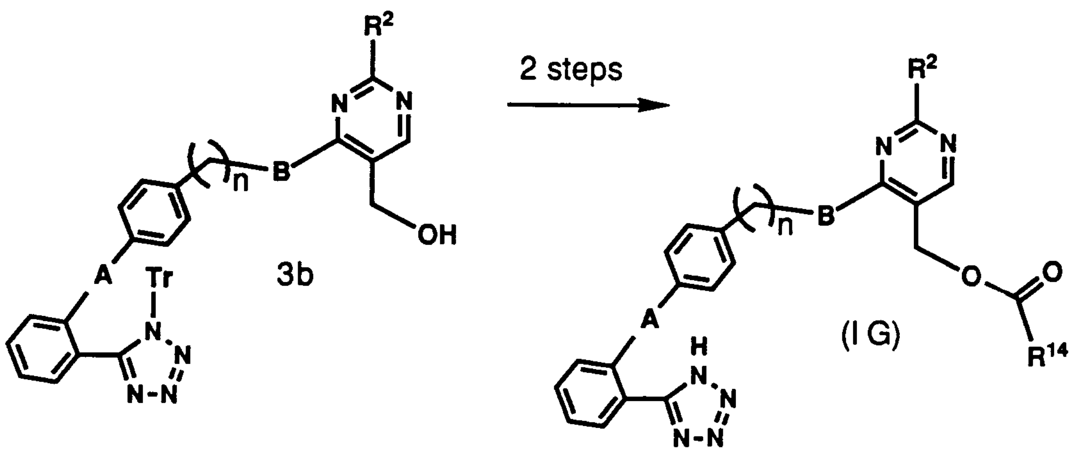


3b

(I G)

Scheme I D

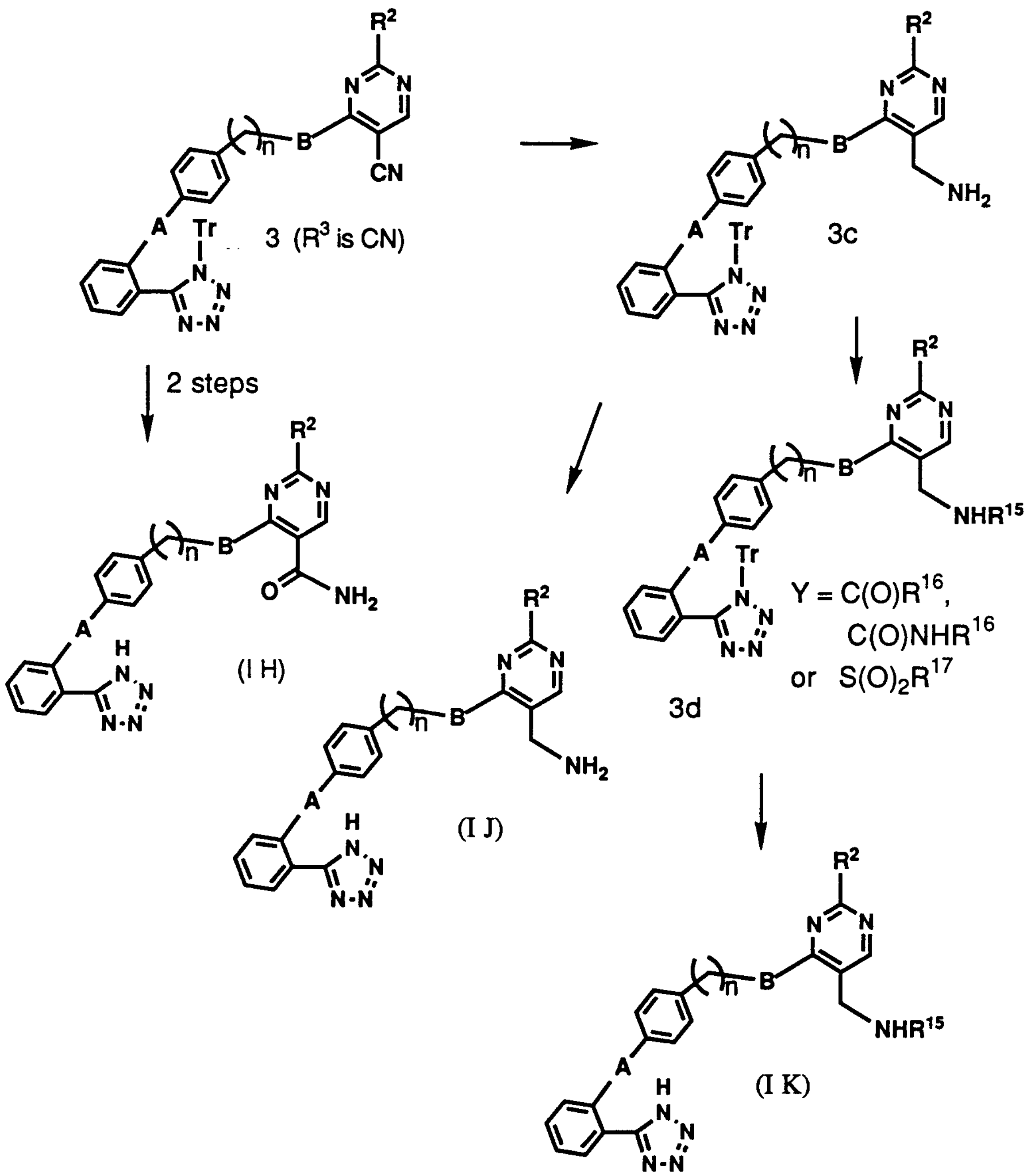

Scheme I E

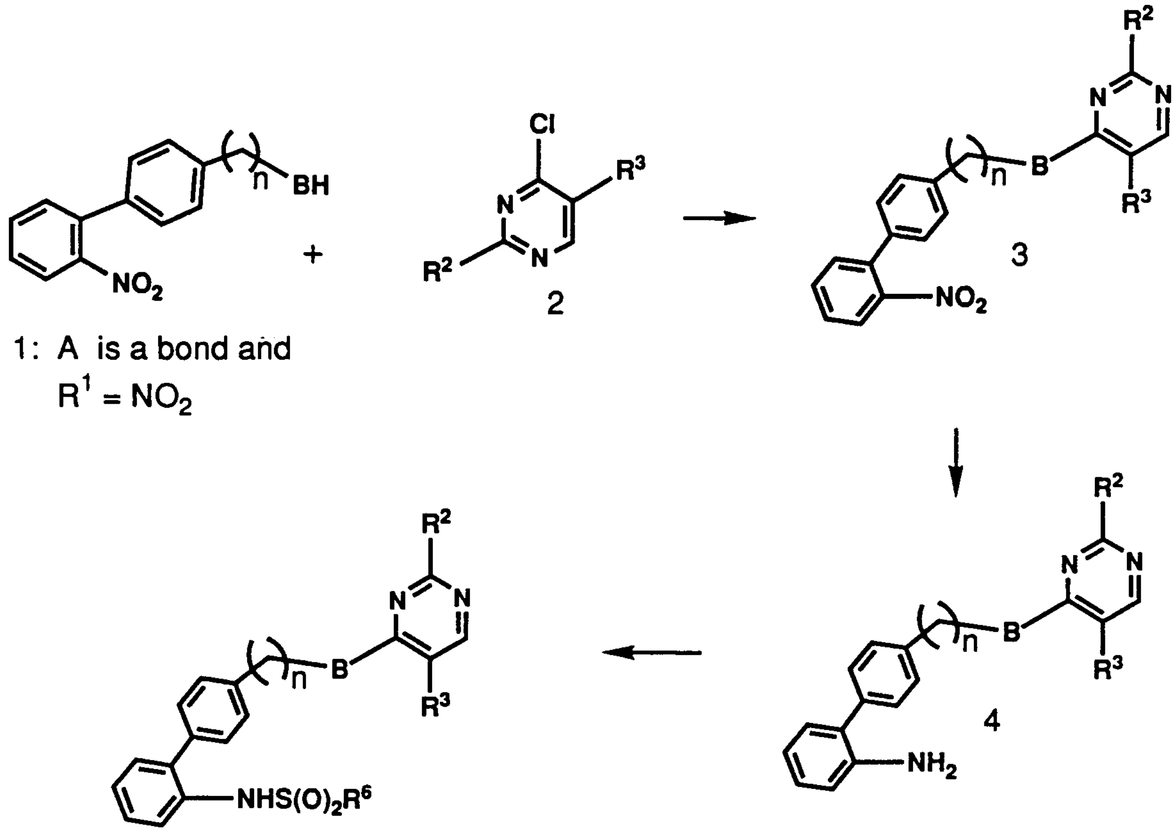

1: A is a bond and $R^1 = NO_2$

2

3

4

(I L)

Scheme I F

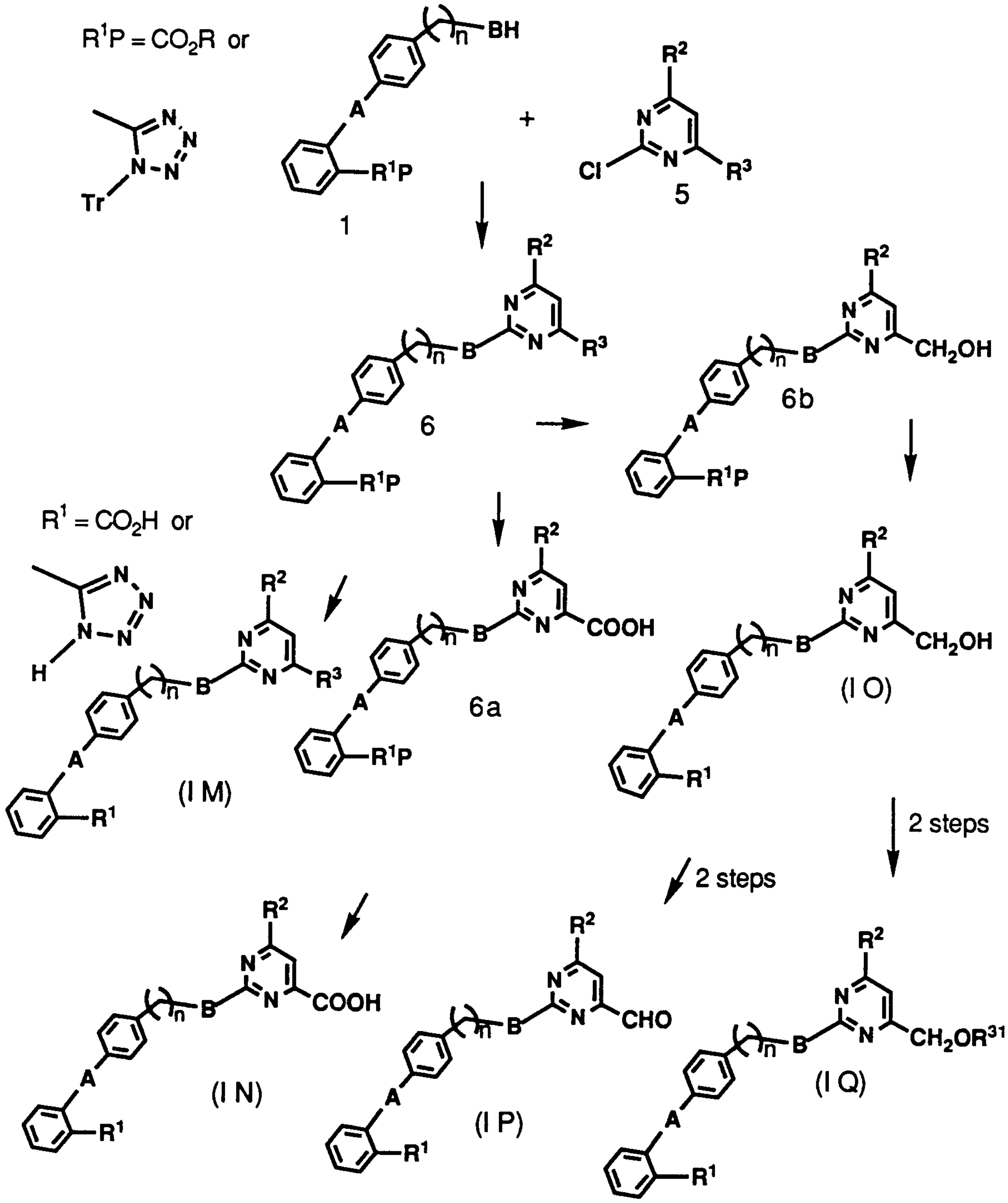


1, 5, 6, 6b, 6a, (I M), (I O), (I N), (I P), (I Q)

Scheme I G

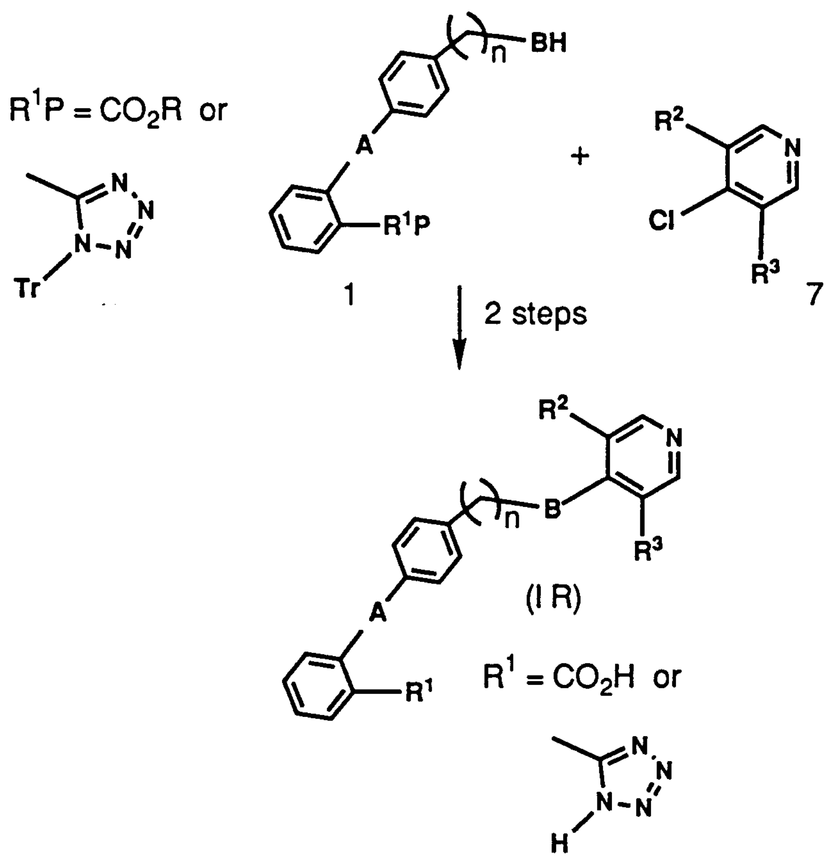


$R^1P = CO_2R$ or

1

7

(I R)

$R^1 = CO_2H$ or

Scheme I H

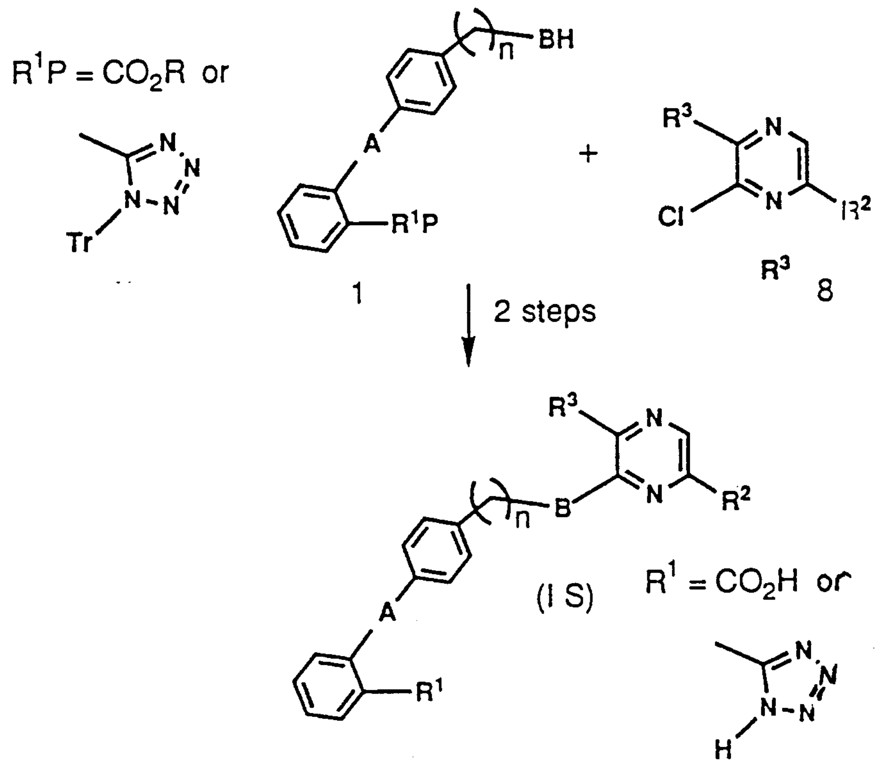


1

8

(I S)

Scheme I J

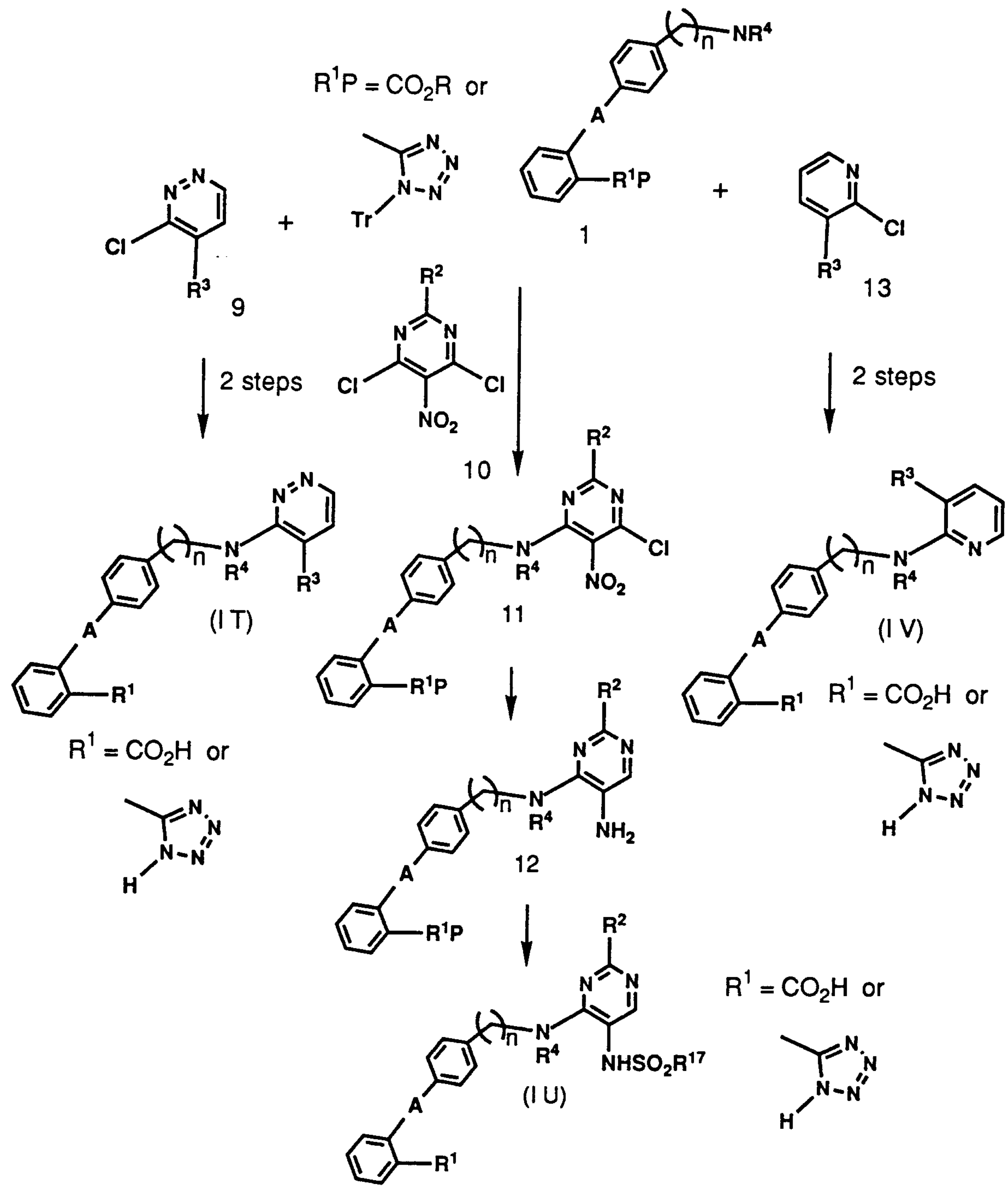


9 1 13 10 11 (I T) (I V) 12 (I U)

Scheme I K

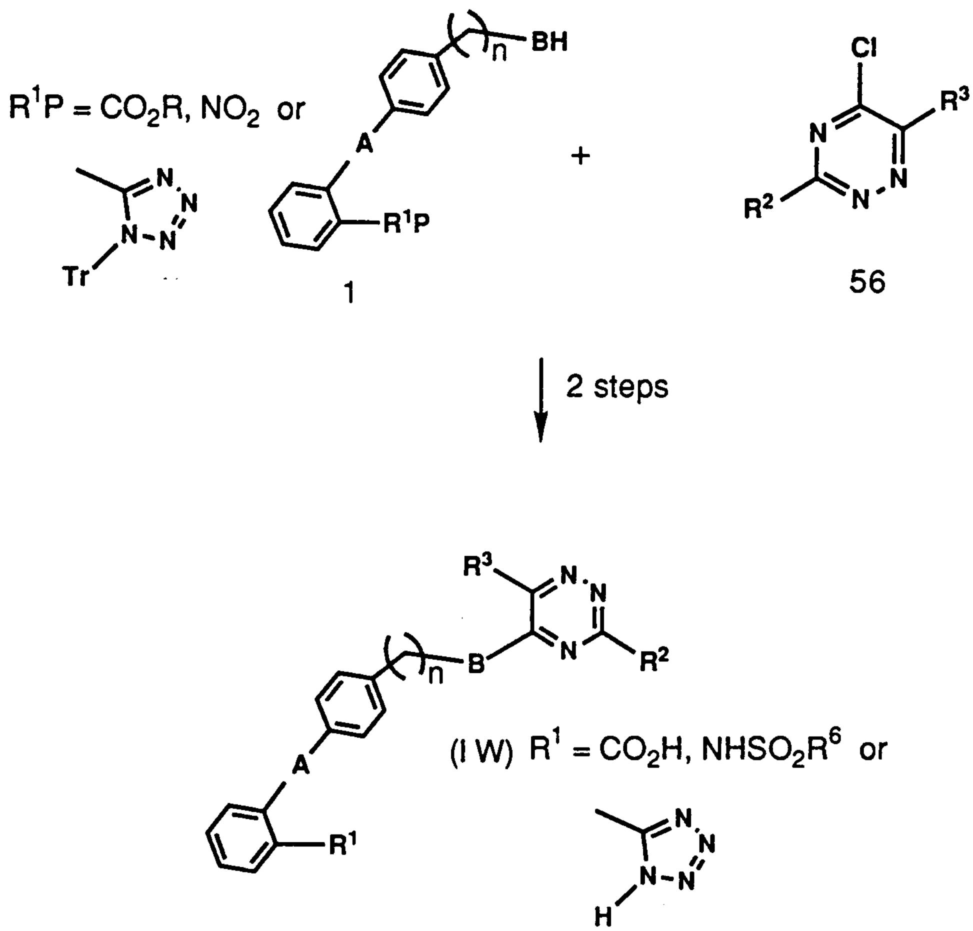


Biphenyl and Related Intermediates

Scheme II

According to reaction scheme II, a compound of Formula 14 is treated with an azide salt such as sodium azide in a polar solvent such as DMF or under phase transfer conditions to afford the corresponding azidomethylbiphenyl compound of Formula 15. The reduction of the azide by standard methods (for example catalytic hydrogenation) affords an aminomethyl compound of Formula 1 in which B is NH. Alternately, treatment of a bromomethyl compound of Formula 14 with a suitable primary amine, for example methylamine, affords an intermediate of Formula 1 in which B is $NR^4$.

Scheme III

According to reaction scheme III, A cyano compound of Formula 16 is treated with an azide salt such as sodium azide and triethylamine hydrochloride in a polar solvent, preferably at 150°C in N-methyl-2-pyrrolidinone, to afford the corresponding tetrazole derivative, followed by protection of the tetrazole by treatment with triphenylmethyl chloride to afford a compound of Formula 17. The nitro compound of Formula 17 is then reduced by standard methods (for example catalytic hydrogenation) to afford the aminobiphenyl compound of Formula 1. Acylation of the amino group by standard methods, followed by reduction of the intermediate amide affords a compound of Formula 1 in which B is $NR^4$.

Scheme IV

According to reaction scheme IV, 4-bromobenzylidenemalononitrile is reacted with N-(1-butadienyl)-morpholine to afford the compound of Formula 20. The compound of Formula 20 is treated with an azide salt such as sodium azide and triethylamine hydrochloride in a polar solvent, preferably at 150° C in N-methyl-2-pyrrolidinone, to afford the corresponding terazole derivative, followed by protection of the tetrazole by treatment with triphenylmethyl chloride to afford a compound of Formula 21. The bromo compound of Formula 21 is converted to the corresponding lithio compound which is then treated with tetraisopropylthiuram disulfide to afford the compound of Formula 22. The compound of Formula 22 is converted to the intermediate of Formula 1 by hydrolysis of the thiocarbamate using for example ethanolic potassium hydroxide.

Scheme V

According to reaction scheme V, 4-acetoxybenzylidenemalononitrile is reacted with N-(1-butadienyl)-morpholine to afford the compound of Formula 24. The compound of Formula 24 is treated with an azide salt such as sodium azide and triethylamine hydrochloride in a polar solvent, preferably at 150° C in N-methyl-2-pyrrolidinone, to afford the corresponding terazole derivative, followed by protection of the tetrazole by treatment with triphenylmethyl chloride to afford a compound of Formula 25. The acetoxy compound of Formula 25 is converted to the intermediate of Formula 1 by hydrolysis of the acetoxy group using for example methanolic lithium hydroxide.

Scheme VI

According to reaction scheme VI, a compound of Formula 4 is treated with potassium acetate to afford a compound of Formula 26. The compound of Formula 26 is, in turn, converted to an intermediate of Formula 1 in which n is 1 and B is an oxygen atom, by hydrolysis of the acetyl group using standard methods. Alternately, the compound of Formula 4 is treated with potassium thioacetate to afford the compound of Formula 27. The compound of Formula 27 is, in turn, converted to an intermediate of Formula 1 in which n is 1 and B is a sulfur atom, by hydrolysis of the acetyl group using standard methods.

Scheme VII

According to reaction scheme VII, a compound of Formula 28 is treated sequentially with a chlorinating agent (for example phosphorus trichloride, thionyl chloride or oxalyl chloride), ammonia and a dehydrating agent, preferably thionyl chloride, to afford the nitrile of Formula 29. The nitrile is converted to the corresponding tetrazole by treatment with an azide salt such as sodium azide and triethylamine hydrochloride, preferably at 150° C in N-methyl-2-pyrrolidinone, and the tetrazole is protected by treatment with triphenylmethyl chloride to afford the compound of Formula 30. The compound of Formula 30 is converted to the compound of Formula 31 by bromination under conditions suitable for selective bromination of the benzylic position. The bromine atom is displaced with sodium azide to afford the compound of Formula 32. The azido compound of Formula 32 is then reduced using standard methods (for example catalytic hydrogenation) to afford the intermediate of Formula 1 in which N is 1, A is an oxygen atom and B is NH.

Alternately, a compound of Formula 28 is etserified by standard methods to afford a compound of Formula 33 which is, in turn, converted to the corresponding azidomethyl compound by the procedures outlined above for the conversion of a compound of Formula 30 to a compound of Formula 32. The azidomethyl compound is reduced using standard methods to afford the intermediate of Formula 1 in which N is 1, A is an oxygen atom and B is NH.

Scheme VIII

According to reaction scheme VIII, The nitrile of Formula 34 is converted to the corresponding tetrazole by treatment with an azide salt such as sodium azide and triethylamine hydrochloride, preferably at 150° C in N-methyl-2-pyrrolidinone, and the tetrazole is protected by treatment with triphenylmethyl chloride to afford the compound of Formula 35. The compound of Formula 35 is converted to the compound of Formula 36 by bromination under conditions suitable for selective bromination of the benzylic position. The bromine atom is displaced with sodium azide to afford the compound of Formula 37. The azido compound of Formula 37 is then reduced using standard methods (for example catalytic hydrogenation) to afford the

intermediate of Formula 1 in which N is 1, A is a carbonyl group and B is NH.

Scheme II

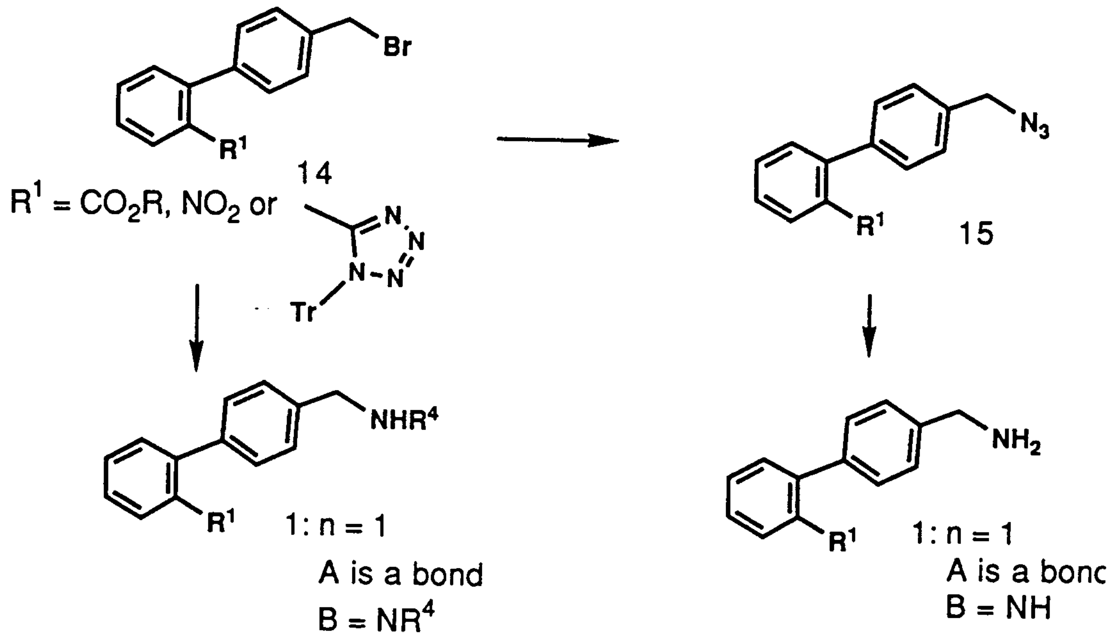

14

$R^1 = CO_2R$, $NO_2$ or

15

1: n = 1
A is a bond
B = $NR^4$

1: n = 1
A is a bonc
B = NH

Scheme III

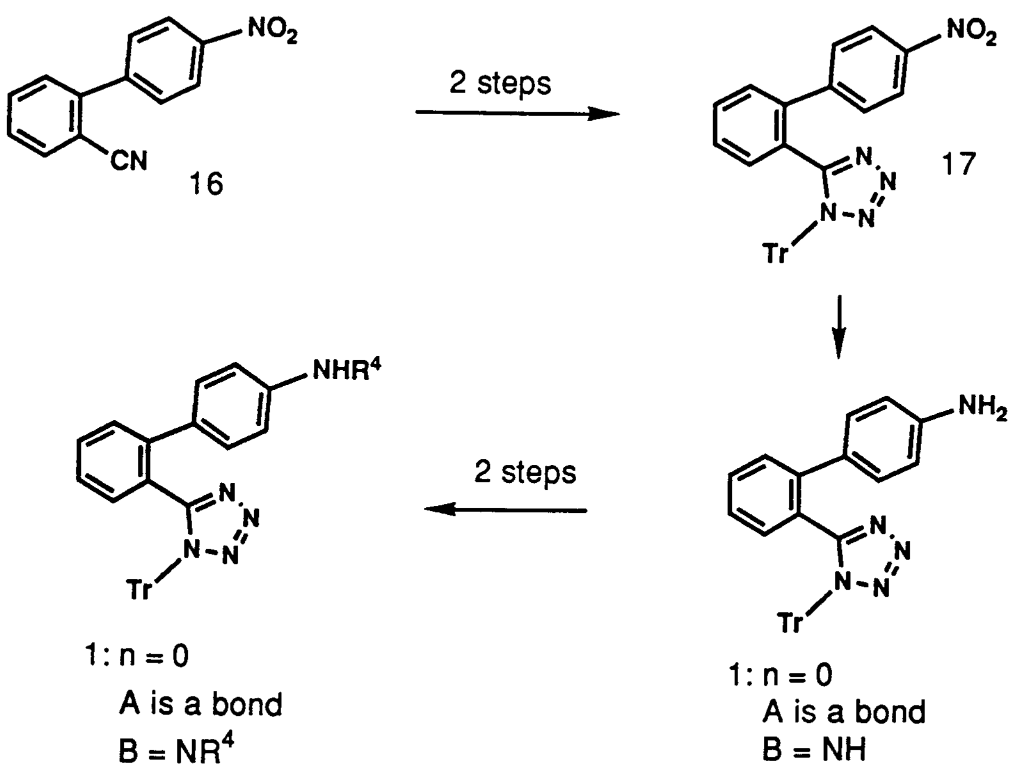


16

17

1: n = 0
A is a bond
B = $NR^4$

1: n = 0
A is a bond
B = NH

Scheme IV

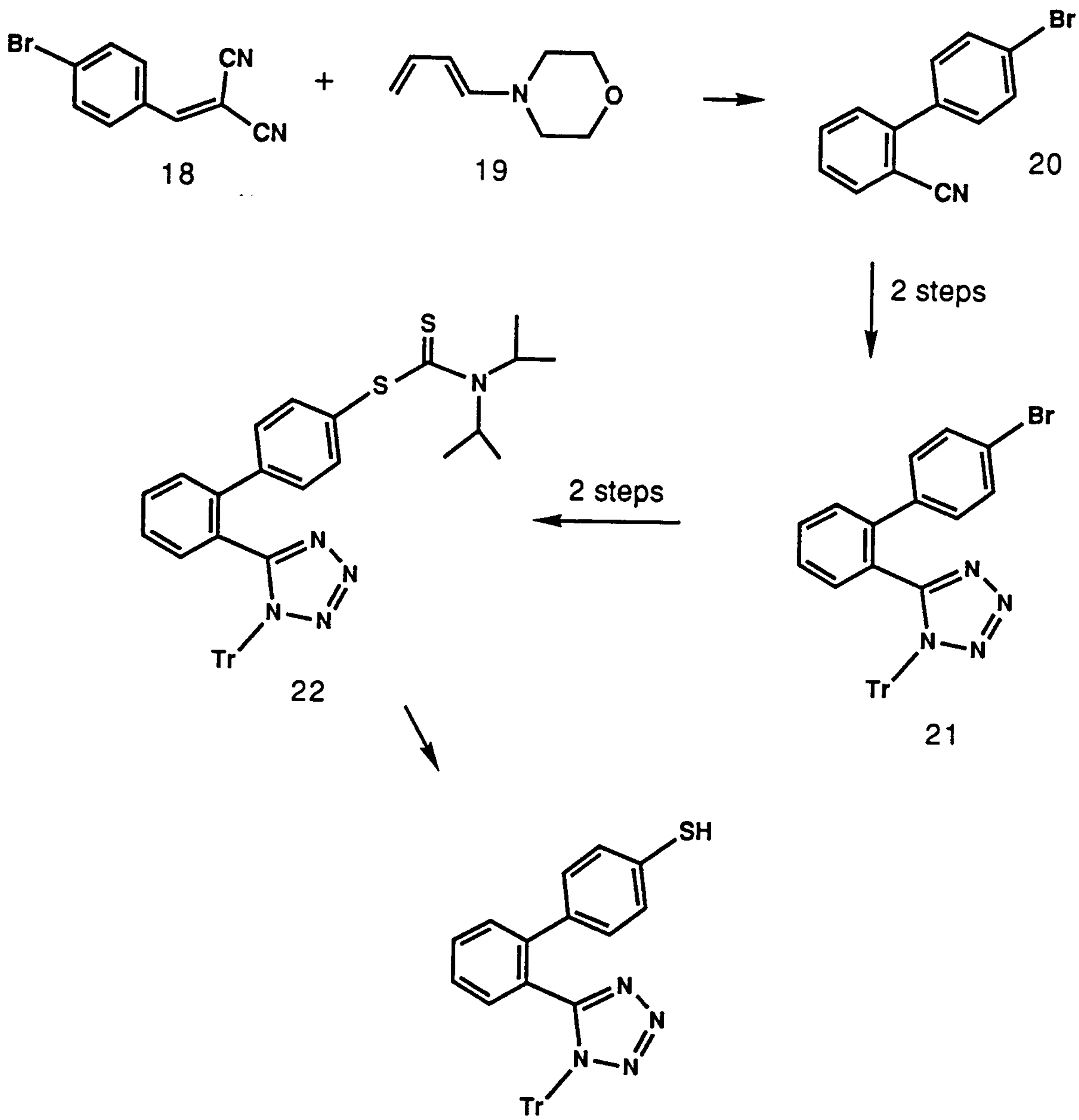


18

19

20

21

22

1: n = 0
A is a bond
B = S

Scheme V

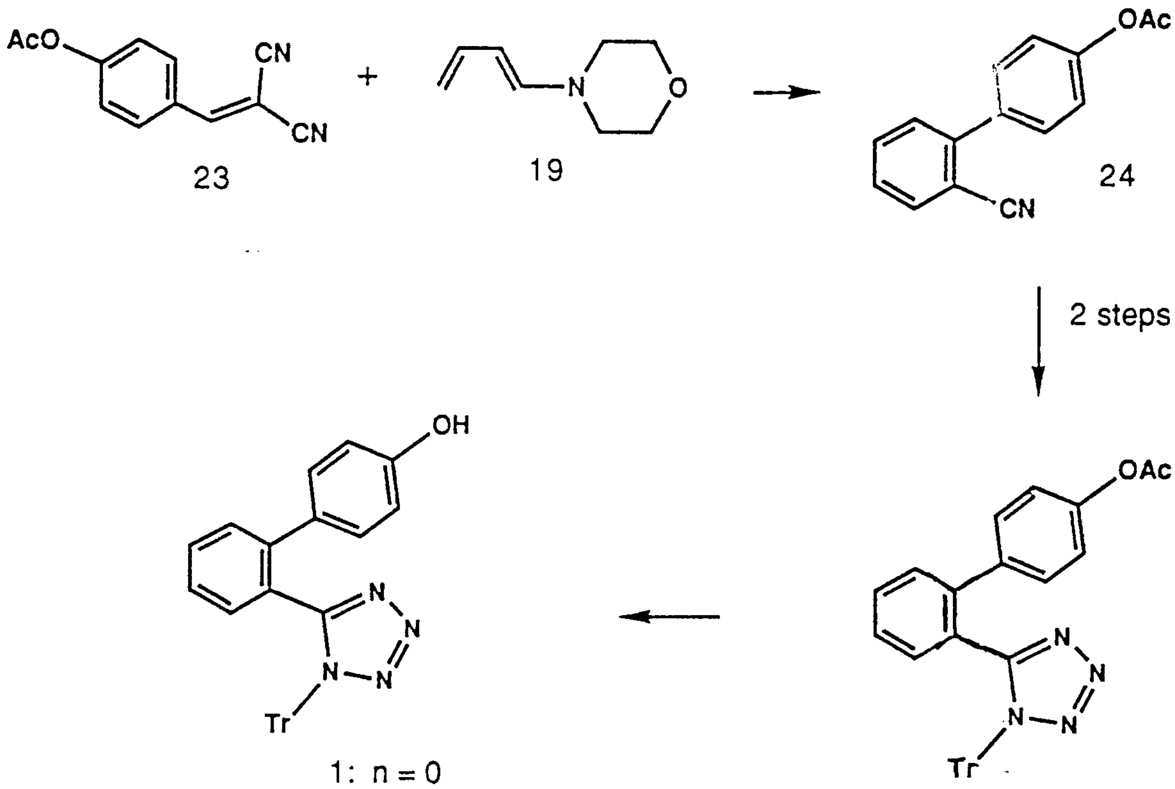


23

19

24

25

1: n = 0
A is a bond
B = O

Scheme VI

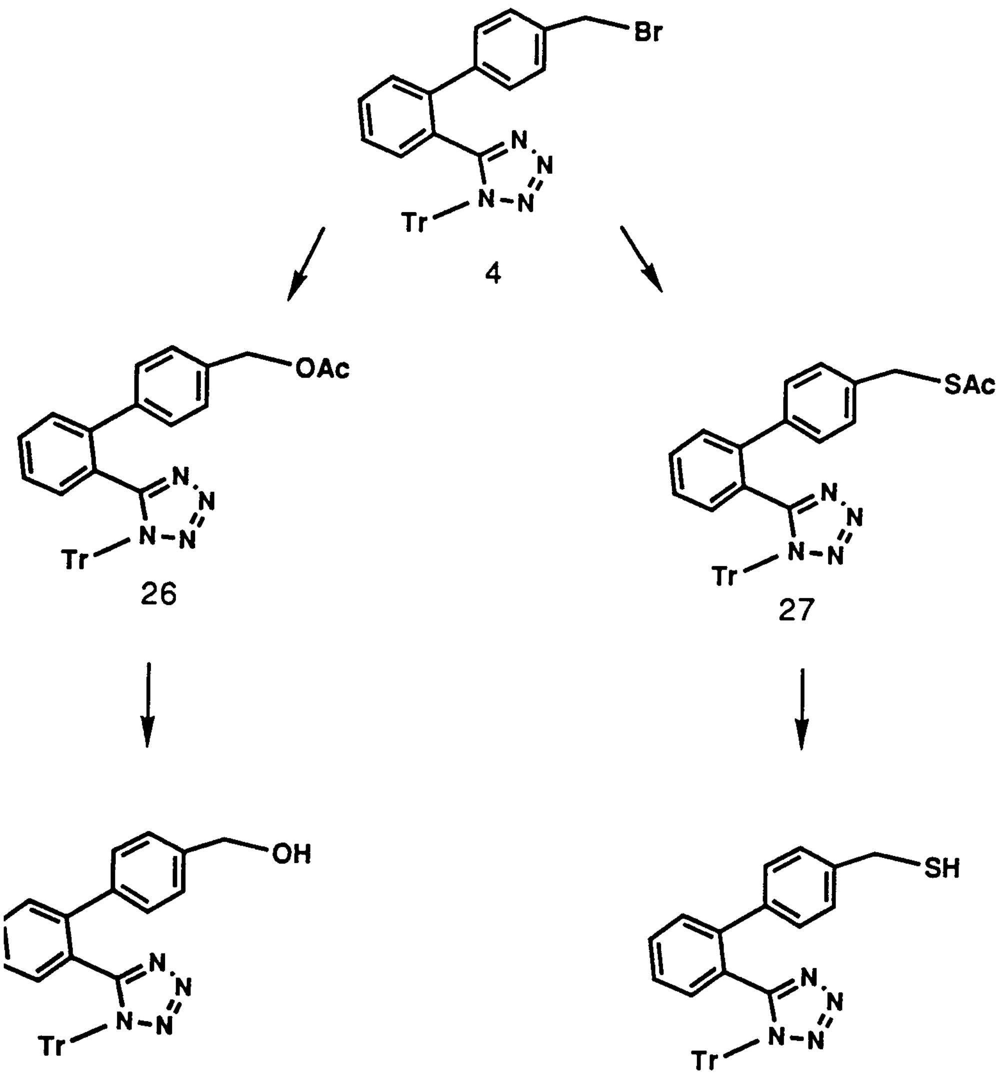

4

26

27

1: n = 1
A is a bond
B = O

1: n = 1
A is a bond
B = S

Scheme VII

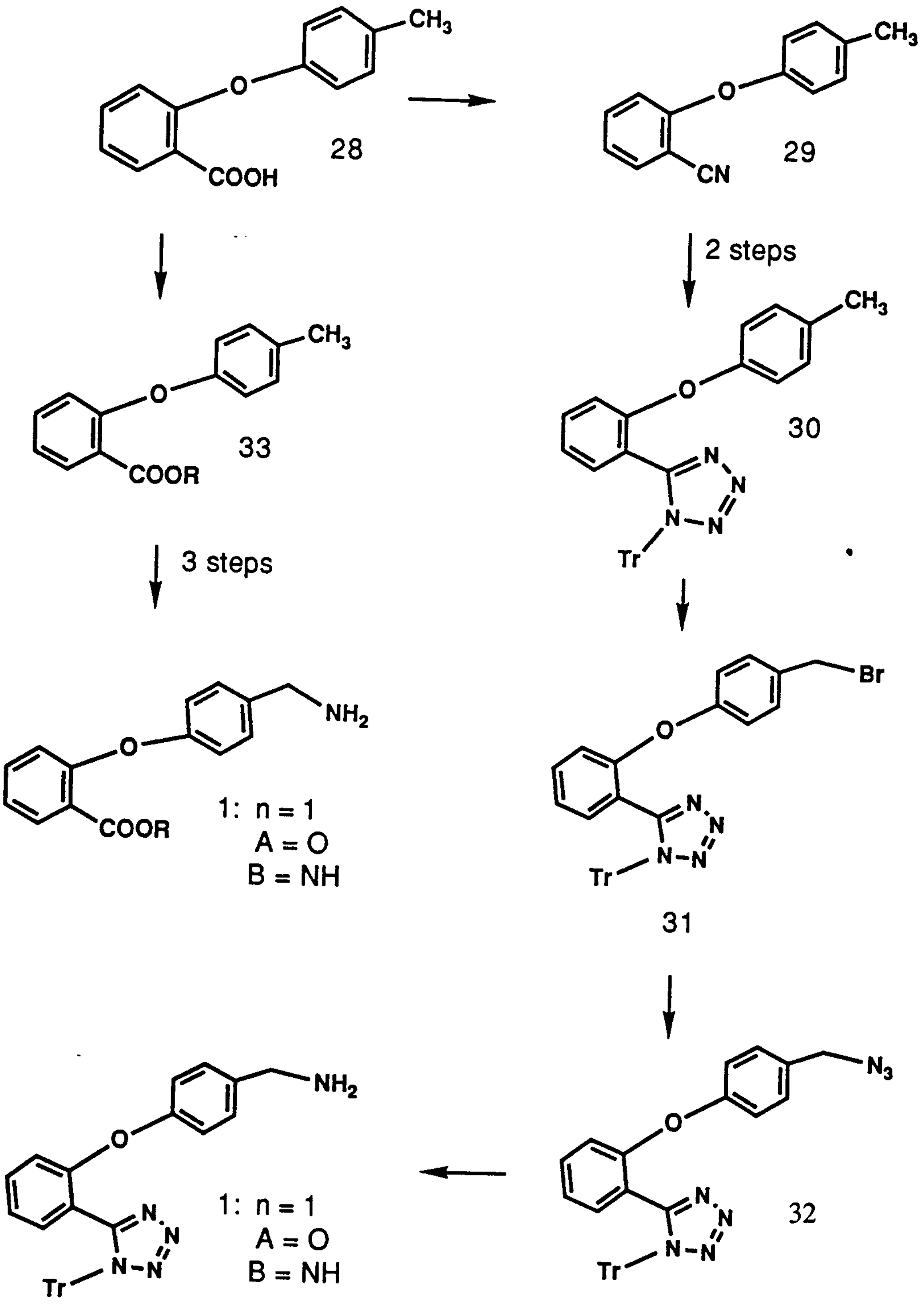


28

29

33

30

1: n = 1
A = O
B = NH

31

32

1: n = 1
A = O
B = NH

Scheme VIII

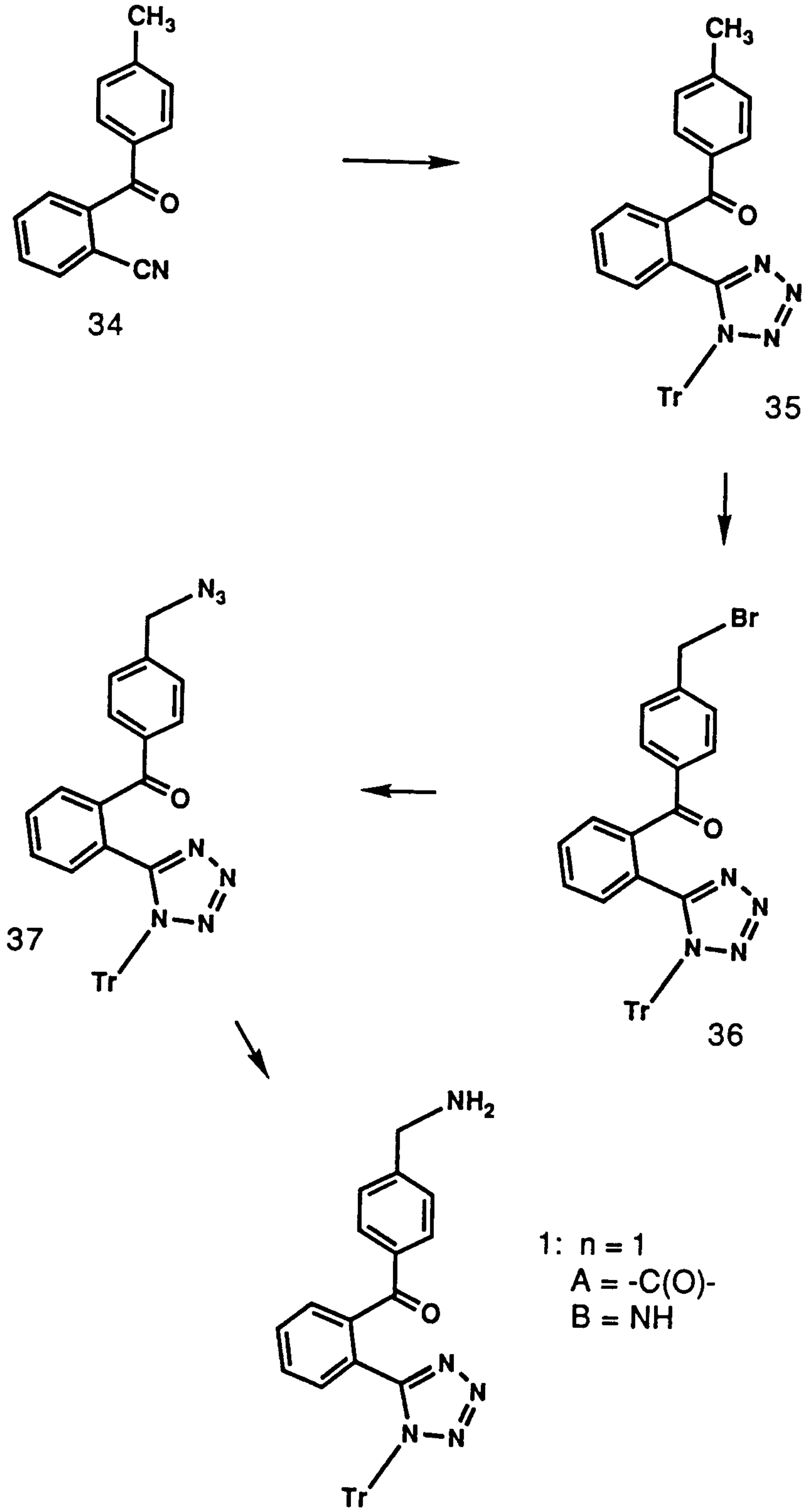

34

35

36

37

1: n = 1
A = -C(O)-
B = NH

Heterocyclic Intermediates

Scheme IX

According to reaction scheme IX, a nitrile of Formula 38 is treated with ethanol and hydrochloric acid to

afford a compound of Formula 39 which, in turn, is treated with ammonia to afford the amidine of Formula 40. The amidine of Formula 40 is then condensed with the ethoxymethylene malonate diester of Formula 41 to afford a hydroxy-pyrimidine of Formula 42. The compounds of Formula 42 are treated with a chlorinating agent, for example phosphorous oxychloride, to afford the intermediates of Formula 2 in which $R^3$ is a carboxylic ester.

Scheme X

According to reaction scheme X, the amidine of Formula 40 is condensed with ethoxymethylene malonate derivative of Formula 43 to afford a hydroxy-pyrimidine of Formula 44. The compounds of Formula 44 are treated with a chlorinating agent, for example phosphorous oxychloride, to afford the intermediates of Formula 2 in which $R^3$ is a cyano group.

Scheme XI

According to reaction scheme XI, an amidine of Formula 40 is condensed with a malonate diester in the presence of a suitable base, preferably sodium ethoxide, to afford the pyrimidine compound of Formula 45. A compound of Formula 45 is nitrated by standard methods to afford a compound of Formula 46. The compounds of Formula 46 are converted into the intermediates of Formula 10 by treatment with a suitable chlorinating agent, for example phosphorous oxychloride.

Scheme XII

According to reaction scheme XII, a ketoester of Formula 47 is condensed with urea to afford a pyrimidine compound of Formula 48. The compounds of Formula 48 are converted into the intermediates of Formula 5 by treatment with a suitable chlorinating agent, for example phosphorous oxychloride.

Scheme XIII

According to reaction scheme XIII, a ketoester of Formula 47 is condensed with triazine in the presence of a suitable base such as sodium ethoxide, to afford the pyridine compounds of Formula 49. The compounds of Formula 49 are converted into the intermediates of Formula 7 by treatment with a suitable chlorinating agent, for example phosphorous oxychloride.

Scheme XIV

According to reaction scheme XIV, an a-keto aldehyde of Formula 50 is condensed with aminomalonamide to afford a pyrazine compound of Formula 51. The compounds of Formula 51 are converted into the intermediates Formula 8 by hydrolysis to the corresponding acid using standard methods, for example using aqueous sodium hydroxide, followed by esterification of the acid and treatment with a suitable chlorinating agent, for example phosphorous oxychloride.

Scheme XV

According to reaction scheme XV, a compound of Formula 39 is condensed with formic hydrazide to afford a compound of Formula 52. A compound of Formula 52 is, in turn, treated with a suitable acid such as hydrochloric acid to afford a compound of Formula 53. A compound of Formula 53 is then condensed with a keto malonic ester of Formula 54 to afford an hydroxy triazine of Formula 55. The compounds of Formula 55 are converted to the intermediates of Formula 56 by treatment with a suitable chlorinating agent, for example phosphorous oxychloride.

Scheme IX

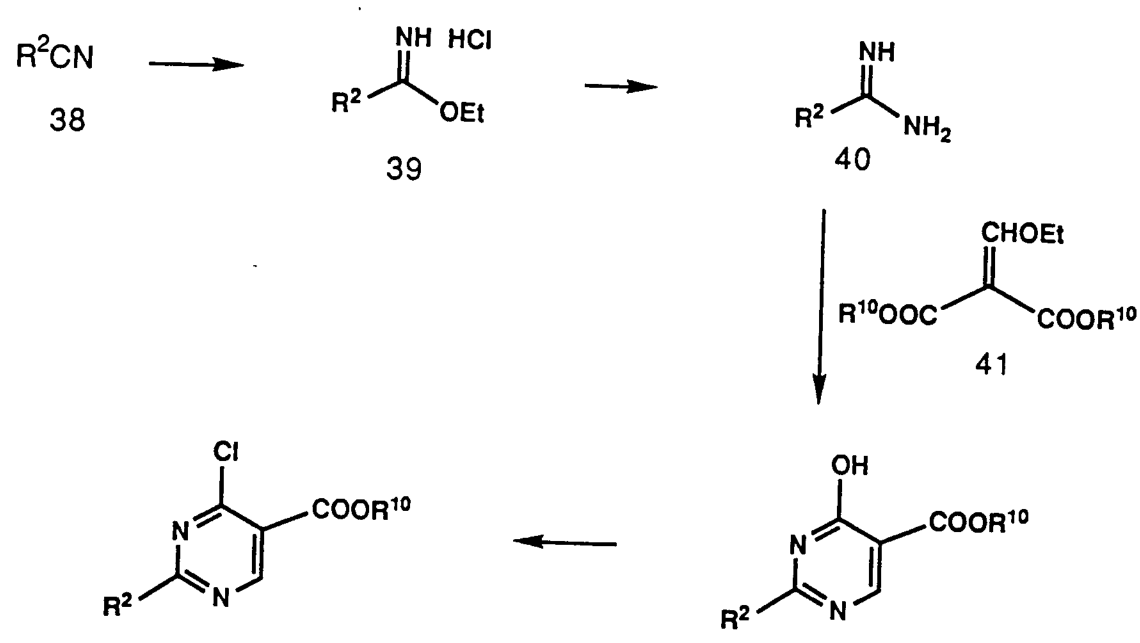

$R^2CN$

38

39

40

41

42

2: $R^3 = COOR^{10}$

Scheme X

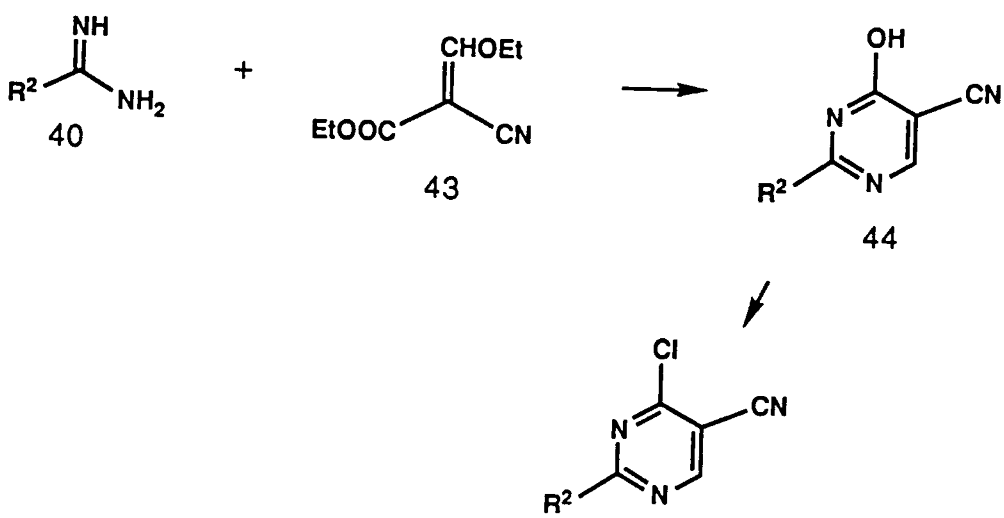

40

43

44

2: $R^3 = CN$

Scheme XI

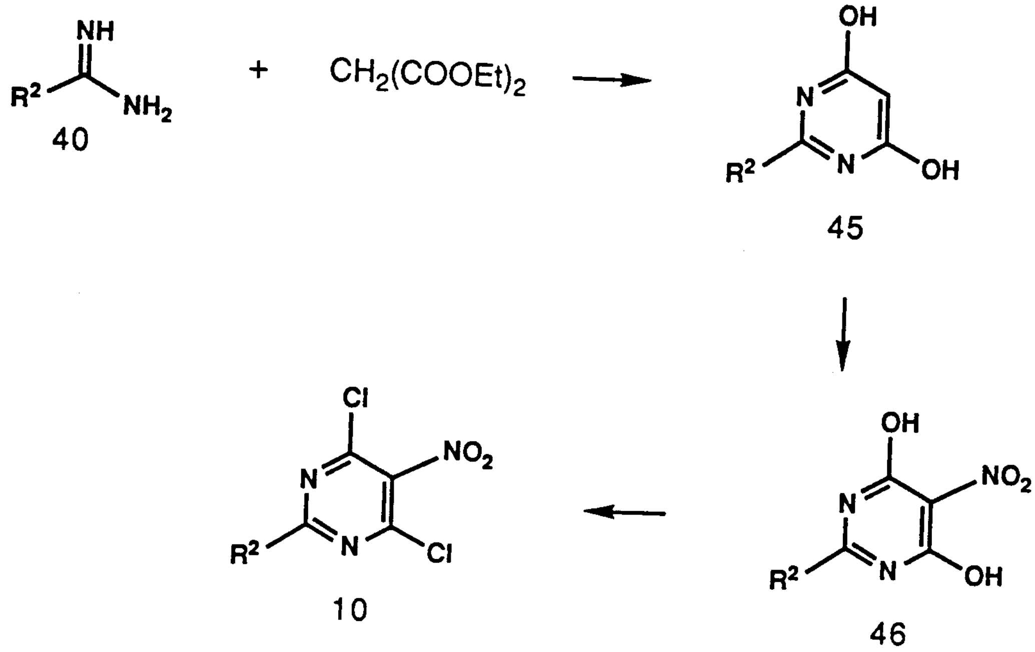

40 45 46 10

Scheme XII

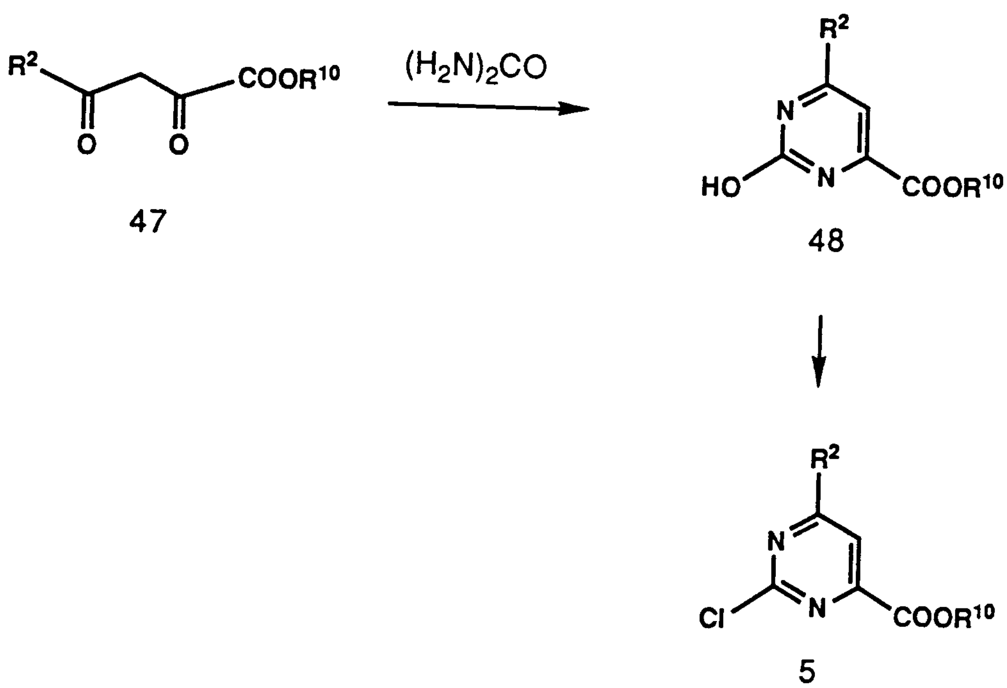


47 48 5

Scheme XIII

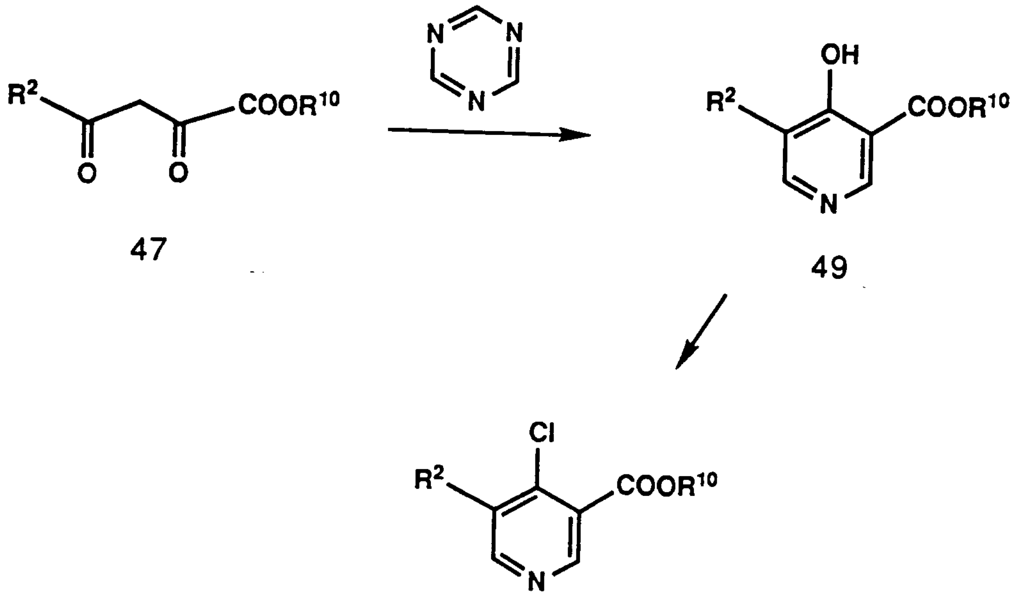

47

49

7

Scheme XIV

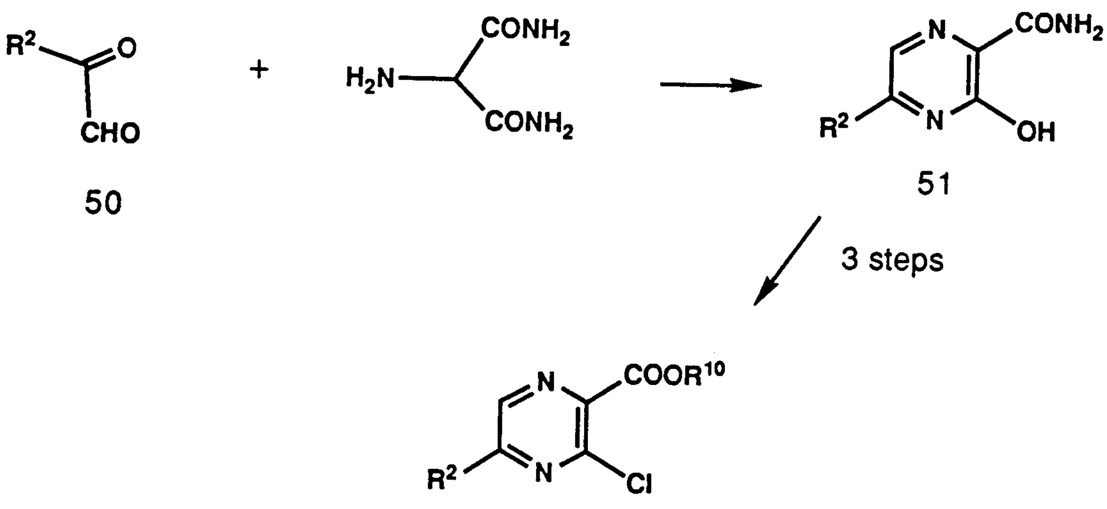


50

51

8

Scheme XV

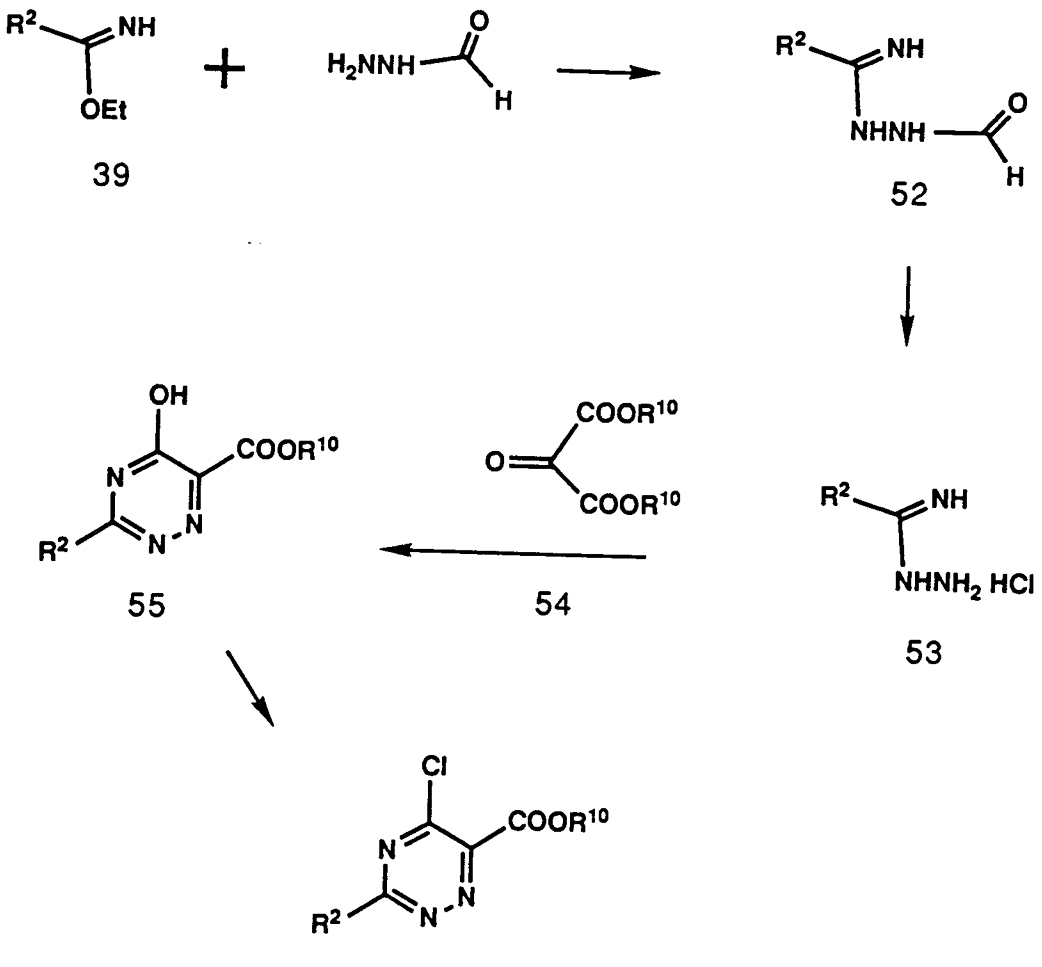

39 52 53 54 55 56

A general process for the preparation of the compounds of the invention is illustrated in Scheme XVI. Reaction of **60** with aryl chloride **61** provides **62.**

An alternative process for the preparation of the compounds of the invention wherein $R^1$ is tetrazolyl is illustrated in Scheme XVII. Amine **70** is reacted with aryl chloride **71** to provide **72.** Reaction of **72** with $POCl_3$ gives nitrile **72,** which can be converted to tetrazole **73.**

SCEME XVI

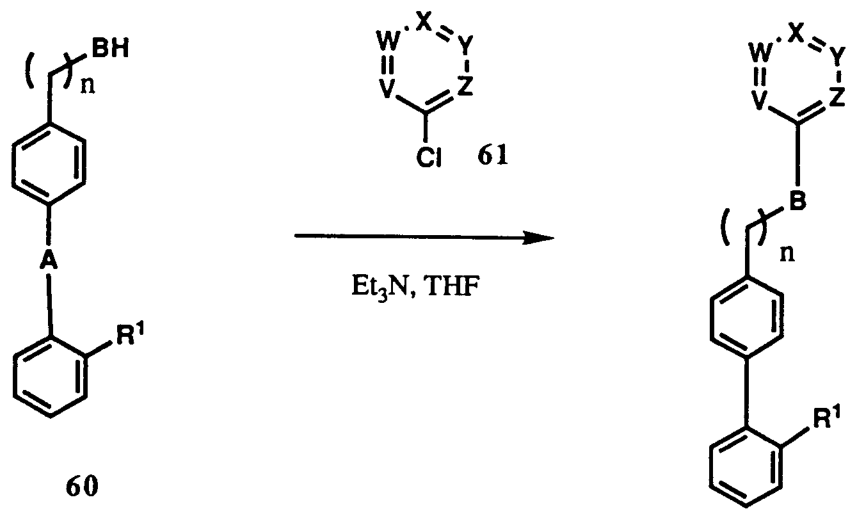


60

61

62

SCEME XVII

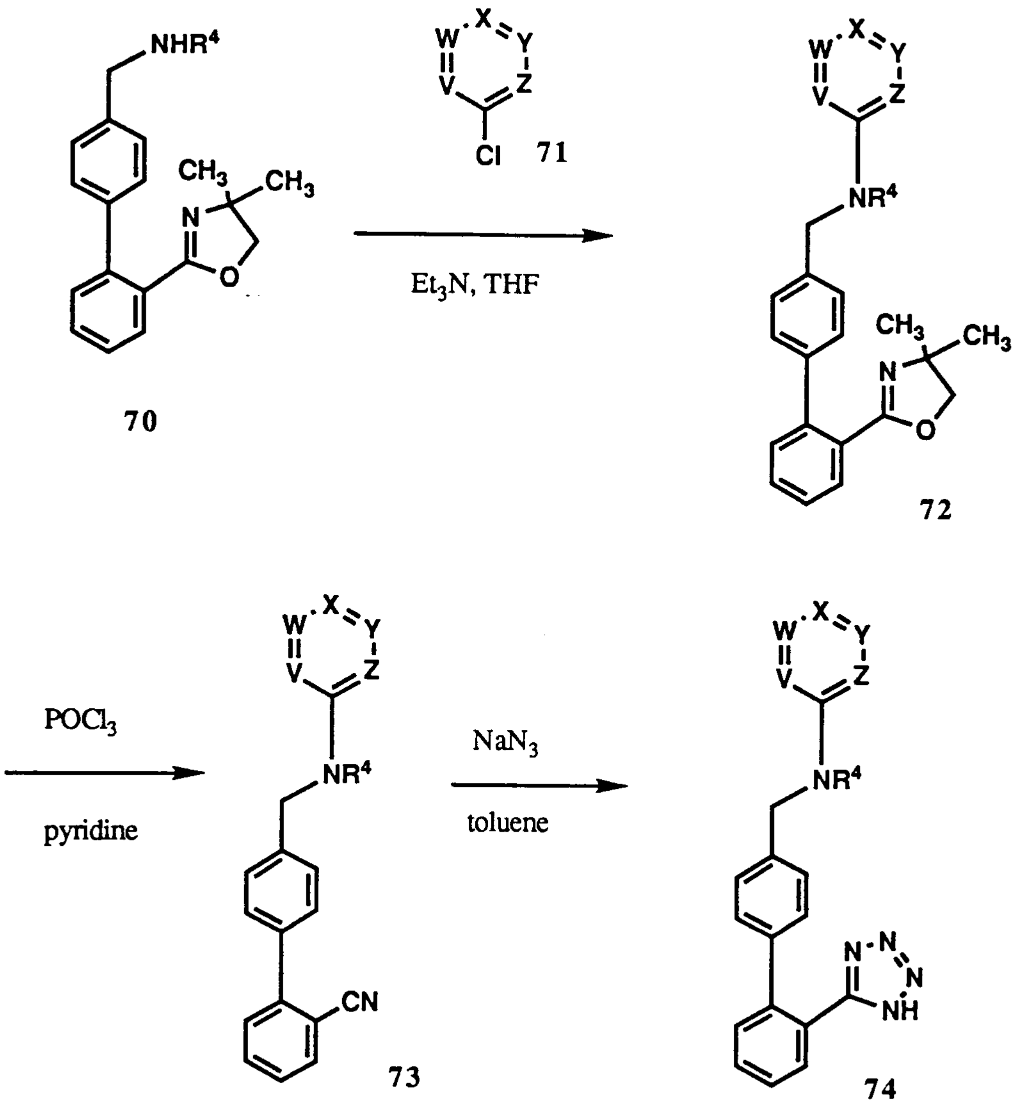


70 71 72 73 74

The foregoing may be better understood from the following examples, which are presented for the purpose of illustration and not intended to limit the scope of the inventive concept.

Example 1

Ethyl 2-n-butyl-4-{N-[(2'-[1H-tetrazol-5-yl]biphenyl-4-yl)methyl]amino}pyrimidine-5-carboxylate

Example 1A

N-Triphenylmethyl-5-[2-(4'-azidomethyl-biphenyl)]tetrazole

N-Triphenylmethyl-5-[2-(4'-bromomethyl)biphenyl)]tetrazole (3.909 g, 7.02 mmol), prepared as described by P.E. Aldrich, *et al.* in European Patent Application Number 291,969, published November 23, 1988 (Example 6), was dissolved in 11 mL of N,N-dimethylformamide (DMF). Sodium azide (1.16 g, 17.8 mmol) was added and the reaction mixture was stirred for 16 h at ambient temperature. Ice water was added and the resulting precipitate was filtered. The solid was dissolved in chloroform and the chloroform solution was dried over anhydrous sodium sulfate, filtered and concentrated *in vacuo*. The residue was crystallized from diethyl ether/hexane (2:1) to give 3.24 g (89% yield) of the title compound, m.p. 142-145° C.

Example 1B

N-Triphenylmethyl-5-[2-(4'-aminomethylbiphenyl)]tetrazole

To the compound resulting from Example 1A (1.0 g, 1.93 mmol) dissolved in tetrahydrofuran (14 mL) under nitrogen and cooled in an ice bath was added lithium aluminum hydride (0.173 g). The reaction mixture was stirred for 30 minutes at 0 °C and then quenched with water (0.5 mL) added dropwise followed by 15% sodium hydroxide (0.5 mL). After stirring for 15 minutes, the solids were removed by filtration and washed with tetrahydrofuran. The filtrate was concentrated under reduced pressure to give the title compound as a solid.

Example 1C

Ethyl 2-n-butyl-4-{N-[(2'-[N-triphenylmethyl-tetrazol-5-yl]biphenyl-4-yl)methyl]amino}pyrimidine-5-carboxylate

The compound resulting from Example 1B was dissolved in 4 mL of THF containing 0.50 g of triethylamine. Ethyl 2-n-butyl-4-chloropyrimidine-5-carboxylate (0.40 g, 1.65 mmol), prepared as described by H. Yamanaka, *et al*. in *Heterocycles*, **12**, 1323-6 (1979), was added and the reaction mixture was stirred at ambient temperature for 1 h. Chloroform was added and the resultant solution was washed with aqueous potassium bicarbonate solution, dried over anhydrous sodium sulfate, filtered and concentrated *in vacuo* to an oil. The oil was chromatographed on silica gel eluting with 10% ethyl acetate in toluene to give 0.927 g of the title compound after crystallization from diethyl ether, m.p. 116-118° C.

Example 1D

Ethyl 2-n-butyl-4-{N-[(2'-[1H-tetrazol-5-yl]biphenyl-4-yl)methyl]amino}pyrimidine-5-carboxylate

The compound resulting from Example 1C (0.25 g, 0.358 mmol) was suspended in 3 mL of ethyl alcohol and three drops of concentrated hydrochloric acid was added. The resultant solution was stirred at ambient temperature for 45 minutes and then concentrated *in vacuo.* Cold water was added to the residue and the solution was neutralized by the addition of potassium acetate. The aqueous mixture was extracted with chloroform. The chloroform solution was dried over anhydrous sodium sulfate, filtered and concentrated *in vacuo*. The residue was crystallized from diethyl ether to give 0.123 g (75% yield) of the title compound, m.p. 153-155° C; $^{1}$H NMR ($CDCl_3$) $\delta$ 0.87 (t, 1 H, J = 7 Hz), 1.31 (m, 2H), 1.35 (t, 3H, J = 7 Hz), 1.62 (m, 2H), 2.51 (t, 2H, J = 7 Hz), 3.48 (q, 2H, J = 7 Hz), 4.30 (q, 2H, J = 7 Hz), 4.81 (d, 1H, J = 6 Hz), 7.00 (d, 2H, J = 9 Hz), 7.20 (d, 2H, J = 9 Hz), 7.40 (dd, 1H), 7.57 (m, 3H), 8.00 (dd, 1H), 8.34 (s, 1H), 8.65 (t, 1H, J = 6 Hz).

Example 2

2-n-Butyl-5-hydroxymethyl-4-{N-[(2-[1H-tetrazol-5-yl]biphenyl-4-yl)methyl]amino}pyrimidine hydrochloride

Example 2A

2-n-Butyl-5-hydroxymethyl-4-{N-[(2-[N-triphenylmethyl-tetrazol-5-yl]biphenyl-4-yl)methyl]amino}pyrimidine

The compound resulting from Example 1C (1.0 g, 1.43 mmol) was dissolved in 15 mL of dry THF. The resultant solution was cooled in an ice bath and 0.22 g (5.79 mmol) of lithium aluminum hydride was added to the cooled solution under a nitrogen atmosphere. After 45 minutes 50 mL of THF was added, followed by the dropwise addition of 0.8 mL of water and 0.5 mL of 15% aqueous sodium hydroxide solution. After stirring the mixture for 15 minutes, the solids were filtered and the filtrate was concentrated *in vacuo*. The residue was taken up in chloroform and the solution was washed with dilute aqueous potassium hydroxide solution, dried over anhydrous sodium sulfate, filtered and concentrated *in vacuo*. The residue was crystallized from diethyl ether to give 0.686 g (73% yield) of the title compound, m.p. 126-129° C.

Example 2B

2-n-Butyl-5-hydroxymethyl-4-{N-[(2-[1H-tetrazol-5-yl]biphenyl-4-yl)methyl]amino}pyrimidine hydrochloride

The compound resulting from Example 2A (0.184 g, 0.28 mmol) was suspended in 2.5 mL of ethyl alcohol and four drops of a concentrated solution of hydrogen chloride in isopropyl alcohol was added. The resultant solution was stirred at ambient temperature for 45 minutes and then concentrated *in vacuo.* The residue was crystallized from isopropylalcohol/diethyl ether to give 118 mg (93% yield) of the title compound as crystals, m.p. 215-216 °C; $^1H$ NMR ($CD_3OD$)$\delta$0.92 (t, 3 H, J = 7 Hz), 1.37 (m, 2H), 1.74 (m, 2H), 2.80 (t, 2H, J = 7 Hz), 4.55 (s, 2H), 4.82 (s, 2H), 7.10 (d, 2H, J = 7 Hz), 7.32 (d, 2H, J = 7 Hz), 7.50-7.70 (m, 4H), 8.00 (s, 1H).

Example 3

Ethyl 2-n-propyl-4-{N-[(2'-(1H-tetrazol-5-yl)biphenyl-4-yl}methyl]amino}pyrimidine-5-carboxylate

Example 3A

Ethyl 2-n-propyl-4-{N-[(2'-(N-triphenylmethyl-1H-tetrazol-5-yl)biphenyl-4-yl}methyl]amino}pyrimidine-5-carboxylate

The compound resulting from Example 1B (4.70 g, 9.53 mmol) and ethyl 2-n-propyl-4-chloropyrimidine-5-carboxylate, prepared as described by H. Yamanaka *et al* in *Heterocycles*, **12**, 1323 (1979), were reacted by the method described in Example 1C to give the title compound (4.71 g). m.p. 132-133 °C.

Example 3B

Ethyl 2-n-propyl-4-{N-[(2'-(1H-tetrazol-5-yl)biphenyl-4-yl}methyl]amino}pyrimidine-5-carboxylate

By the procedure described in Example 1D, the compound resulting from Example 3A (400 mg, 0.584 mmol) was treated with hydrochloric acid in ethanol to give the title compound (175 mg). m.p. 146-147 °C. $^1H$ NMR ($CDCl_3$, 300 MHz) $\delta$ 0.90 (t, J = 7Hz, 3H), 1.33 (t, J = 7Hz, 3H), 1.58 (m, 2H), 2.49 (t, J = 7Hz, 2H), 4.30 (q, J = 7Hz, 2H), 4.81 (d, J = 6Hz, 2H), 7.00 (d, J = 9Hz, 2H), 7.20 (d, J = 9Hz, 2H), 7.40 (dd, 1H), 7.57 (m, 3H), 8.00 (dd, 1H), 8.34 (s, 1H), 8.65 (t, J = 6Hz, 1H).

Example 4

Ethyl 2-n-pentyl-4-{N-[(2'-(1H-tetrazol-5-yl)biphenyl-4-yl}methyl]amino}pyrimidine-5-carboxylate

Example 4A

Ethyl 2-n-Pentyl-4-hydroxy-pyrimidine-5-carboxylate

Hexanecarboxamidine hydrochloride (22.36 g, 0.148 mol) and diethyl ethoxymethylene malonate (31.08 g, 0.143 mol) were dissolved in ethanol (70 mL) in an ice bath. A solution of sodium ethoxide, prepared from dissolving sodium (6.65 g, 0.289 mol) in ethanol (120 mL), was added slowly. The resulting solution was heated at reflux for 2 hours and then concentrated *in vacuo.* Ether and water (50 mL) were added and the resulting sodium salt was removed by filtration and washed with ether. It was suspended in water and acidified with acetic acid to give the title compound (19.69 g). m.p. 127-129 °C after crystallization from chloroform/ether.

Example 4B

Ethyl 2-n-pentyl-4-chloro-pyrimidine-5-carboxylate

To the compound resulting from Example 4A suspended in phosphorus oxychloride (160 mL) was added triethylamine (8.32 g). The reaction mixture was warmed at 45 °C for 30 minutes. The phosphorus oxychloride was removed under reduced pressure, toluene was added and then also removed under reduced pressure. The residue obtained was dissolved in toluene, washed with water and sodium bicarbonate solution, dried over magnesium sulfate and concentrated under reduced pressure. The crude

product was chromatographed on silica gel eluting with methylene chloride to give the title compound (18.27 g).

Example 4C

Ethyl 2-n-pentyl-4-{N-[(2'-(N-triphenylmethyl-1H-tetrazol-5-yl)biphenyl-4-yl}methyl]amino}pyrimidine-5-carboxylate

The compound resulting from Example 1B (2.97 g, 6.02 mmol) and the compound resulting from Example 4B 1.05 g) were reacted by the procedure described in Example 1C to give the title compound (1.38 g). m.p. 107-108 °C.

Example 4D

Ethyl 2-n-pentyl-4-{N-[(2'-(1H-tetrazol-5-yl)biphenyl-4-yl}methyl]amino}pyrimidine-5-carboxylate

The compound resulting from Example 4C (300 mg, 0.421 mmol) was treated with hydrochloric acid in ethanol by the procedure described in Example 1D to give the title compound (130 mg). m.p. 131-132. $^1H$ NMR $CDCl_3$, 300 MHz) $\delta$ 0.82 (t, J = 7Hz, 3H), 1.25 (m, 4H), 1.35 (t, J = 7Hz, 3H), 1.65 (m, 2H), 2.50 (t, J = 7Hz, 2H), 4.30 (t, J = 7Hz, 2H), 4.80 (d, J = 7Hz, 2H), 7.00 (d, J = 8Hz, 2H), 7.20 (d, J = 8Hz, 2H), 7.40 (dd, 1H), 7.57 (m, 3H), 8.00 (dd, 1H), 8.34 (s, 1H), 8.65 (t, J = 6Hz, 1H).

Examples 5-10

Following the procedures described in Example 4, replacing hexanenitrile with the appropriate nitrile, the intermediate 4-chloropyrimidine-5-carboxylatecompounds are prepared. These intermediates are then reacted, according to the procedures described in Example 1C, with N-triphenylmethyl-5-[2-(4'-aminometh yl-biphenyl]tetrazole and the triphenylmethyl group is removed as described in Example 1D to give the compounds of Examples 5 - 10 as disclosed below in Table 1.

Table 1

| Example No. | Compound name |
|---|---|
| 5 | Ethyl 2-(1-methylbutyl)-4-{N-[(2'-[1H-tetrazol-5-yl]biphenyl-4-yl)methyl]amino}pyrimidine-5-carboxylate |
| 6 | Ethyl 2-(1,1-dimethylbutyl)-4-{N-[(2'-[1H-tetrazol-5-yl]biphenyl-4-yl)methyl]amino}pyrimidine-5-carboxylate |
| 7 | Ethyl 2-(3-methylbutyl)-4-{N-[(2'-[1H-tetrazol-5-yl]biphenyl-4-yl)methyl]amino}pyrimidine-5-carboxylate |
| 8 | Ethyl 2-[2-(4'fluorophenyl)ethyl]-4-{N-[(2'-[1H-tetrazol-5-yl]biphenyl-4-yl)methyl]amino}pyrimidine-5-carboxylate |
| 9 | Ethyl 2-(2-ethoxyethyl)-4-{N-[(2'-[1H-tetrazol-5-yl]biphenyl-4-yl)methyl]amino}pyrimidine-5-carboxylate |
| 10 | Ethyl 2-(2-ethylthioethyl)-4-{N-[(2'-[1H-tetrazol-5-yl]biphenyl-4-yl)methyl]amino}pyrimidine-5-carboxylate |

Example 11

2-n-Butyl-4-{N-[(2'-carboxy-biphenyl-4-yl)methyl]amino}-5-carboethoxypyrimidine

Example 11A

t-Butyl 4'-aminomethyl-biphenyl-2-carboxylate

Following the procedures described in Example 1A, replacing N-triphenylmethyl-5-[2-(4'-bromomethyl)-biphenyl)]tetrazole with t-butyl 4'-bromomethyl-biphenyl-2-carboxylate, prepared as described by D.J Carini, *et al.* in European Patent Application Number 324,377, published July, 19, 1989, the intermediate t-butyl 4'-azidomethyl-biphenyl-2-carboxylate is prepared. The intermediate azidomethyl compound is then hydrogenated as described in Example 1B, using palladium on carbon catalyst instead of Lindlar catalyst, to give the title compound.

Example 11B

2-n-Butyl-4-{N-[(2'-carboxy-biphenyl-4-yl)methyl]amino}-5-carboethoxypyrimidine

t-Butyl 4'-aminomethyl-biphenyl-2-carboxylate from Step 1 is reacted, by the method described in Step 3 of Example 1, with ethyl 2-n-butyl-4-chloropyrimidine-5-carboxylate (0.40 g, 1.65 mmol) to give 2-n-butyl-4-{N-[(2'-t-butoxycarbonyl-biphenyl-4-yl)methyl]amino]-5-carboethoxypyrimidine. The t-butyl protecting group is removed by treatment with trifluoroacetic acid in methylene chloride at ambient temperature to afford the title compound.

Example 12

Ethyl 2-n-butyl-4-{N-[(2'-[1H-tetrazol-5-yl]biphenyl-4-yl)]amino}pyrimidine-5-carboxylate

Example 12A

N-Triphenylmethyl-5-[2-(4'-amino-biphenyl]tetrazole

4'-Nitrobiphenyl-2-carbonitrile, prepared as described by B. Sain and J.S. Sandhu in *J. Organic Chem.*, **55**, 2545-6 (1990), is heated with triethylamine hydrochloride and sodium azide in N-methyl-2-pyrrolidone at 150 ° C as described by P.R. Bernstein and E.P. Vacek in *Synthesis*, 1133 (1987), to give the intermediate 5-[2-(4'-nitro)biphenyl]tetrazole. This intermediate is treated with triphenylmethyl chloride in methylene chloride to give N-triphenylmethyl-5-[2-(4'-nitro)biphenyl)]tetrazole which is reduced by hydrogenation to give the title compound.

Example 12B

Ethyl 2-n-butyl-4-{N-[(2'-[1H-tetrazol-5-yl]biphenyl-4-yl)]amino}pyrimidine-5-carboxylate

By the procedures described in Example 1C and 1D, N-triphenylmethyl-5-[2-(4'-amino)biphenyl]-tetrazole from Example 1C is reacted, with ethyl 2-n-butyl-4-chloropyrimidine-5-carboxylate and the condensation product is deprotected to afford the title compound.

Example 13

Ethyl 2-Butyl-4-{N-methyl-N-(2'-[1H-tetrazol-5-yl]biphenyl-4-yl)]amino}pyrimidine-5-carboxylate

Example 13A

N-Triphenylmethyl-5-[2-(4'-methylamino)biphenyl]]tetrazole

The compound resulting from Example 12A, is refluxed with ethyl formate and toluene to give the intermediate N-triphenyl-5-[2-(4'-formylamino)biphenyl]tetrazole. This intermediate is then reduced with lithium aluminum hydride to afford the title compound.

Example 13B

Ethyl 2-butyl-4-{N-[methyl, (2'-[1H-tetrazol-5-yl]biphenyl-4-yl)amino}pyrimidine-5-carboxylate

By the procedures described in Steps 3 and 4 of Example 1, N-triphenyl-5-[2-(4'-aminomethyl)-biphenyl)]tetrazole from Step 1 is reacted with ethyl 2-n-butyl-4-chloropyrimidine-5-carboxylate and the condensation product is deprotected to afford the title compound.

Example 14

2-Butyl-4-{N-[(2'-[1H-tetrazol-5-yl]biphenyl-4-yl)methyl]amino}pyrimidine-5-carbonitrile

Example 14A

]2-Butyl-4-chloropyrimidine-5-carbonitrile

Pentanecarboxamidine and ethyl (ethoxymethylene)cyanoacetate (commercially available from Aldrich Chemical Co.) are reacted according to the method of S. Nishigaki, *et al*. in *Chem Pharm. Bull*., **18**, 1003 (1970), to give 2-butyl-4-hydroxypyrimidine-5-carbonitrile which is then reacted with phosphorous oxychloride, according to the method of A.R. Todd and F. Bergel in *J. Chem. Soc*., 364- (1937), to give the title compound.

Example 14B

2-Butyl-4-{N-[(2'-[1H-tetrazol-5-yl]biphenyl-4-yl)methyl]amino}pyrimidine-5-carbonitrile

By the procedures described in Example 1C and 1D, N-tri-phenylmethyl-5-[2-(4'-aminomethyl)biphenyl)-]tetrazole, the product of Example 1B, is reacted with 2-butyl-4-chloropyrimidine-5-carbonitrile and the condensastion product is deprotected to give the title compound.

Example 15

2-Butyl-4-{N-[(2'-[1H-tetrazol-5-yl]biphenyl-4-yl)methyl]amino}pyrimidine-5-carboxamide

2-Butyl-4-{N-[(2'-[1H-tetrazol-5-yl]biphenyl-4-yl)methyl]amino}pyrimidine-5-carbonitrile, the product of Example 14, is heated in dilute aqueous potassium hydroxide. Neutralization with acetic acid affords the title compound.

Example 16

2-Butyl-5-methoxymethyl-4-{N-[(2'-[1H-tetrazol-5-yl]biphenyl-4-yl)methyl]amino}pyrimidine

2-n-Butyl-5-hydroxymethyl-4-{N-[(2-1H-tetrazol-5-yl]biphenyl-4-yl)methyl]amino}pyrimidine hydrochloride, the product of Example 2B, is converted to 2-butyl-5-bromomethyl-4-{N-[(2'-[1H-tetrazol-5-yl]biphenyl-4-yl)methyl]amino}pyrimidine by treatment with hydrogen bromide in acetic acid according to the method of A. Schellenberger and K. Winter in *Z. Physiol. Chem*., **322**, 164 (1960). The bromomethyl intermediate is then converted to the title compound by treatment with sodium methoxide in methanol.

Example 17

2-Butyl-4-{N-[(2'-[1H-tetrazol-5-yl]biphenyl-4-yl)methyl]amino}pyrimidine-5-carboxylic acid hydrochloride

To a solution of 0.300 g (0.43 mmol) of ethyl 2-n-butyl-4-{N-[(2'-[N-triphenylmethyl-tetrazol-5-yl]-biphenyl-4-yl)methyl]amino}pyrimidine-5-carboxylate (0.25 g, 0.358 mmol), the product of Example 1C, in 3

mL of tetrahydrofuran and 2 mL of methanol, was added a solution of 90 mg of lithium hydroxide hydrate in 0.5 mL of water. After 1 h at ambient temperature, 14 drops of concentrated hydrochloric acid was added. After another hour, the solution was concentrated *in vacuo* and the residue was treated with cold water and diethyl ether. The resultant solid was filtered and then suspended in acetonitrile containing isopropyl alcohol saturated with hydrogen chloride. The resultant solid was collected by filtration to give 0.19 g (95% yield) of the title compound, m.p. 193-195 °C; $^1$H NMR ($CD_3OD$) δ 0.92 (t, 2H, J = 7 Hz), 1.38 (m, 2H), 1.75 (m, 2H), 2.51 (t, 2H, J = 7 Hz), 4.86 (s, 1H), 7.09 (d, 2H, J = 8 Hz), 7.32 (d, 2H, J = 8 Hz), 7.50-7.70 (m, 4H), 8.60 (s, 1H).

Example 18

Ethyl 2-n-Butyl-4-{N-methyl-N-[(2'-[1H-tetrazol-5-yl]biphenyl-4-yl)methyl]amino}pyrimidine-5-carboxylate

Example 18A

N-Triphenylmethyl-5-[2-(4'-methylaminomethyl-biphenyl)]tetrazole

To N-Triphenylmethyl-5-[2-{4'-bromomethyl-biphenyl)]tetrazole (3.00 g, 5.39 mmol), prepared as described by P.E. Aldrich *et al* in European Patent Application Number 291965, November 23, 1988, dissolved in tetrahydrofuran (75 mL) was added a solution of 40% methylamine in water (35 mL). The resulting solution was stirred at room temperature for 2 hours and then concentrated *in vacuo*. The residue obtained was dissolved in chloroform, washed with dilute potassium hydroxide solution, dried over potassium carbonate, and concentrated under reduced pressure to give the title compound.

Example 18B

Ethyl 2-n-Butyl-4-{N-methyl-N-[(2'-[N-triphenylmethyl-1H-tetrazol-5-yl]biphenyl-4-yl)methyl]-amino}pyrimidine-5-carboxylate

The product resulting from Example 18A (5.39 mmol) was dissolved in tetrahydrofuran (15 mL) containing triethylamine (2.75 mL). Ethyl 2-n-butyl-4-chloropyrimidine-5-carboxylate (1.31 g, 5.39 mmol), prepared as described by H. Yamanaka *et al* in *Heterocycles*, **12**, 1323 (1979), was added and the solution was stirred at room temperature for 2 hours. The solution was concentrated under reduced pressure and the residue obtained dissolved in toluene, washed with potassium bicarbonate solution, dried over sodium sulfate and concentrated *in vacuo*. The crude product was chromatographed on silica gel eluting with 8% ethyl acetate in toluene to give the title compound (2.826 g). m.p. 134-137 °C dec (crystallized from 1:1 ether/heptane).

Example 18C

Ethyl 2-n-Butyl-4-{N-methyl-N-[(2'-[1H-tetrazol-5-yl]biphenyl-4-yl)methyl]amino}pyrimidine-5-carboxylate

To the compound resulting from Example 18B (330 mg, 0.463 mmol) dissolved in ethanol (4 mL) was added concentrated hydrochloric acid (0.25 mL). The reaction mixture was stirred 1.5 hours at room temperature and then concentrated under reduced pressure. The residue obtained was dissolved in water and treated with potassium acetate until neutral pH was obtained. The mixture was extracted with chloroform and the combined organic extracts dried over sodium sulfate and concentrated *in vacuo*. Trituration with ether afforded the title compound m.p. 159-161 °C. $^1$H NMR ($CDCl_3$, 300 MHz) δ 0.82 (t, J = 7Hz, 3H), 1.22 (m, 2H), 1.31 (t, J = 7Hz, 3H), 1.57 (m, 2H), 2.38 (t, J = 7Hz, 2H) 2.70 (s, 3H), 4.29 (q, J = 7Hz, 2H), 4.93 (s, 2H), 6.95 (d, J = 8Hz, 2H), 7.05 (d, J = 8Hz, 2H), 7.42 (dd, 1H), 7.50-7.65 (m, 2H), 7.92 (dd, 1H), 8.04 (s, 1H).

Example 19

Ethyl 2-methyl-4-{N-butyl-N-[(2'-[1H-tetrazol-5-yl]biphenyl-4-yl)methyl]amino}pyrimidine-5-carboxylate

Example 19A

N-Triphenylmethyl-5-[2-(4'-butylaminomethyl-biphenyl)]tetrazole

To N-Triphenylmethyl-5-[2-(4'-bromomethyl-biphenyl)]tetrazole (6.00 g, 10.7 mmol), prepared as described by P.E. Aldrich *et al* in European Patent Application Number 291,969, published November 23, 1988, dissolved in tetrahydrofuran (55 mL) was added butylamine (40 mL). The reaction mixture was stirred at room temperature for 2 hours and then concentrated *in vacuo*. The residue obtained was dissolved in chloroform, washed with dilute potassium hydroxide solution, dried over potassium carbonate and concentrated under reduced pressure to afford the title compound.

Example 19B

Ethyl 2-methyl-4-{N-butyl-N-[(2'-[N-triphenylmethyl-1H-tetrazol-5-yl]biphenyl-4-yl)methyl]amino}pyrimidine-5-carboxylate

The compound resulting from Example 19A (10.7 mmol) was dissolved in tetrahydrofuran (15 mL) containing triethylamine (4.6 mL). A solution of ethyl 2-methyl-4-chloropyrimidine-5-carboxylate (1.94 g, 9.7 mmol), prepared as described by A.W. Spears and H. Tieckelmann in *J. Org. Chem.*, **25**, 2137 (1960), dissolved in tetrahydrofuran (2 mL) was added and the reaction mixture stirred at room temperature for 2 hours. The reaction mixture was concentrated under reduced pressure and the residue dissolved in toluene, washed with postassium bicarbonate, dried over sodium sulfate and concentrated under reduced pressure. The crude material was chromatographed on silica gel eluting with 12% ethyl acetate in toluene to afford the title compound (4.97 g), which was crystallized from 1:1 ether/heptane. m.p. 130-132 °C.

Example 19C

Ethyl 2-methyl-4-{N-butyl-N-[(2'-[1H-tetrazol-5-yl]biphenyl-4-yl)methyl]amino}pyrimidine-5-carboxylate

The compound resulting from Example 19B (3.50 g, 4.91 mmol) was deprotected by the procedure described in Example 18C to give the title compound (2.08 g). m.p. 164-166 °C. $^1H$ NMR ($CDCl_3$, 300 MHz) $\delta$ 0.85 (t, J = 7Hz, 3H), 1.25 (m, 2H), 1.32 (t, J = 7Hz, 3H), 4.30 (q, J = 7Hz, 2H), 1.50 (m, 2H), 2.24 (s, 3H), 3.38 (t, J = 7Hz, 2H), 4.30 (q, J = 7Hz, 2H), 4.73 (s, 2H), 7.00 (m, 4H), 7.42 (dd, J = 8Hz, 1Hz, 1H), 7.50-7.62 (m, 2H), 7.75 (dd, J = 8Hz, 1Hz, 1H)., 8.02 (s, 1H).

Examples 20 - 22

Following the procedures described in Example 1C and 1D, the intermediates shown in Table 2 are condensed and the condensation products deprotected to give the compounds of Examples 20, 21 and 22 as disclosed below in Table 2. The intermediates are prepared by literature methods.

Table 2: Examples 20 - 22

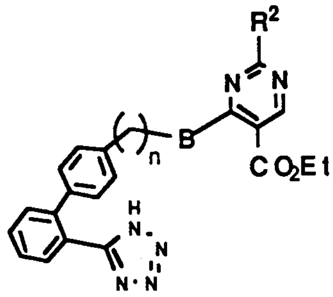

| Example No. | B | R | n | Intermediate 1 | Intermediate 2 |
|---|---|---|---|---|---|
| 20 | -S- | -nButyl | 0 | 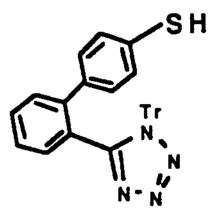 | 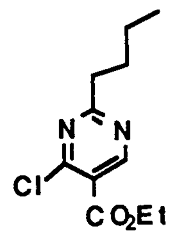 |
| 21 | -O- | -nButyl | 1 | 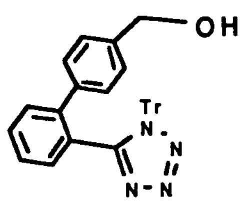 | 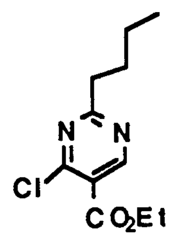 |
| 22 | -O- | -nButyl | 0 | 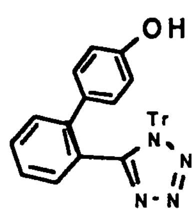 | 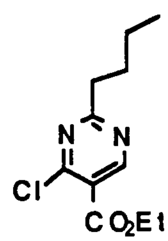 |

Example 23

2-Butyl-5-[N-pyrrolidinylcarbonyl]-4-{N-[(2'-[1H-tetrazol-5-yl]biphenyl-4-yl)methyl]amino}pyrimidine

Ethyl 2-butyl-4-{N-[(2'-N-triphenylmethyl-tetrazol-5-yl]biphenyl-4-yl)methyl]amino}pyrimidine-5-carboxylate is hydrolyzed to the corresponding acid as described in Example 17. The lithium hydroxide is neutralized with acetic acid instead of hydrochloric acid to avoid removing the triphenylmethyl protecting group from the tetrazole ring. The carboxylic acid (1 mmol) is dissolved in DMF (10 mL) and the following reagents are sequentially added: N-methylmorpholine (1.1 equivalents), 3-(dimethylaminopropyl)-3-ethylcarbodiimide hydrochloride (1.1 equivalents), and pyrrolidine (1.1 equivalents). After stirring for 16 h at ambient temperature, the reaction mixture is diluted with ethyl acetate and washed with dilute aqueous sodium bicarbonate solution, dried over anhydrous sodium sulfate, filtered and concentrated *in vacuo* to give 2-butyl-5-[N-pyrrolidinylcarbonyl]-4-{N-[(2'-[N-triphenylmethyl-tetrazol-5-yl]biphenyl-4-yl)methyl]-amino}pyrimidine. The triphenylmethyl group is removed, as described in Step 4 of Example 1, to give the title compound.

Examples 24 - 30

Following the procedures of Example 23, replacing pyrrolidine with the appropriate amine (commercially available from Aldrich Chemical Company or readily prepared according to published methods), Examples 24 - 30 are prepared as disclosed in Table 3.

Table 3 Examples 24 - 30

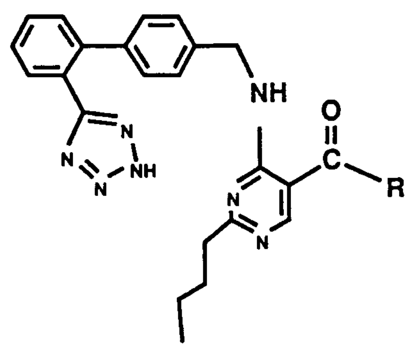

| Example Number | R |
|---|---|
| 24 | 1-morpholino |
| 25 | 4-methoxymethoxy-1-piperidinyl |
| 26 | $-N(CH_3)_2$ |
| 27 | $-NHCH_3$ |
| 28 | $-NHCH_2CH_2OH$ |
| 29 | $-NHCH_2CH_2OCH_3$ |
| 30 | $-NH(CH_2)_2O(CH_2)_2OCH_3$ |

Example 31

2-Butyl-4-{N-[(2'-[1H-tetrazol-5-yl]biphenyl-4-yl)methyl]amino}pyrimidine-5-carboxaldehyde

2-Butyl-5-hydroxymethyl-4-{N-[(2'-[N-triphenylmethyl-tetrazol-5-yl]biphenyl-4-yl)methyl]-amino}pyrimidine, the product of Example 2A, is dissolved in methylene chloride. Excess activated manganese dioxide is added and the reaction mixture is stirred at ambient temperature until the reaction is complete according to TLC analysis. The reaction mixture is then filtered and the filtrate is concentrated *in vacuo* to give the intermediate 2-butyl-4-{N-[(2'-[N-triphenylmethyl-tetrazol-5-yl]biphenyl-4-yl)methyl]-amino}pyrimidine-5-carboxaldehyde. The triphenylmethyl group is removed, as described in Example 1D, to give the title compound.

Example 32

5-Aminomethyl-2-butyl-4-{N-[(2'-[1H-tetrazol-5-yl]biphenyl-4-yl)methyl]amino)pyrimidine

2-Butyl-4-{N-[(2'-[N-triphenylmethyl-tetrazol-5-yl]biphenyl-4-yl)methyl]amino}pyrimidine-5-carbonitrile, the intermediate in Step 2 of Example 14, is reduced with excess lithium aluminum hydride to afford 5-aminomethyl-2-butyl-4-{N-[(2'-[N-triphenylmethyl-tetrazol-5-yl]biphenyl-4-yl)methyl]amino}pyrimidine. The triphenylmethyl group is removed, as described in Step 4 of Example 1, to give the title compound.

Example 33

2-Butyl-5-[methylaminocarbonylaminomethyl]-4-{N-[(2'-[1H-tetrazol-5-yl]biphenyl-4-yl)methyl]-amino}pyrimidine

5-Aminomethyl-2-butyl-4-{N-[(2'-[N-triphenylmethyl-tetrazol-5-yl]biphenyl-4-yl)methyl)amino}pyrimidine, the intermediate from Example 32 is dissolved in THF and treated with one equivalent of methyl isocyanate. Evaporation of the solvent affords 2-butyl-5-[methylaminocarbonylaminomethyl]-4-{N-[(2'-[N-triphenylmethyl-5-yl]biphenyl-4-yl)methyl]amino}pyrimidine. The triphenylmethyl group is removed, as described in Step 4 of Example 1, to give the title compound.

Example 34

2-Butyl-5-[acetylaminomethyl]-4-{N-[(2'-[1H-tetrazol-5-yl]biphenyl-4-yl)methyl]amino}pyrimidine

5-Aminomethyl-2-butyl-4-{N-[(2'-[N-triphenylmethyl-tetrazol-5-yl]biphenyl-4-yl)methyl]amino}pyrimidine, the intermediate from Example 32, is dissolved in THF containing 1.1 equivalents of triethyl]amine and was treated with one equivalent of acetyl chloride. After the reaction is complete according to TLC analysis, the reaction mixture is washed with dilute aqueous sodium bicarbonate solution, dried and concentrated *in vacuo* to give 2-butyl-5-[acetylaminomethyl]-4-{N-[(2'-[N-triphenylmethyl-5-yl]biphenyl-4-yl)methyl]-amino}pyrimidine. The triphenylmethyl group is removed, as described in Example 1D, to give the title compound.

Example 35

2-Butyl-5-[benzoyloxymethyl]-4-{N-[(2'-[1H-tetrazol-5-yl]biphenyl-4-yl)methyl]amino}pyrimidine

2-n-Butyl-5-hydroxymethyl-4-{N-[(2-[N-triphenylmethyl-tetrazol-5-yl]biphenyl-4-yl)methyl]-amino}pyrimidine, the product of Example 2A, is dissolved in chloroform containing triethylamine. One equivalent of benzoyl chloride is added. After the reaction is complete according to TLC analysis, the reaction mixture is washed with dilute aqueous sodium bicarbonate solution, dried and concntrated *in vacuo* to give 2-butyl-5-[benzoyloxymethyl]-4-{N-[(2'-[N-triphenylmethyl-tetrazol-5-yl]biphenyl-4-yl)methyl]-amino}pyrimidine. The triphenylmethyl group is removed, as described in Step 4 of Example 1, to give the title compound.

Example 36

2-Butyl-5-[methanesulfonamidomethyl]-4-{N-[(2'-[1H-tetrazol-5-yl]biphenyl-4-yl)methyl]amino}pyrimidine

5-Aminomethyl-2-butyl-4-{N-[(2'-[N-triphenylmethyl-tetrazol-5-yl]biphenyl-4-yl)methyl]amino}pyrimidine, the intermediate from Example 32, is dissolved in THF containing triethylamine and treated with one equivalent of methanesulfonyl chloride. After the reaction is complete according to TLC analysis, the reaction mixture is washed with dilute aqueous sodium bicarbonate solution, dried and concentrated *in vacuo* to give 2-butyl-5-[methanesulfonamidomethyl]-4-{N-[(2'-[N-triphenylmethyl-tetrazol-5-yl]biphenyl-4-yl)methyl]amino}pyrimidine. The triphenylmethyl group is removed, as described in Example 1D, to give the title compound.

Example 37

2-Butyl-5-[1H-tetrazol-5-yl]-4-{N-[(2'-[1H-tetrazol-5-yl]biphenyl-4-yl)methyl]amino}pyrimidine

2-Butyl-4-{N-[(2'-[1H-tetrazol-5-yl]biphenyl-4-yl)methyl]amino}pyrimidine-5-carbonitrile, the product of Example 14B, is dissolved in N-methylpyrrolidone containing 3 equivalents of triethylamine hydrochloride and 6 equivalents of sodium azide. The reaction mixture is heated at 150 ° C for 10 h and then cooled to ambient temperature. It is then neutralized with dilute aqueous hydrochloric acid and filtered to afford the

title compound.

Example 38

Ethyl 2-butyl-4-{N-[methyl-(2'-trifluoromethylsulfonylamido-biphenyl-4-yl)methyl]amino}pyrimidine-5-carboxylate

4-Bromomethyl-2-nitrobiphenyl, prpepared as described by D.J. Carini, *et al*. D.J. Carini, *et al*. on page 105 in European Application Number 324,377 published July, 19, 1989, is reacted with excess methylamine in THF in a manner similar to that described in Example 18A, to give 4'-methylaminomethyl-2-nitrobiphenyl. 4'-Methylaminomethyl-2-nitrobiphenyl is reacted, according to the procedures described in Example 1C, with ethyl 2-butyl-4-chloropyrimidine-5-carboxylate to give ethyl 2-butyl-4-{N-[N-methyl-(2'-nitrobiphenyl-4-yl)methyl]amino)pyrimidine-5-carboxylate. Hydrogenation over palladium on carbon in methanol gives the intermediate ethyl 2-butyl-4-{N-[methyl-(2'-aminobiphenyl-4-yl)methyl]amino}pyrimidine-5-carboxylate. The intermediate is dissolved in methylene chloride containing triethylamine and treated at low temperature with trifluoromethanesulfonyl chloride to give the title compound.

Example 39

Ethyl 2-butyl-4-{N-[4-(2'-[1H-tetrazol-5-yl]phenoxy)phenylmethyl]amino}pyrimidine-5-carboxylate

Example 39A

N-Triphenylmethyl-5-[2-(4-aminomethylphenoxy)phenyl]tetrazole

2-[4-Methylphenoxy]benzoic acid, prepared as described by D.J. Carini in *J. Medicinal Chem*., **33**, 1330- (1990), is converted to the corresponding acid chloride with phosphorous trichloride in benzene. The acid chloride is reacted with ammonia to give 2-(4-methylphenoxy)-benzenecarboxamide. The carboxamide is then dehydrated by treatment with thionyl chloride to give 2-(4'-methylphenoxy)benzonitrile.

The 2-(4'-methylphenoxy)benzonitrile is converted to 2-(4'-methylphenoxy)phenyl-tetrazole by heating at 150 ° C with 3 equivalents of triethylamine hydrochloride and 6 equivalents of sodium azide in N-methyl-2-pyrrolidone. The tetrazole intermediate is reacted with triphenylmethyl chloride and triethylamine in refluxing methylene chloride to give N-triphenylmethyl-5-[2-(4'-methylphenoxy)phenyl]tetrazole.

The N-triphenylmethyl-5-[2-(4'-methylphenoxy)phenyl]tetrazole is brominated by treatment with one equivalent of N-bromosuccinimide in refluxing carbon tetrachloride containing a catalytic amount of dibenzoyl peroxide. The resulting bromomethyl compound is reacted with sodium azide in DMF as described in Example 1A to give the corresponding azidomethyl compound. The azidomethyl compound is then reduced, as described in Example 1B, by hydrogenation over Lindlar's catalyst to give the title compound.

Example 39B

Ethyl 2-butyl-4-{N-[4-(2'-[1H-tetrazol-5-yl]phenoxy)phenylmethyl]amino}pyrimidine-5-carboxylate

Following the procedures described in Example 1C and 1D, the compound resulting from Example 39A is reacted with ethyl 2-butyl-4-chloropyrimidine-5-carboxylate and the condensation product is deprotected to give the title compound.

Examples 40 - 44

Following the procedures described in Example 1C and 1D, the intermediates shown in Table 4 are condensed and the condensation products deprotected to give the compounds of Examples 40 - 44 as disclosed in Table 4. The intermediates are prepared by literature methods.

Table 4: Examples 40 - 44

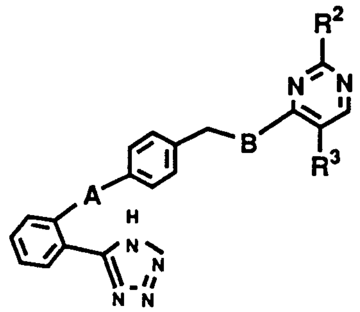

| Ex No | A | B | $R^2$ | $R^3$ | Intermediate 1 | Intermediate 2 |
|---|---|---|---|---|---|---|
| 40 | 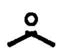 | -NH- | n-butyl | COOEt | 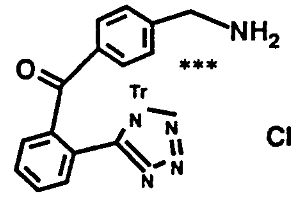 *** | 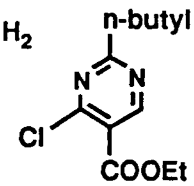 |
| 41 | bond | -S- | n-butyl | COOEt | 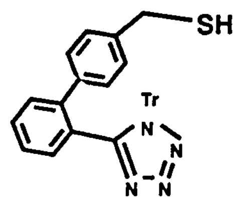 | 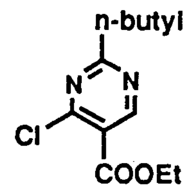 |
| 42 | bond | -NH- | -S-propyl | COOEt | 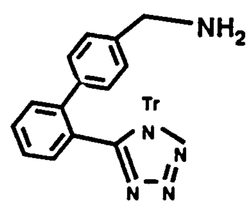 | 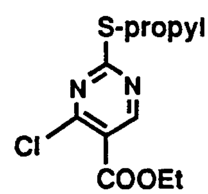 |
| 43 | bond | -NH- | 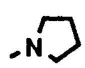 | COOEt | 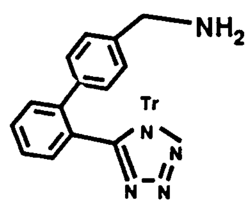 | 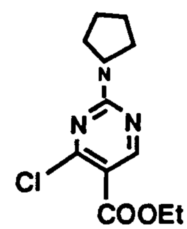 |
| 44 | bond | -N(Me) | n-pentyl | $NO_2$ | 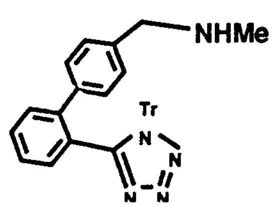 | 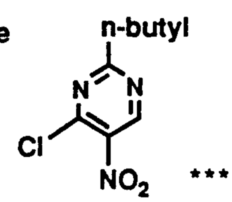 *** |

N-Triphenylmethyl-5-[2-(4-aminomethylbenzoylphenyl)]tetrazole

2-Cyano-4'-methylbenzophenone, prepared as described by G.W. Ebert and R.D. Rieke in *J. Organic Chem.*, **49**, 5280-2 (1984), is converted to 5-[2-(4-methylbenzoyl)phenyl]tetrazole by the procedures described in Step 1 of Example 39. Reaction with triphenylmethyl chloride gives N-triphenylmethyl-5-[2-(4-methylbenzoyl)phenyl]tetrazole.

The N-triphenylmethyl-5-[2-(4'-methylbenzoyl)phenyl]tetrazole is brominated with N-bromosuccinimide

followed by reaction with sodium azide and reduction as described in Step 1 of Example 39 to afford the title compound.

Example 45

Methyl 6-n-butyl-2-{N-[(2'-[1H-tetrazol-5-yl]biphenyl-4-yl)methyl]amino}pyrimidine-4-carboxylate

Example 45A

Methyl 6-n-butyl-2-chloropyrimidine-4-carboxylate

Methyl 6-n-butyl-2-hydroxypyrimidine-4-carboxylate (prepared as described by Z. Budesinsky and F. Roubinek in *Collection Czechoslovak* Chem *Commun.*, **26**, 2871-2885 (1961)) is refluxed with phosphorous oxychloride, as described by the same authors for the chlorination of methyl 6-methyl-2-hydroxypyrimidine-4-carboxylate, to give the title compound.

Example 45B

Methyl 6-n-butyl-2-{N-[(2'-[1H-tetrazol-5-yl]biphenyl-4-yl)methyl]amino}pyrimidine-4-carboxylate

By the procedures described in Example 1C and 1D, N-triphenylmethyl-5-[2-(4'-aminomethyl-biphenyl]-tetrazole and the compound resulting from Example 45A, are condensed and deprotected to give the title compound.

Example 46

2-n-Butyl-5-amino-4-{N-methyl-N-[(2'-(1H-tetrazol-5-yl]biphenyl-4-yl)methyl]amino}pyrimidine

2-n-Butyl-6-chloro-5-nitro-4-{N-[N-methyl-2'-[1H-tetrazol-5-yl]biphenyl-4-yl)methyl]amino}pyrimidine, the product of Example 45 is hydrogenated until 4 equivalents of hydrogen are consumed to yield the title compound.

Example 47

2-n-Butyl-5-(methanesulfonylamino)-4-{N-methyl-N-[(2-[1H-tetrazol-5-yl]biphenyl-4-yl)methyl]-amino}pyrimidine

2-n-Butyl-5-amino-4-{N-[N-methyl-(2'-(1H-tetrazol-5-yl]biphenyl-4-yl)methyl]amino}pyrimidine, the product of Example 46, is dissolved in chloroform containing triethylamine and treated with one equivalent of methanesulfonyl chloride to give the title compound.

Example 48

Ethyl 5-n-butyl-4-{N-[(2'-[1H-tetrazol-5-yl]biphenyl-4-yl)methyl]amino}pyridine-3-carboxylate

Example 48A

Ethyl 5-n-butyl-4-chloropyridine-3-carboxylate

Ethyl 5-n-butyl-4-oxo-1,4-dihydropyridine-3-carboxylate (prepared as described by M. Balogh, *et al.* in *J. Heterocyclic Chem.*, **17**, 359-368 (1980)) is refluxed in phosphorous oxychloride to give the title compound.

Example 48B

Ethyl 5-n-butyl-4-{N-[(2'-[1H-tetrazol-5-yl]biphenyl-4-yl)methyl]amino}pyridine-3-carboxylate

N-triphenylmethyl-5-[2-(4'-aminomethyl-biphenyl)tetrazole is reacted with the compound resulting from

Example 48A, to give an intermediate which is treated with hydrogen chloride as described in Example 1D to give the title compound.

Example 49

Methyl 2-{N-butyl-N-[(2'-[1H-tetrazol-5-yl]biphenyl-4-yl)methyl]amino}pyridine-3-carboxylate

N-Triphenylmethyl-5-[2-(4'-butylaminomethyl-biphenyl)]tetrazole, the product of Example 19A, is reacted with methyl 2-chloropyridine-3-carboxylate (prepared as described by F.G. Mann and J.A Reid in *J. Chem. Soc.*, 2057-62 (1952)) in a manner similar to that described in Example 1C, to give an intermediate which is treated with hydrogen chloride, as described in Example 1D, to give the title compound.

Example 50

Ethyl 3-{N-[(2'-[1H-tetrazol-5-yl]biphenyl-4-yl)methyl]amino}pyridazine-4-carboxylate

By the procedures described in Example 1C and 1D, the compound resulting from Example 19A and ethyl 3-chloropyridazine-4-carboxylate (prepared as described by A. Dornow and W. Abele in *Chem. Berichte*, **97**, 3349-3353 (1964)) are condensed and deprotected to give the title compound.

Example 51

Methyl 5-n-butyl-3-{N-[(2'-[1H-tetrazol-5-yl]biphenyl-4-yl)methyl]amino}pyrazine-2-carboxylate

Example 51A

Methyl 5-n-butyl-3-chloropyrazine-2-carboxylate

2-Oxohexanal (F. Ballistreri, *et al.*, *J. Organic Chem.*, **53**, 830 (1988) is reacted (according to the method described by F.L. Muehlmann and A.R. Day in *J. American Chem. Soc.*, **78**, 242-4 (1956) substituting 2-oxohexanal for the 2-oxopropanal used in the synthesis of 5-methyl-3-hydroxypyrazine-2-carboxamide) with aminomalonamide to give 5-n-butyl-3-hydroxypyrazine-2-carboxamide. The amide is hydrolyzed with sodium hydroxide in water at 95 °C to give 5-n-butyl-3-hydroxypyrazine-2-carboxylic acid. The acid is converted to the corresponding ester with methanol and hydrochloric acid and the ester is chlorinated with phosphorous oxychloride (according to the method described by G. Dick and H. Wood in *J. Chem. Soc.*, 1955, 1379-82) to give the title compound.

Example 51B

Methyl 5-n-butyl-3-{N-[(2'-[1H-tetrazol-5-yl]biphenyl-4-yl)methyl]amino}pyrazine-2-carboxylate

By the procedures described in Example 1C and 1D, N-triphenylmethyl-5-[2-(4'-aminomethyl-biphenyl]-tetrazole and the compound resulting from Example 51A, are condensed and deprotected to give the title compound.

Example 52

Ethyl 3-butyl-5-{N-[(2'-[1H-tetrazol-5-yl]biphenyl-4-yl)methyl]amino}-1,2,4-triazine-6-carboxylate

Example 52A

Ethyl-3-butyl-5-chloro-1,2,4-triazine-6-carboxylate

Pentanamidrazone hydrochloride (H. Paul, *et al., Chem Berichte*, **10**1:2033 (1968)) is dissolved in ethyl alcohol containing an equivalent of sodium ethoxide. Diethyl ketomalonate (1 equivalent) is added and the reactionmixture stirred at ambient temperature for 6 h and refluxed for 3 h to give ethyl 3-butyl-5-hydroxy-1,2,4-triazine-6-carboxylate by the method of E.C. Taylor and S.F. Martin, *J. Organic Chem.*, **37**:3958 (1972). This intermediate is refluxed for 15 minutes in phosphorous oxychloride to give the title compound.

Example 52B

Ethyl 3-butyl-5-{N-[(2'-[1H-tetrazol-5-yl]biphenyl-4-yl)methyl]amino}-1,2,4-triazine-6-carboxylate

By the procedures described in Example 1C and 1D, N-triphenylmethyl-5-[2-(4'-aminomethyl-biphenyl]-tetrazole and the compound resulting from Example 52A, are condensed and deprotected to give the title compound.

Example 53

4-{N-butyl-N-[(2'-[1H-tetrazol-5-yl]biphenyl-4-yl)methyl]amino}pyrimidine-5-carboxylic acid hydrochloride

The compound resulting from Example 83 (7.08 g, 15.5 mmol) was dissolved in water (150 mL) containing potassium hydroxide (8.28 g). The reaction mixture was stirred at room temperature for 24 hours and then acidified with concentrated hydrochloric acid (15 mL). The resulting solid was removed by filtration, dissolved in tetrahydrofuran, dried over sodium sulfate and concentrated under reduced pressure. Trituration of the residue with ether afforded the title compound (7.57 g). $^1H$ NMR (DMSO-$d_6$, 300 MHz) $\delta$ 0.85 (t, J = 7Hz, 3H), 1.18 (m, 2H), 1.52 (m, 2H), 3.45 (t, J = 7Hz, 2H), 4.90 (s, 2H), 7.05 (d, J = 8Hz, 2H), 7.20 (d, J = 8Hz, 2H), 7.50-7.70 (m, 4H), 8.55 (s, 1H), 8.65 (s, 1H).

Example 54

2-n-Propyl-4-{N-[(2'-[1H-tetrazol-5-yl]biphenyl-4-yl)methyl]amino}pyrimidine-5-carboxylic acid hydrochloride

The compound resulting from Example 3 (500 mg, 0.73 mmol) was hydrolyzed by the procedure described in Example 17 to give the title compound (250 mg). m.p. 223-225 °C. $^1H$ NMR ($CD_3OD$, 300 MHz) $\delta$ 0.99 (t, J = 7Hz, 3H), 1.82 (m, 2H), 2.82 (t, J = 7Hz, 2H), 4.90 (s, 1H), 7.10 (d, J = 8Hz, 2H), 7.32 (d, J = 8Hz, 2H), 7.50-7.70 (m, 4H), 8.70 (s, 1H).

Example 55

2-n-Propyl-5-hydroxymethyl-4-{N-[(2'-[1H-tetrazol-5-yl]biphenyl-4-yl)methyl]amino}pyrimidine hydrochloride

By the procedure described in Example 2, the compound resulting from Example 3C (1.00 g, 1.46 mmol) was reduced with lithium aluminum hydride (200 mg) to give 2-n-propyl-5-hydroxymethyl-4-{N-[(2'-[N-triphenylmethyl-1H-tetrazol-5-yl]biphenyl-4-yl)methyl]amino}pyrimidine. m.p. 134-137 °C.

The above compound was treated with hydrochloric acid in ethanol by the procedure described in Example 2B to give the title compound (440 mg). m.p. 214-216 °C. $^1H$ NMR ($CD_3OD$, 300 MHz) $\delta$ 0.95 (t, J = 7Hz, 3H), 1.79 (m, 2H), 2.76 (t, J = 7Hz, 2H), 4.55 (s, 2H), 4.83 (s, 2H), 7.10 (d, J = 7Hz, 2H), 7.32 (d, J = 7Hz, 2H), 7.50-7.70 (m, 4H), 8.02 (s, 1H).

Example 56

2-n-Pentyl-4-{N-[(2'-[1H-tetrazol-5-yl]biphenyl-4-yl)methyl]amino}pyrimidine-5-carboxylic acid hydrochloride

By the procedure described in Example 17, the compound resulting from Example 4C (300 mg, 0.421 mmol) was treated with lithium hydroxide followed by concentrated hydrochloric acid to give the title compound (166 mg). m.p. 166-168 °C. $^1H$ NMR ($CD_3OD$, 300 MHz) $\delta$ 0.90 (t, J = 7Hz, 3H), 1.36 (m, 4H), 1.80 (m, 2H), 2.84 (t, J = 7Hz, 2H), 4.92 (s, 2H), 7.12 (d, J = 7Hz, 2H), 7.32 (d, J = 7Hz, 2H), 7.50-7.70 (m, 4H), 8.72 (s, 1H).

Example 57

2-n-Butyl-4-{N-methyl-N-[(2'-[1H-tetrazol-5-yl]biphenyl-4-yl)methyl]amino}pyrimidine-5-carboxylic acid hydrochloride

By the procedure described in Example 17, the compound resulting from Example 18B (679 mg, 0.952 mmol) was treated with lithium hydroxide followed by concentrated hydrochloric acid to give the title

compound (469 mg). m.p. 165-169 °C. $^1$H NMR ($CD_3OD$, 300 MHz) δ 0.92 (t, J = 7Hz, 3H), 1.48 (m, 2H), 1.73 (m, 2H), 2.85 (t, J = 7Hz, 2H), 3.20 (s, 3H), 5.18 (s, 2H), 7.12 (d, J = 7Hz, 2H), 7.27 (d, J = 8Hz, 2H), 7.50-7.70 (m, 4H), 8.58 (s, 1H).

Example 58

2-n-Butyl-5-hydroxymethyl-4-{N-methyl-N-[(2'-[1H-tetrazol-5-yl]biphenyl-4-yl)methyl]amino}pyrimidine hydrochloride

By the procedure described in Example 2, the compound resulting from Example 18B (700 mg, 0.982 mmol) was reduced with lithium aluminum hydride (140 mg) to give.2-n-butyl-5-hydroxymethyl-4-{N-methyl-N-[(2'-[N-triphenylmethyl-1H-tetrazol-5-yl]biphenyl-4-yl)methyl]amino}pyrimidine (387 mg). m.p. 133-137 °C.

The above compound was treated with hydrochloric acid in ethanol by the procedure described in Example 2B to give the title compound (185 mg). m.p. 152-158 °C. $^1$H NMR ($CD_3OD$, 300 MHz) δ 0.92 (t, J = 7Hz, 3H), 1.38 (m, 2H), 1.73 (m, 2H), 2.80 (t, J = 7Hz, 2H), 3.57 (s, 3H), 4.62 (s, 2H), 5.15 (s, 2H), 7.13 (d, J = 8Hz, 2H), 7.25 (d, J = 8Hz, 2H), 7.50-7.70 (m, 4H), 8.15 (s, 1H).

Example 59

Ethyl 2-(2-methoxyethyl)-4-{N-[(2'-[1H-tetrazol-5-yl]biphenyl-4-yl)methyl]amino}pyrimidine-5-carboxylate

Example 59A

2-Methoxypropionamidine hydrochloride

Methyl 2-methoxypropionimidate hydrochloride (U.S. Patent 4007200) (53 g, 0.345 mol) was dissolved in methanol (400 mL) containing liquid ammonia (70 mL) and kept at room temperature overnight in an autoclave. The reaction mixture was concentrated under reduced pressure, and the residue obtained was dissolved in isopropanol and filtered. The filtrate was concentrated under reduced pressure and the residue crystallized from ether to afford the title compound (47 g).

Example 59B

Ethyl 2-(2-methoxyethyl)-4-hydroxy-pyrimidine-5-carboxylate

To the compound resulting from Example 59A (0.345 mol) dissolved in ethanol (200 mL) and cooled in an ice bath was slowly added a solution of 21% sodium ethoxide in ethanol (223 g) followed by the slow addition of diethyl ethoxymethylene malonate (74.6 g, 0.345 mol). The solution was refluxed 2 hours and then concentrated under reduced pressure. Water was added and the soluton neutralized with hydrochloric acid and extracted with chloroform (4x). The combined organic extracts were dried over magnesium sulfate and concentrated under reduced pressure. The residue obtained crystallized from ether to afford the title compound in 75% yield. m.p. 115-118 °C.

Example 59C

Ethyl 2-(2-methoxyethyl)-4-{N-[(2'-[N-triphenylmethyl-1H-tetrazol-5-yl]biphenyl-4-yl)methyl]-amino}pyrimidine-5-carboxylate

To the compound resulting from Example 59B (1.84 g, 8.14 mmol) dissolved in methylene chloride (13 mL) containing triethylamine (1.38 mL) and cooled in an ice bath was added methanesulfonyl chloride (1.025 g, 8.96 mmol). After stirring for 5 minutes, a solution of the compound resulting from Example 1B (3.993 g, 8.11 mmol) and triethylamine (1.38 g) in chloroform (5 mL) were added. The mixture was stirred at room temperature for 1.5 hours. Methylene chloride (50 mL) was added and the solution washed with sodium bicarbonate solution, dried over sodium sulfate and concentrated under reduced pressure. The residue obtained was chromatographed on silica gel eluting with 25% ethyl acetate in toluene to give the title compound (4.615 g). m.p. 128-130 °C.

Example 59D

Ethyl 2-(2-methoxyethyl)-4-{N-[(2'-[1H-tetrazol-5-yl]biphenyl-4-yl)methyl]amino}pyrimidine-5-carboxylate

By the method described in Example 1D, the compound resulting from Example 59C (500 mg, 0.713 mmol) was treated with hydrochloric acid in ethanol to give the title compound (220 mg). m.p. 122-124 °C. $^1$H NMR ($CDCl_3$, 300 MHz) δ 1.35 (t, J = 7Hz, 3H), 2.90 (t, J = 7Hz, 2H), 3.25 (s, 3H), 3.78 (t, J = 7Hz, 2H), 4.35 (q, J = 7Hz, 2H), 4.75 (d, J = 7Hz, 2H), 7.07 (d, J = 8Hz, 2H), 7.25 (d, J = 8Hz, 2H), 7.42 (dd, 1H), 7.55 (m, 4H), 7.98 (dd, 1H), 8.57 (s, 1H), 8.70 (t, J = 7Hz, 1H).

Example 60

2-(2-Methoxyethyl)-4-{N-[(2'-[1H-tetrazol-5-yl]biphenyl-4-yl)methyl]amino}pyrimidine-5-carboxylic acid hydrochloride

By the method described in Example 17, the compound resulting from Example 59B (600 mg, 0.856 mmol) was treated with lithium hydroxide followed by concentrated hydrochloric acid to give the title compound (364 mg). m.p. 275 °C. $^1$H NMR ($CD_3OD$, 300 MHz) δ 3.20 (t, J = 7Hz, 2H), 3.29 (s, 3H), 3.80 (t, J = 7Hz, 2H), 4.90 (s, 2H), 7.12 (d, J = 8Hz, 2H), 7.35 (d, J = 8Hz, 2H), 7.50-7.70 (m, 4H), 8.72 (s, 1H).

Example 61

2-(2-Methoxyethyl)-5-hydroxymethyl-4-{N-[(2'-[1H-tetrazol-5-yl]biphenyl-4-yl)methyl]-amino}pyrimidinehydrochloride

By the method of Example 2, the compound resulting from Example 59C (1.00 g, 1.43 mmol) was reduced with lithium aluminum hydride (210 mg) to give 2-(2-methoxyethyl)-5-hydroxymethyl-4-{N-[(2'-[N-triphenylmethyl-1H-tetrazol-5-yl]biphenyl-4-yl)methyl]amino}pyrimidine (405 mg). m.p. 118-120 °C.

The above compound was treated with hydrochloric acid in ethanol by the procedure described in Example 2B to give the title compound (265 mg). m.p. 218-220 °C. $^1$H NMR ($CD_3OD$, 300 MHz) δ 3.01 (t, J = 7Hz, 2H), 3.22 (s, 3H), 3.75 (t, J = 7Hz, 2H), 4.55 (s, 2H), 4.72 (s, 2H), 7.10 (d, J = 8Hz, 2H), 7.32 (d, J = 8Hz, 2H), 7.50-7.70 (m, 4H), 8.00 (s, 1H).

Example 62

Ethyl 2-ethyl-4-{N-[(2'-[1H-tetrazol-5-yl]biphenyl-4-yl)methyl]amino}pyrimidine-5-carboxylate

Example 62A

Ethyl 2-ethyl-4-{N-[(2'-[N-triphenylmethyl-1H-tetrazol-5-yl]biphenyl-4-yl)methyl]amino}pyrimidine-5-carboxylate

By the method described in Example 1C, the compound resulting from Example 1B (3.75 g, 6.85 mmol) was reacted with ethyl 2-ethyl-4-chloropyrimidine-5-carboxylate (1.62 g, 7.54 mmol) to give the title compound (3.709 g). m.p. 134-136 °C.

Example 62B

Ethyl 2-ethyl-4-{N-[(2'-[1H-tetrazol-5-yl]biphenyl-4-yl)methyl]amino}pyrimidine-5-carboxylate

By the method described in Example 1D, the compound resulting from Example 62A (530 mg, 0.745 mmol) was treated with hydrochloric acid in ethanol to give the title compound (202 mg). m.p. 114-116 °C. $^1$H NMR ($CDCl_3$, 300 MHz) δ 1.19 (t, J = 7Hz, 3H), 1.34 (t, J = 7Hz, 3H), 2.55 (q, J = 7Hz, 2H), 4.30 (q, J = 7Hz, 2H), 4.80 (d, J = 7Hz, 2H), 6.99 (d, J = 8Hz, 2H), 7.20 (d, J = 8Hz, 2H), 7.40 (dd, 1H), 7.55 (m, 2H), 7.95 (dd, 1H), 8.32 (s, 1H), 8.65 (t, J = 7Hz, 1H).

Example 63

2-Ethyl-4-{N-[(2'-[1H-tetrazol-5-yl]biphenyl-4-yl)methyl]amino}pyrimidine-5-carboxylic acid hydrochloride

By the method described in Example 17, the compound resulting from Example 62A (500 mg, 0.745 mmol) was treated with lithium hydroxide followed by concentrated hydrochloric acid to give the title compound (314 mg, 96%). m.p. 200-202 °C. $^1$H NMR ($CD_3OD$, 300 MHz) δ 1.32 (t, J = 7Hz, 3H), 2.90 (q, J = 7Hz, 2H), 4.95 (s, 2H), 7.12 (d, J = 8Hz, 2H), 7.35 (d, J = 8Hz, 2H), 7.50-7.70 (m, 4H), 8.72 (s, 1H).

Example 64

2-Ethyl-5-hydroxymethyl-4-{N-[(2'-[1H-tetrazol-5-yl]biphenyl-4-yl)methyl]amino}pyrimidine hydrochloride

By the method described in Example 2, the compound resulting from Example 62A (1.00 g, 1.49 mmol) was reduced with lithium aluminum hydride (200 mg) to give 2-ethyl-5-hydroxymethyl-4-{N-[(2'-[N-triphenylmethyl-1H-tetrazol-5-yl]biphenyl-4-yl)methyl]amino}pyrimidine (831 mg). m.p. 118-120 °C.

The above compound was reacted with hydrochloric acid in ethanol by the procedure described in Example 2B to give the title compound (488 mg). m.p. 217-219 °C. $^1$H NMR ($CD_3OD$, 300 MHz) δ 1.31 (t, J = 7Hz, 3H), 2.83 (q, J = 7Hz, 2H), 4.56 (s, 2H), 4.85 (s, 2H), 7.10 (d, J = 8Hz, 2H), 7.32 (d, J = 8Hz, 2H), 7.50-7.70 (m, 4H), 8.00 (s, 1H).

Example 65

Ethyl 2-methyl-4-{N-[(2'-[1H-tetrazol-5-yl]biphenyl-4-yl)methyl]amino}pyrimidine-5-carboxylate

Example 65A

Ethyl 2-methyl-4-{N-[(2'-[N-triphenylmethyl-1H-tetrazol-5-yl]biphenyl-4-yl)methyl]amino}pyrimidine-5-carboxylate

By the method described in Example 1C, the compound resulting from Example 1B (4.20 g, 8.52 mmol) was reacted with ethyl 2-methyl-4-chloropyrimidine-5-carboxylate (1.75 g, 8.72 mmol), prepared as described by H. Yamanaka in *Heterocycles*, **12**, 1323 (1979), to afford the title compound (4.705 g, 84%). m.p. 155-156 °C.

Example 65B

Ethyl 2-methyl-4-{N-[(2'-[1H-tetrazol-5-yl]biphenyl-4-yl)methyl]amino}pyrimidine-5-carboxylate

By the method described in Example 1D, the compound resulting from Example 65A (1.00 g, 1.52 mmol) was treated with hydrochloric acid in ethanol to give the title compound (439 mg). m.p. 121-123 °C. $^1$H NMR ($CDCl_3$, 300 MHz) δ 1.32 (t, J = 7Hz, 3H), 2.28 (s, 3H), 4.71 (q, J = 7Hz, 2H), 4.80 (d, J = 7Hz, 2H), 7.00 (d, J = 8Hz, 2H), 7.15 (d, J = 8Hz, 2H), 7.42 (dd, 1H), 7.55 (m, 2H), 7.98 (dd, 1H), 8.30 (s, 1H), 8.70 (t, J = 7Hz, 1H).

Example 66

2-Methyl-4-{N-[(2'-[1H-tetrazol-5-yl]biphenyl-4-yl)methyl]amino}pyrimidine-5-carboxylic acid hydrochloride

By the method described in Example 17, the compound resulting from Example 65A (1.00 g, 1.52 mmol) was treated with lithium hydroxide followed by concentrated hydrochloric acid to give the title compound (590 mg, 92%). m.p. 211-213 °C. $^1$H NMR ($CD_3OD$, 300 MHz) δ 2.62 (s, 3H), 4.90 (s, 2H), 7.12 (d, J = 8Hz, 2H), 7.35 (d, J = 8Hz, 2H), 7.50-7.70 (m, 4H), 8.72 (s, 1H).

Example 67

2-Methyl-5-hydroxymethyl-4-{N-[(2'-[1H-tetrazol-5-yl]biphenyl-4-yl)methyl]amino}pyrimidine hydrochloride

By the method of Example 2, the compound resulting from Example 65A (1.00 g, 1.52 mmol) was reduced lithium aluminum hydride (220 mg) to give 2-methyl-5-hydroxymethyl-4-{N-[(2'-(N-triphenylmethyl-1H-tetrazol-5-yl)biphenyl-4-yl)methyl]amino}pyrimidine (849 mg). m.p. 112-114 °C.

The above compound was reacted with hydrochloric acid in ethanol by the procedure described in

Example 2B to give the title compound (450 mg). m.p. 185-187 °C. $^1$H NMR ($CD_3OD$, 300 MHz) δ 2.56 (s, 3H), 4.55 (s, 2H), 4.85 (s, 2H), 7.10 (d, J = 8Hz, 2H), 7.32 (d, J = 8Hz, 2H), 7.50-7.70 (m, 4H), 8.02 (s, 1H).

Example 68

Ethyl 2-methylthio-4-{N-butyl-N-[(2'-[1H-tetrazol-5-yl]biphenyl-4-yl)methyl]amino)pyrimidine-5-carboxylate

Example 68A

Ethyl 2-methylthio-4-{N-butyl-N-[(2'-[N-triphenylmethyl-1H-tetrazol-5-yl]biphenyl-4-yl)methyl]-amino}pyrimidine-5-carboxylate

Using the procedure described in Examples 19A and B, N-triphenylmethyl-5[2-(4'-bromomethyl-biphenyl)]tetrazole (2.80 g, 5.00 mmol), butylamine and commercially available ethyl 4-chloro-2-methylthio-pyrimidine-5-carboxylate (1.31 g, 5.64 mmol) were reacted to give the title compound (3.02 g). m.p. 122-124 °C.

Example 68B

Ethyl 2-methylthio-4-{N-butyl-N-[(2'-[1H-tetrazol-5-yl]biphenyl-4-yl)methyl]amino}pyrimidine-5-carboxylate

The compound resulting from Example 68A (500 mg, 0.671 mmol) was dissolved in ethanol and treated with hydrochloric acid by the procedure described in Example 19C to give the title compound (262 mg). m.p. 161-163 °C following crystallization from ether. $^1$H NMR (DMSO-$d_6$, 300 MHz) δ 0.82 (t, J = 7Hz, 3H), 1.21 (t, J = 7Hz, 3H), 1.55 (m, 2H), 2.38 (s, 3H), 3.40 (t, J = 7Hz, 2H), 4.18 (q, J = 7Hz, 2H), 4.79 (s, 2H), 7.05 (d, J = 8Hz, 2H), 7.15 (d, J = 8Hz, 2H), 7.50-7.70 (m, 4H), 8.32 (s, 1H).

Example 69

2-Methylthio-4-{N-butyl-N-[(2'-[1H-tetrazol-5-yl]biphenyl-4-yl)methyl]amino}pyrimidine-5-carboxylic acid hydrochloride

By the method described in Example 17, the compound resulting from Example 68A (500 mg, 0.671 mmol) was treated with lithium hydroxide followed by concentrated hydrochloric acid to give the title compound (321 mg). $^1$H NMR (DMSO-$d_6$, 300 MHz) δ 0.85 (t, J = 7Hz, 3H), 1.20 (m, 2H), 1.55 (m, 2H), 2.38 (s, 3H), 3.46 (t, J = 7Hz, 2H), 4.86 (s, 2H), 7.05 (d, J = 8Hz, 2H), 7.18 (d, J = 8Hz, 2H), 7.50-7.70 (m, 4H), 8.40 (s, 1H.)

Example 70

Ethyl 4-{N-propyl-N-[(2'-[1H-tetrazol-5-yl]biphenyl-4-yl)methyl]amino}pyrimidine-5-carboxylate

Example 70A

Ethyl 4-{N-propyl-N-[(2'-[N-triphenylmethyl-1H-tetrazol-5-yl]biphenyl-4-yl)methyl]amino}pyrimidine-5-carboxylate

Using the procedure described in Examples 19A and B, N-triphenylmethyl-5[2-(4'-bromomethyl-biphenyl)]tetrazole, propylamine, and ethyl 4-chloropyrimidine-5-carboxylate (1.00 g, 5.38 mmol), prepared as described by H. Bredereck, F. Effenberger and E.H. Schweizer in *Chem. Ber.*, **95**, 803 (1962), were reacted to give the title compound (2.694 g). m.p. 100-103 °C.

Example 70B

Ethyl 4-{N-propyl-N-[(2'-[1H-tetrazol-5-yl]biphenyl-4-yl)methyl]amino}pyrimidine-5-carboxylate

The compound resulting from Example 70A (2.60 g, 3.79 mmol) was dissolved in ethanol and treated with hydrochloric acid by the procedure described in Example 19C to give the title compound (1.13 g). m.p.

160-162 °C. $^1$H NMR (DMSO-$d_6$, 300 MHz) δ 0.77 (t, J = 7Hz, 3H), 1.23 (t, J = 7Hz, 3H), 1.55 (m, 2H), 3.35 (m, 2H), 4.20 (q, J = 7Hz, 2H), 4.71 (s, 2H), 7.03 (d, J = 8Hz, 2H), 7.15 (d, J = 8Hz, 2H), 7.50-7.70 (m, 4H), 8.45 (s, 1H), 8.58 (s, 1H).

Example 71

4-{N-Propyl-N-[(2'-[1H-tetrazol-5-yl]biphenyl-4-yl)methyl]amino}pyrimidine-5-carboxylic acid hydrochloride

The compound resulting from Example 70 (300 mg, 0.677 mmol) was hydrolyzed with potassium hydroxide in water using the procedure described in Example 53 to give the title compound (280 mg). $^1$H NMR (DMSO-$d_6$, 300 MHz) δ 0.79 (t, J = 7Hz, 3H), 1.58 (m, 2H), 3.44 (m, 2H), 4.90 (s, 2H), 7.05 (d, J = 8Hz, 2H), 7.20 (d, J = 8Hz, 2H), 7.50-7.70 (m, 4H), 8.60 (s, 1H), 8.70 (s, 1H).

Example 72

Ethyl 3-methyl-5-{N-propyl-N-[(2'-[1H-tetrazol-5-yl]biphenyl-4-yl)methyl]amino}-1,2,4-triazine-6-carboxylate

Example 72A

Ethyl 3-methyl-5-chloro-1,2,4-triazine-6-carboxylate

To 3-methyl-5-hydroxy-1,2,4-triazino-6-carboxylate (1.50 g, 8.20 mmol), prepared as described by E.C. Taylor and S.F. Martin in *J. Org. Chem.*, **37**, 3958 (1972), suspended in phosphorus oxychloride (12 mL) was added triethylamine (827 mg, 8.20 mmol). The reaction mixture was stirred at room temperature for 1 hour and then the solvent was removed under reduced pressure. To the residue obtained was added toluene, which was then removed under reduced pressure. The residue obtained was dissolved in toluene and washed with water (3 mL) and dilute sodium bicarbonate solution. The organic phase was dried over magnesium sulfate and concentrated under reduced pressure. The residue obtained was dissolved in heptane, filtered and concentrated *in vacuo* to afford the title compound as an oil.

Example 72B

Ethyl 3-methyl-5-{N-propyl-N-[2'-[N-triphenylmethyl-1H-tetrazol-5-yl]biphenyl-4-yl)methyl]amino}-1,2,4-triazine-6-carboxylate

N-Triphenylmethyl-5-[2-(4'-bromomethyl-biphenyl)]tetrazole (3.28 g, 5.87 mmol) was treated with propylamine by the procedure described in Example 19A to give N-triphenylmethyl-5-[2-(4'-propylaminomethyl-biphenyl)]tetrazole. This was dissolved in tetrahydrofuran (8.5 mL) containing triethylamine (3.1 mL). The compound resulting from Example 72A (1.30 g, 6.47 mmol) was added, and the mixture was stirred at room temperature for 2 hours. The solvents were removed *in vacuo* and the residue obtained dissolved in toluene and washed with sodium bicarbonate solution. The organic phase was dried over sodium suflate and concentrated under reduced pressure. The residue obtained was chromatographed on silica gel eluting with 25% ethyl acetate in toluene to afford the title compound (3.91 g). m.p. 178-179 °C.

Example 72C

Ethyl 3-methyl-5-{N-propyl-N-[(2'-[1H-tetrazol-5-yl]biphenyl-4-yl)methyl]amino}-1,2,4-triazine-6-carboxylate

The compound resulting from Example 72B (600 mg, 0.857 mmol) was suspended in ethanol (8 mL) and treated with hydrochloric acid as described in Example 19C to give the title compound (303 mg). m.p. 120-122 °C. $^1$H NMR (DMSO-$d_6$, 300 MHz) δ 0.80 (t, J = 7Hz, 3H), 1.22 (t, J = 7Hz, 3H), 1.55 (m, 2H), 3.40 (m, 2H), 4.28 (q, J = 7Hz, 2H), 4.82 (s, 2H), 7.05 (d, J = 8Hz, 2H), 7.15 (d, J = 8Hz, 2H), 7.50-7.70 (m, 4H).

Example 73

3-Methyl-5-{N-propyl-N-[(2'-[1H-tetrazol-5-yl]biphenyl-4-yl)methyl]amino)-1,2,4-triazine-6-carboxylic acid hydrochloride

The compound resulting from Example 72 (200 mg, 0.436 mmol) was dissolved in methanol (10 mL) and water (1 mL) containing sodium hydroxide (175 mg). The solution was refluxed 1 hour, cooled in an ice bath and neutralized with concentrated hydrochloric acid. The solvent was removed under reduced pressure and the residue obtained was dissolved in chloroform, dried over sodium sulfate and concentrated *in vacuo* to give the title compound (173 mg). $^1$H NMR (DMSO-$d_6$, 300 MHz) δ 0.89 (t, J = 7Hz, 3H), 1.58 (m, 2H), 2.45 (s, 3H), 3.52 (m, 2H), 4.90 (s, 2H), 7.05 (m, 2H), 7.22 (m, 2H), 7.50-7.70 (m, 4H).

Example 74

Ethyl 4-{N-[(2'-[1H-tetrazol-5-yl]biphenyl-4-yl) methyl]amino}pyrimidine-5-carboxylate.

Example 74A

Ethyl 4-{N-[(2'-[N-triphenylmethyl-tetrazol-5-yl]biphenyl-4-yl)methyl]amino}pyrimidine-5-carboxylate

The product resulting from Example 1A (3,75 g, 7.2 mmol) was treated with lithium aluminum hydride in tetrahydrofuran by the procedure described in Example 1B to afford an amorphous solid. To this solid dissolved in tetrahydrofuran (50 mL) was added N-methylmorpholine (2.2 g, 22 mmol) and ethyl 4-chloropyrimidine-5-carboxylate (1.35 g, 7.2 mmol). The mixture was stirred overnight at room temperature and then concentrated under reduced pressure and the residue obtained partitioned between water and ethyl acetate. The organic layer was washed with water and brine, dried over sodium carbonate/sodium sulfate and concentrated under reduced pressure to afford an amorphous solid. Silica gel chromatography eluting with ethyl acetate in toluene afforded the title compound as a colorless amorphous solid (3.00 g, 81%). $^1$H NMR ($CDCl_3$, 300 MHz) δ 1.37 (t, J = 7Hz, 3H), 2.36 (s, 3H), 4.32 (q, J = 7Hz, 2H), 4.68 (d, J = 6Hz, 2H), 7.50-6.90 (m, 22H), 7.95 (m, 1H), 8.52 (br t, 1H), 8.66 (s, 1H), 8.88 (s, 1H). MS ($DCl/NH_3$) m/e 644 $(M+H)^+$.

Example 74B

Ethyl 4-{N-[(2'-[5H-tetrazol-5-yl]biphenyl-4-yl)methyl]amino}pyrimidine-5-carboxylate

To the product resulting from Example 74A (1.5 g, 2.3 mmol) dissolved in tetrahydrofuran (15 mL) was added acetic acid (15 mL) and water (2 mL). The solution was refluxed for 1 hour, cooled to room temperature and concentrated under reduced pressure. The residue obtained was triturated with hexane and ether to afford a solid which was recrystallized from ethyl acetate/hexane and then from methylene chloride/hexane. The off-white crystals were dried overnight under vacuum at 60 °C to afford the title compound (670 mg, 62%). $^1$H NMR (DMSO-$d_6$, 300 MHz) δ 1.32 (t, J = 8 Hz, 3H), 4.32 (q, J = 8 Hz, 2H), 4.72 (d, J = 6 Hz, 2H), 7.05 (d, J = 9Hz, 2H), 7.25 (d, J = 9 Hz, 2H), 7.70-7.50 (m, 4H), 8.60 (s, 1H), 8.70 (brt, 1H,), 8.78 (s, 1H). Anal calcd for $C_{21}H_{19}N_7O_2$: C, 62.83; H, 4.77; N, 24.42. Found: C, 62.93; H, 4.82; N, 24.30. MS ($DCl/NH_3$) m/e 402 $(M+H)^+$.

Example 75

4-{N-[(2'-[5H-tetrazol-5-yl]biphenyl-4-yl)methyl]amino}pyrimidine-5-carboxylic acid

The product resulting from Example 74B (300 mg, 0.75 mmol) was suspended in ethanol (10 mL). A solution of sodium hydroxide (130 mg, 3 mmol) in water (1 mL) was added and the clear solution was stirred for 45 minutes. The reaction was concentrated under reduced pressure and the residue obtained dissolved in water (50 mL). The solution was acidified with acetic acid. The solid was removed by filtration and dissolved in a warm ethanol/methanol mixture and filtered. The filtrate was concentrated under reduced pressure and the colorless powder was dried overnight on hi-vac at 60° C to afford the title compound (245 mg ,88%). $^1$H NMR (DMSO-$d_6$, 300 MHz) δ 4.74 (d, J = 6 Hz, 2H), 7.05 (d, J = 7 Hz, 2H), 7.25 (d, J = 7 Hz, 2H), 7.70-7.50 (m, 4H), 8.59 (s, 1H), 8.72 (s, 1H), 8.91 (brt, 1H). Anal calcd for $C_{19}H_{15}N_7O_2 \cdot 0.65$ EtOH: C, 60.45; H, 4.72; N, 24.39. Found: C, 60.04; H, 4.56; N, 24.60. MS ($DCl/NH_3$) m/e 374 $(M+H)^+$.

Example 76

2-Methyl-4-{N-butyl-N-[(2'-[1H-tetrazol-5-yl]biphenyl-4-yl)methyl]amino}pyrimidine-5-carboxylic acid hydrochloride

To the compound resulting from Example 19B (0.6 g, 0.842 mmol) dissolved in methanol (4 mL) and tetrahydrofuran (6 mL) was added a solution of lithium hydroxide monohydrate (180 mg) in water (1.5 mL). The mixture was heated at reflux for 5 hours and then concentrated *in vacuo*. The residue obtained was crystallized from acetonitrile to afford the title compound (289 mg). m.p. 155-158 °C. $^1$H NMR ($CD_3OD$, 300 MHz) δ 0.95 (t, J = 7Hz, 3H), 1.30 (m, 2H), 1.69 (m, 2H), 2.60 (s, 3H), 3.70 (m, 2H), 5.08 (m, 2H), 7.10 (d, 2H), 7.20 (s, 2H), 7.55 (d, 2H), 7.50-7.70 (m, 4H), 8.55 (s, 1H).

Example 77

2-Methyl-4-{N-butyl-N-[(2'-[1H-tetrazol-5-yl]biphenyl-4-yl)methyl]amino}pyrimidine-5-carboxamide

Ethyl 2-methyl-4-{N-[(2'-[N-triphenylmethyl-tetrazol-5-yl]biphenyl-4-yl)methyl]amino}pyrimidine-5-carboxylate was hydrolyzed with sodium hydroxide and neutralized with one equivalent of hyochloric acid to give 2-methyl-4-{N-[(2'-[N-triphenylmethyl-tetrazol-5-yl]biphenyl-4-yl)methyl]amino}pyrimidine-5-carboxylic acid. This compound was treated with thionyl chloride at room temperature for 2 hours. The reaction mixture was concentrated under reduced pressure and the residue obtained dissolved in methylene chloride and cooled to 0 °C. Ammonia was added, and the reaction mixture was stirred at 0 °C for several hours. The solvent was removed under reduced pressure and the residue obtained chromatographed on silica gel. The triphenylmethyl protecting group was removed with aqueous hydrochloric acid in dioxane to give the title compound in high yield. $^1$H NMR (DMSO-$d_6$, 300 MHz) δ 0.85 (t, J = 7Hz, 3H), 1.22 (m, 2H), 1.55 (m, 2H), 2.55 (s, 3H), 3.58 (m, 2H), 5.00 (s, 2H), 7.08 (d, J = 7Hz, 2H), 7.25 (d, J = 7Hz, 2H), 7.55 (d, J = 7Hz, 1H), 7.59 (d, J = 7Hz, 1H), 7.68 (m, 2H), 8.01 (s, 1H), 8.40 (s, 1H), 8.42 (s, 1H). MS (DCI/$NH_3$) m/e 443 $(M+H)^+$.

Example 78

2-Methyl-4-{N-butyl-N-[(2'-[1H-tetrazol-5-yl]biphenyl-4-yl)methyl]amino}pyrimidine-5-(N-morpholinyl)-carboxamide

The title compound was prepared in analogy to Example 77 using morpholine in place of ammonia. $^1$H NMR (DMSO-$d_6$, 300 MHz) δ 0.88 (t, J = 7Hz, 3H), 1.24 (q, J = 7Hz, 2H), 1.56 (m, 2H), 2.52 (s, 3H), 3.30-3.70 (m, 10H), 4.88 (d, J = 15Hz, 1H), 5.00 (d, J = 15Hz, 1H), 7.08 (d, J = 7Hz, 2H), 7.18 (d, J = 7Hz, 2H), 7.52 (d, J = 7Hz, 1H), 7.59 (d, J = 7Hz, 1H), 7.68 (m, 2H), 8.34 (s, 1H). MS (DCI/$NH_3$) m/e 513 $(M+H)^+$.

Example 79

2-Methyl-4-{N-butyl-N-[(2'-[1H-tetrazol-5-yl]biphenyl-4-yl)methyl]amino}pyrimidine-5-(N-methoxy)-carboxamide

The title compound was prepared in analogy to Example 77 using methoxylamine in place of ammonia. $^1$H NMR (DMSO-$d_6$, 300 MHz) δ 0.85 (t, J = 7Hz, 3H), 1.20 (q, J = 7Hz, 2H), 1.53 (m, 2H), 2.55 (s, 3H), 3.54 (m, 2H), 3.68 (s, 3H), 5.00 (s, 2H), 7.08 (d, J = 7Hz, 2H), 7.24 (d, J = 7Hz, 2H), 7.53 (d, J = 7Hz, 2H), 7.59 (d, J = 7Hz, 2H), 7.68 (m, 2H), 8.49 (s, 1H), 12.24 (s, 1H). MS (DCI/$NH_3$) m/e 473 $(M+H)^+$.

Example 80

Ethyl 2-methyl-4-{N-methoxyethyl-N-[(2'-[1H-tetrazol-5-yl]biphenyl-4-yl)methyl]amino}pyrimidine-5-carboxylate

The title compound was prepared in analogy to Example 19 using methoxyethylamine instead of n-butylamine in the first step. $^1$H NMR (DMSO-$d_6$, 300 MHz) δ 1.22 (t, J = 7Hz, 3H), 2.56 (s, 3H), 3.19 (s, 3H), 3.55 (t, J = 6Hz, 2H), 3.78 (bt, 2H), 4.20 (q, J = 7Hz, 2H), 4.98 (s, 2H), 7.09 (d, J = 7Hz, 2H), 7.19 (d, J = 7Hz, 2H), 7.54 (d, J = 6Hz, 2H), 7.59 (d, J = 6Hz, 2H), 7.68 (m, 2H), 8.62 (s, 1H). Anal calcd for $C_{25}H_{27}N_7O_3 \cdot HCl$: C, 58.88; H, 5.53; N, 19.22. Found: C, 58.88; H, 5.43; N, 19.03. MS (DCI/$NH_3$) m/e 474 $(M+H)^+$.

Example 81

Ethyl 2-methyl-4-{N-methoxyethyl-N-[(2'-[1H-tetrazol-5-yl]biphenyl-4-yl)methyl]amino}pyrimidine-5-carboxylic acid hydrochloride

The compound resulting from Example 80 was hydrolyzed by the procedure described in Example 53 to afford the title compound. $^1H$ NMR (DMSO-$d_6$, 300 MHz) $\delta$ 2.55 (s, 3H), 3.18 (s, 3H), 3.55 (t, J = 6Hz, 2H), 3.78 (t, J = 6Hz, 2H), 5.02 (s, 2H), 7.08 (d, J = 7Hz, 2H), 7.23 (d, J = 7Hz, 2H), 7.54 (d, J = 7Hz, 1H), 7.59 (d, J = 7Hz, 1H), 7.68 (t, J = 7Hz, 2H), 8.65 (s, 1H). Anal calcd for $C_{23}H_{23}N_7O_3 \cdot HCl \cdot 0.6\ H_2O$: C, 55.98; H, 4.87; N, 19.88. Found: C, 56.25; H, 5.13; N, 19.44. MS (DCI/$NH_3$) m/e 446 $(M+H)^+$.

Example 82

Pivaloyloxymethyl 4-{N-butyl-N-[(2'-[1H-tetrazol-5-yl]biphenyl-4-yl)methyl]amino}pyrimidine-5-carboxylate

Example 82A

Pivaloyloxymethyl 4-{N-butyl-N-[(2'-[N-triphenylmethyl-1H-tetrazol-5-yl]biphenyl-4-yl)methyl]-amino}pyrimidine-5-carboxylate

The compound resulting from Example 119A (300 mg, 0.7 mmol) was dissolved in tetrahydrofuran (6 mL) and treated with 10 drops of triethylamine and triphenylmethyl chloride (300 mg, 1.05 mmol). After stirring for 15 hours at room temperature, the mixture was diluted with ethyl acetate (200 mL) and washed with 0.5*N* HCl, saturated brine, and dried over sodium sulfate. The filtered solution was concentrated under reduced pressure to afford a colorless solid. The solid was dissolved in ethyl acetate (20mL) and hexane (200 mL) was added. The solid was removed by filtration and dried to afford 4-{N-butyl-N-[(2'-[N-triphenylmethyl-1H-tetrazol-5-yl]biphenyl-4-yl)methyl]amino}pyrimidine-5-carboxylic acid (300 mg, 64 %) as an amorphous solid.

The above compound (250 mg, 0.37 mmol) was dissolved in anhydrous dimethylformamide (0.5 mL). Triethylamine (75 mg, 0.75 mmol) and chloromethyl pivalate (112 mg, 0.75 mmol) were added and the reaction was stirred for 16 hours at room temperature. The mixture was poured into ethyl acetate (200 mL) and this solution was washed with water, brine, and dried over sodium carbonate and sodium sulfate. The filtered solution was concentrated under reduced pressure to afford a yellow amorphous solid. Chromatography on silica gel eluting with ethyl acetate-hexane mixtures afforded the title compound as an amorphous solid (200 mg, 68 %). $^1H$ NMR ($CDCl_3$, 300 MHz) $\delta$ 0.85 (t, J = 7 Hz, 3H), 1.30-1.05 (m, 5H), 1.55 (m, 3H), 3.35 (t, J = 7 Hz), 4.68 (s, 2H) 5.90 (s, 2H), 6.80 - 7.50 (m, 22H), 7.92 (m, 1H), 8.60 (s, 1H), 8.76 (s, 1H). MS (FAB) m/e 786 $(M+H)^+$, 808 $(M+Na)^+$.

Example 82B

Pivaloyloxymethyl 4-{N-butyl-N-[(2'-[1H-tetrazol-5-yl]biphenyl-4-yl)methyl]amino}pyrimidine-5-carboxylate

The compound resulting from Example 82A (280 mg, 0.36 mmol) was dissolved in tetrahydrofuran (6 mL). Acetic acid (6 mL) and water (1 mL) were added and the solution was refluxed for one hour. The cooled solution was concentrated *in vacuo* and the residue obtained chromatographed on silica gel eluting with ethanol/methylene chloride mixtures to afford the title compound as an off-white amorphous solid (123 mg, 64%). $^1H$ NMR ($CDCl_3$) $\delta$ 0.89 (t, J = 7 Hz, 3H), 1.18 (s, 9H), 1.27 (m, 2H), 1.62 (m, 2H), 3.50 (br t, J = 7Hz, 2H), 4.82 (s, 2H), 5.86 (s, 2H), 7.18 (m, 4H), 7.52 (m, 3H), 8.10 (m, 1H), 8.42 (s, 1H), 8.49 (s, 1H). MS (DCI) m/e 544 $(M+H)^+$.

Example 83

Ethyl 4-{N-butyl-N-[(2'-[1H-tetrazol-5-yl]biphenyl-4-yl)methyl]amino}pyrimidine-5-carboxylate

Example 83A

Ethyl 4-{N-butyl-N-[(2'-[N-triphenylmethyl-1H-tetrazol-5-yl]biphenyl-4-yl)methyl]amino}pyrimidine-5-carboxylate

The compound resulting from Example 19A (5.35 mmol) was reacted with ethyl 4-chloropyrimidine-5-carboxylate (1.00 g, 5.38 mmol), prepared as described by H. Bredereck, F. Effenberger and E.H. Schweizer, *Chem. Ber.*, **95**, 803 (1962), by the procedure described in Example 19B to afford the title compound (2.57 g). m.p. 142-144 °C.

Example 83B

Ethyl 4-{N-butyl-N-[(2'-[1H-tetrazol-5-yl]biphenyl-4-yl)methyl]amino}pyrimidine-5-carboxylate

The compound resulting from Example 83A (2.57 g, 3.75 mmol) was deprotected by the procedure described in Example 19C to afford the title compound (1.286 g) which was crystallized from ether. m.p. 109-111 °C. $^1H$ NMR (DMSO-$d_6$, 300 MHz) δ 0.82 (t, J = 7Hz, 3H), 1.17 (m, 2H), 1.22 (t, J = 7Hz, 3H), 1.51 (m, 2H), 3.39 (t, J = 7Hz, 2H), 4.21 (q, J = 7Hz, 2H), 4.71 (s, 2H), 7.05 (d, J = 8Hz, 2H), 7.15 (d, J = 8Hz, 2H), 7.50-7.72 (m, 4H), 8.48 (s, 1H), 8.60 (s, 1H).

Example 84

3-Nitro-2-[N-propyl-N-[(2'-[1H-tetrazol-5-yl]biphenyl-4-yl)methyl]amino}pyridine

Example 84A

3-Nitro-2-[N-propyl-N-[2'-[N-triphenylmethyl-1H-tetrazol-5-yl]biphenyl-4-yl)methyl]amino}pyridine

N-Triphenylmethyl-5-[2-(4'-propylaminomethyl-biphenyl)]tetrazole (4.03 g, 7.53 mmol), prepared as described in Example 72B, was dissolved in 8 mL of tetrahydrofuran containing 2.5 mL of triethylamine. 2-Chloro-3-nitropyridine (1.29 g, 8.14 mmol) was added and the solution was refluxed for 2.5 hours. The solvent was removed *in vacuo* and the residue obtained dissolved in toluene. This solution was washed with sodium bicarbonate solution, dried over sodium sulfate and concentrated *in vacuo* to afford crude product. Chromatography on silica gel eluting with 25% ethyl acetate in toluene afforded the title compound in 88% yield (4.35 g, 6.61 mmol).

Example 84B

3-Nitro-2-[N-propyl-N-[(2'-[1H-tetrazol-5-yl]biphenyl-4-yl)methyl]amino}pyridine

The compound resulting from Example 84A (1.00 g, 1.52 mmol) was dissolved in ethanol (13 mL) containing 1.3 mL of concentrated hydrochloric acid. After 18 hours at ambient temperature, the ethanol was removed *in vacuo*. Potassium acetate was added to neutralize the remaining hydrochloric acid and the mixture extracted with chloroform. The combined organic extracts were dried over sodium sulfate and the solvent removed under reduced pressure. The residue obtained was crystallized from ether to give 0.53 g of the title compound. $^1H$ NMR (DMSO-$d_6$, 300 MHz) δ 0.72 (t, J = 7Hz, 3H), 1.50 (m, 2H), 3.18 (t, J = 7Hz, 2H), 4.71 (s, 2H), 6.89 (dd, J = 4Hz, 6Hz, 1H), 7.03 (d, J = 8Hz, 2H), 7.19 (d, J = 8Hz, 2H), 7.50-7.70 (m, 4H), 8.18 (dd, J = 1Hz, 8Hz, 1H), 8.40 (dd, J = 1Hz, 6Hz, 1H).

Example 85

Ethyl 6-methyl-2-{N-butyl-N-[(2'-[1H-tetrazol-5-yl]biphenyl-4-yl)methyl]amino}pyridine-3-carboxylate

Example 85A

Ethyl 2-chloro-6-methyl pyridine-3-carboxylate

To 2-hydroxy-6-methyl pyridine-3-carboxylic acid (25 g, 0.163 mol) suspended in 50 mL of phosphorous oxychloride and cooled in an ice bath was added phosphorous pentachloride (68 g, 0.313 mol) in portions. The mixture was stirred at 115 °C for 2 hours. The phosphorous oxychloride was removed *in vacuo* and chased with toluene. Ethanol (80 mL) was added with cooling, and the solution was refluxed for 20 minutes. The ethanol was removed under reduced pressure and the residue obtained dissolved in toluene. The toluene solution was washed with sodium bicarbonate solution, dried over magnesium sulfate

and concentrated *in vacuo*. The residue obtained was dissolved in heptane, stirred with silica gel and filtered. The filtrate was concentrated *in vacuo* to afford 25.10 g of the title compound.

Example 85B

Ethyl 2-butylamino-6-methyl pyridine-3-carboxylate

The compound resulting from Example 85A (15 g, 0.075 mol) was combined with 30 mL of butylamine and 60 mL of ethanol in a bomb and heated at 100 °C for 6 hours. The solution was concentrated *in vacuo*. The residue obtained was dissolved in toluene, washed with dilute potassium hydroxide solution, dried over sodium sulfate and concentrated *in vacuo* to give an oil. Purification by column chromatography on silica gel eluting with 5% ethyl acetate in hexane afforded 15.58 g of the title compound.

Example 85C

Ethyl 6-methyl-2-{N-butyl-N-[(2'-[N-triphenylmethyl-1H-tetrazol-5-yl]biphenyl-4-yl)methyl]amino}pyridine-3-carboxylate

The compound resulting from Example 85B (1.60 g, 6.77 mmol) in 8 mL of tetrahydrofuran and 1,3-dimethyl-3,4,5,6-tetrahydro-2(1H)-pyrimidinone (1.65 g) dissolved in tetrahydrofuran (5 mL) were combined and cooled in an ice bath. Lithium hexamethyldisilazide (6.51 mL of a 1 *M* solution in tetrahydrofuran) was added dropwise and the resulting yellow solution was stirred for 10 minutes at 0 °C. N-Triphenylmethyl-5-[2-(4'-bromomethylbiphenyl)]tetrazole (3.55 g, 6.37 mmol) was dissolved in 8 mL of tetrahydrofuran and added slowly to the above solution. The solution was stirred for 1.5 hours at ambient temperature. Concentrated hydrochloric acid (2 drops) was added, and the solution was concentrated *in vacuo*. Water was added and the mixture extracted with toluene. The combined organic extracts were washed with water (2x), dried over sodium sulfate and concentrated *in vacuo*. The residue obtained was chromatographed on silica gel eluting with 2% ether in toluene to give 2.41 g of the title compound. m.p. 120-122 °C (crystallized from heptane-ether).

Example 85D

Ethyl 6-methyl-2-{N-butyl-N-[(2'-[1H-tetrazol-5-yl]biphenyl-4-yl)methyl]amino}pyridine-3-carboxylate

The compound resulting from Example 85C (2.35 g) was treated with hydrogen chloride in ethanol by the procedure described in Example 18C. The product was purified by chromatography on silica gel eluting with 25% ethyl acetate in toluene containing 0.5% formic acid to afford the title compound (1.38 g). $^1$H NMR ($CDCl_3$, 300 MHz) δ 0.98 (t, J = 7Hz, 3H), 1.26 (m, 2H), 1.31 (t, J = 7Hz, 3H), 1.62 (m, 2H), 2.41 (s, 3H), 3.40 (t, J = 7Hz, 2H), 4.27 (q, J = 7Hz, 2H), 4.77 (s, 2H), 6.57 (d, J = 8Hz, 1H), 7.12 (d, J = 8Hz, 2H), 7.31 (d, J = 8Hz, 2H), 7.41 (dd, J = 8Hz, 1Hz, 1H), 7.50-7.60 (m, 2H), 7.81 (d, J = 8Hz, 1H), 8.24 (dd, J = 8Hz, 1Hz, 1H).

Example 86

6-Methyl-2-{N-butyl-N-[(2'-[1H-tetrazol-5-yl]biphenyl-4-yl)methyl]amino}pyridine-3-carboxylic acid

To the compound resulting from Example 85D (850 mg) dissolved in ethanol (40 mL) was added a solution of 0.90 g of potassium hydroxide dissolved in 5 mL of water. The reaction mixture was refluxed 90 minutes, acetic acid (4 mL) was added and the solution was concentrated *in vacuo*. Water was added and the resulting solid filtered. This solid was dissolved in chloroform; the solution was dried over sodium sulfate and concentrated *in vacuo.* The residue obtained was crystallized from ether to give 530 mg of the title compound. m.p. 175-177 °C. $^1$H NMR ($CDCl_3$, 300 MHz) δ 0.91 (t, J = 7Hz, 3H), 1.39 (m, 2H), 1.51 (bm, 2H), 2.65 (s, 2H), 3.50 (bm, 2H), 4.30 (s, 2H), 6.84 (d, J = 8Hz, 2H), 6.95 (d, J = 8Hz, 2H), 7.26 (d, J = 8Hz, 1H), 7.40 (dd, J = 8Hz, 1Hz, 1H), 7.45-7.55 (m, 2H), 7.92 (dd, J = 8Hz, 1Hz, 1H), 8.40 (d, J = 8Hz, 1H).

Example 87

Ethyl 2-{N-propyl-N-[(2'-[1H-tetrazol-5-yl]biphenyl-4-yl)methyl]amino}pyridine-3-carboxylate

Example 87A

Ethyl 2-chloropyridine-3-carboxylate

2-Chloropyridine-3-carboxylic acid (25 g) was refluxed 3 hours in 200 mL of benzene containing 150 mL of thionyl chloride. The reaction mixture was concentrated *in vacuo* and chased with toluene. The residue obtained was treated with 100 mL of ethanol and refluxed an additional 20 minutes. The ethanol was removed under reduced pressure and the product was dissolved in toluene. The toluene solution was dried over magnesium sulfate and concentrated *in vacuo* to give the product as a colorless oil which was used without further purification in the next step.

Example 87B

Ethyl 2-Propylamino-pyridine-3-carboxylate

The compound resulting from Example 87A (10.0 g) was treated with 20 mL of propylamine in 40 mL of ethanol according the the procedure described in Example 85B to afford the title compound (9.10 g) as a colorless oil.

Example 87C

Ethyl 2-{N-propyl-N-[(2'-[N-triphenylmethyl-1H-tetrazol-5-yl]biphenyl-4-yl)methyl]amino}pyridine-3-carboxylate

To the compound resulting from Example 87B (1.41 g, 6.78 mmol) dissolved in 5 mL of tetrahydrofuran was added 1.65 g of 1,3-dimethyl-3,4,5,6-tetrahydro-2(1H)-pyrimidinone. This mixture was treated with 6.78 mL of 1 *M* lithium hexamethyldisilazide followed by 3.55 g (6.35 mmol) of N-triphenylmethyl-5-[2-(4'-bromomethyl-biphenyl)]tetrazole by the procedure described in Example 85C to give 2.25 g of the title compound. m.p. 129-131 °C.

Example 87D

Ethyl 2-{N-propyl-N-[(2'-[1H-tetrazol-5-yl]biphenyl-4-yl)methyl]amino}pyridine-3-carboxylate

The compound resulting from Example 87C (2.00 g, 2.92 mmol) was treated with hydrogen chloride in ethanol by the procedure described in Example 18C to give the title compound (1.09 g) in 87% yield. $^1H$ NMR ($CDCl_3$, 300 MHz) $\delta$ 0.82 (t, J = 7Hz, 3H), 1.35 (t, J = 7Hz, 3H), 1.60 (m, 2H), 3.23 (t, J = 7Hz, 2H), 4.30 (q, J = 7Hz, 2H), 4.60 (s, 2H), 6.70 (dd, J = 8Hz, 4Hz, 1H), 7.10 (d, J = 8Hz, 2H), 7.24 (d, J = 8Hz, 2H), 7.42 (dd, J = 8Hz, 1Hz, 1H), 7.50-7.65 (m, 2H), 7.91 (dd, J = 8Hz, 2Hz, 1H), 8.04 (dd, J = 4Hz, 2Hz, 1H), 8.13 (dd, J = 8Hz, 1Hz, 1H).

Example 88

2-{N-Propyl-N-[(2'-[1H-tetrazol-5-yl]biphenyl-4-yl)methyl]amino}pyridine-3-carboxylic acid

The compound of Example 87D (400 mg) was converted to the title compound using the procedure described in Example 86 to afford the title compound (278 mg) in 74%. m.p. 202-204 °C. $^1H$ NMR (DMSO-$d_6$, 300 MHz) $\delta$ 0.73 (t, J = 7Hz, 3H), 1.50 (m, 2H), 3.22 (t, J = 7Hz, 2H), 4.67 (s, 2H), 6.80 (dd, J = 8Hz, 4Hz, 1H), 7.11 (d, J = 8Hz, 2H), 7.21 (d, J = 8Hz, 2H), 7.50-7.70 (m, 4H), 7.76 (dd, J = 8Hz, 2Hz, 1H), 8.21 (dd, J = 4Hz, 2Hz, 1H).

Example 89

Ethyl 6-methyl-2-{N-propyl-N-[(2'-[1H-tetrazol-5-yl]biphenyl-4-yl)methyl]amino}pyrazine-3-carboxylate

Example 89A

Ethyl 2-hydroxy-6-methylpyrazine-3-carboxylate

2-Hydroxy-6-methylpyrazine-3-carboxylic acid (32.5 g), prepared as described in *J. Chem.* Soc. **1955**, 1379, was suspended in 500 mL of ethanol. The solution was cooled in an ice bath and hydrogen chloride gas was bubbled into the solution for 15 minutes. The mixture was stirred overnight at ambient temperature and then refluxed for 2 hours. The ethanol was removed under reduced pressure and the residue obtained crystallized from ethanol/ether to give 21.48 g of the title compound. m.p. 153 °C.

Example 89B

Ethyl 6-methyl-2-{N-propyl-N-[(2'-[N-triphenylmethyl-1H-tetrazol-5-yl]biphenyl-4-yl)methyl]amino}pyrazine-3-carboxylate

To the compound resulting from Example 89A (1.43 g, 7.86 mml) suspended in 8 mL of dimethylformamide was added triethylamine (1.8 g). To the resulting solution was added benzenesulfonyl chloride (1.45 g, 8.21 mmol). The mixture was stirred for 5 minutes at ambient temperature. N-Triphenylmethyl-5-[2-(4'-propylaminomethyl-biphenyl)]tetrazole (3.89 g, 7.17 mmol) dissolved in 3 mL of toluene was then added. The mixture was stirred at 45 °C for 18 hours. Dilute potassium bicarbonate was added and the mixture was extracted with toluene. The combined organic extracts were washed with sodium chloride solution, dried over sodium sulfate and concentrated under reduced pressure. The residue obtained was chromatographed on silica gel eluting with 15% ethyl acetate in toluene to give 3.00 g of the title compound in 60% yield. m.p. 110-112 °C.

Example 89C

Ethyl 6-methyl-2-{N-propyl-N-[(2'-[1H-tetrazol-5-yl]biphenyl-4-yl)methyl]amino}pyrazine-3-carboxylate

The compound resulting from Example 89B (3.00 g, 1.43 mmol) was treated with hydrogen chloride in ethanol by the procedure described in Example 18C to give the title compound (1.08 g). m.p. 172-174 °C (crystallized from acetonitrile). $^1H$ NMR ($CDCl_3$, 300 MHz) δ 0.77 (t, J = 7Hz, 3H), 1.17 (t, J = 7Hz, 3H), 1.52 (m, 2H), 2.50 (s, 3H), 3.21 (t, J = 7Hz, 2H), 4.15 (q, J = 7Hz, 2H), 4.72 (s, 2H), 7.10 (d, J = 8Hz, 2H), 7.20 (d, J = 8Hz, 2H), 7.44 (dd, J = 8Hz, 1Hz, 1H), 7.50-7.65 (m, 2H), 7.65 (s, 1H), 8.12 (dd, J = 8Hz, 1Hz, 1H).

Example 90

6-Methyl-2-[N-propyl-N-[(2'-(1H-tetrazol-5-yl]biphenyl-4-yl)methyl]amino}pyrazine-3-carboxylic acid

The compound resulting from Example 89C (250 mg) was converted to the title compound by the procedure described in Example 86 to give 253 mg of the title compound which crystallized with one equivalent of ether. $^1H$ NMR ($CDCl_3$, 300 MHz) δ 0.90 (t, J = 7Hz, 3H), 1.20 (t, J = 7Hz, 6H), 1.62 (m, 2H), 2.52 (s, 3H), 3.48 (q, J = 7Hz, 4H), 3.52 (t, J = 7Hz, 2H), 4.66 (s, 2H), 7.05 (d, J = 8Hz, 2H), 7.15 (d, J = 8Hz, 2H), 7.40 (dd, J = 8Hz, 1Hz, 1H), 7.45-7.60 (m, 4H), 7.91 (s, 1H), 8.01 (dd, J = 8Hz, 1Hz, 1H).

Example 91

2-{N-Butyl-N-[(2'-[1H-tetrazol-5-yl]biphenyl-4-yl)methyl]amino}-4,6-dimethylpyrimidine

Example 91A

2-{N-Butyl-N-[(2'-[N-triphenylmethyl-1H-tetrazol-5-yl]biphenyl-4-yl)methyl]amino}-4,6-dimethylpyrimidine

2-Butylamino-4,6-dimethylpyrimidine (968 mg, 5.41 mmol), prepared as described by D.J. Brown and J.M. Lyall, *Aust. J. Chem.* **1964** *17*: 794, and 1,3-dimethyl-3,4,5,6-tetrahydro-2(1H)-pyrimidinone (1.38 mL) were dissolved in 2 mL of tetrahydrofuran. While cooling in an ice bath, 5.5 mL of a 1 *M* solution of lithium hexamethyldisilazide in tetrahydrofuran was added. After 12 minutes at 0 °C, a solution of 2.52 g (4.52 mmol) of N-triphenylmethyl-5-[2-(4'-bromomethyl-biphenyl)]tetrazole in 8 mL of tetrahydrofuran was added. The solution was stirred for 90 minutes at ambient temperature and then quenched by the addition of

concentrated hydrochloric acid (2 drops). The reaction mixture was concentrated *in vacuo* and the residue obtained dissolved in toluene. The toluene solution was washed with dilute sodium hydroxide solution and water, dried over sodium sulfate and concentrated *in vacuo*. The residue obtained was chromatographed on silica gel eluting with 2% ether in toluene to give 1.65 g of the title compound. m.p. 131-133 °C.

Example 91B

2-{N-Butyl-N-[(2'-[1H-tetrazol-5-yl]biphenyl-4-yl)methyl]amino}-4,6-dimethylpyrimidine

The compound resulting from Example 91A (1.50 g, 2.29 mmol) was dissolved in 14 mL of methylene chloride and 21 mL of 88% formic acid. After 1 hour at ambient temperature, the solution was concentrated *in vacuo*. Water was added and the mixture was concentrated again, and the residue obtained was extracted with ether. The combined organic extracts were then extracted with potassium hydroxide solution. The combined aqueous extracts were acidified with formic acid. The oil which separated was extracted with chloroform and the combined organic extracts were dried over sodium sulfate and concentrated *in vacuo*. The residue obtained was crystallized from ether to afford 708 mg of the title compound. m.p. 148-150 °C. $^1$H NMR ($CDCl_3$, 300 MHz) δ 0.92 (t, J = 7Hz, 3H), 1.32 (m, 2H), 1.60 (m, 2H), 2.27 (s, 6H), 3.62 (t, J = 7Hz, 2H), 4.88 (s, 2H), 6.26 (s, 1H), 7.09 (d, J = 8Hz, 2H), 7.20 (d, J = 8Hz, 2H), 7.38 (dd, J = 8Hz, 1Hz, 1H), 7.45-7.60 (m, 2H), 8.13 (dd, J = 8Hz, 1Hz, 1H).

Example 92

4-{N-Butyl-N-[(2'-[1H-tetrazol-5-yl]biphenyl-4-yl)methyl]amino}-2,6-dimethylpyrimidine

Example 92A

4-{N-Butyl-N-[(2'-[N-triphenylmethyl-1H-tetrazol-5-yl]biphenyl-4-yl)methyl]amino}-2,6-dimethylpyrimidine

4-[N-Butylamino-2,6-dimethylpyrimidine (806 mg, 4.50 mmol), prepared as described by D.J. Brown and J.M. Lyall, *Aust. J. Chem*. **1964**, *17*: 794, was reacted by the procedure described in Example 91A. The crude product was purified by chromatography on silica gel eluting with 40% ethyl acetate in toluene to afford the title compound (1.55 g). m.p.125-127 °C.

Example 92B

4-{N-Butyl-N-[(2'-[1H-tetrazol-5-yl]biphenyl-4-yl)methyl]amino}-2,6-dimethylpyrimidine

The compound resulting from Example 92A (1.55 g, 2.37 mmol) was dissolved in 14 mL of methylene chloride and 21 mL of 88% formic acid. After 1 hour at ambient temperature, the solution was concentrated *in vacuo*. The residue obtained was treated with 50 mL of water and the solid obtained removed by filtration. The filtrate was neutralized to pH 8 with sodium bicarbonate and then acidified with 0.5 mL acetic acid. The solid which separated was dissolved in chloroform, and the solution obtained was dried over sodium sulfate and concentrated *in vacuo* to afford 803 mg of the title compound. $^1$H NMR (DMSO-$d_6$, 300 MHz) δ 0.90 (t, J = 7Hz, 3H), 1.29 (m, 2H), 1.50 (m, 2H), 2.21 (s, 3H), 2.34 (s, 3H), 3.42 (bm, 2H), 4.77 (bs, 2H), 6.42 (s, 1H), 7.05 (d, J = 8Hz, 2H), 7.12 (d, J = 8Hz, 2H), 7.45-7.58 (m, 4H).

Example 93

3-Amino-2-{N-propyl-N-[(2'-[1H-tetrazol-5-yl]biphenyl-4-yl)methyl]amino}-2,6-dimethylpyridine hydrochloride

Example 93A

3-Amino-2-{N-propyl-N-[(2'-[N-triphenylmethyl-1H-tetrazol-5-yl]biphenyl-4-yl)methyl]amino}-2,6-dimethylpyridine hydrochloride

The compound resulting from Example 84A (2.00 g) was dissolved in 250 mL of ethyl acetate and hydrogenated using 200 mg of 10% palladium on carbon as a catalyst. Upon completion of the reaction, the catalyst was removed by filtration and the filtrate concentrated *in vacuo*. The residue obtained was

chromatographed on silica gel eluting with 25% ethyl acetate in hexane to give 1.20 g of the title compound.

Example 93B

3-Amino-2-{N-propyl-N-[(2'-[1H-tetrazol-5-yl]biphenyl-4-yl)methyl]amino}-2,6-dimethylpyridine hydrochloride

The compound resulting from Example 93A (420 mg) was refluxed in a mixture of 7 mL of tetrahydrofuran, 7 mL of acetic acid, and 0.25 mL of water for 90 minutes. The solvents were removed under reduced pressure and the crude product chromatographed on silica gel eluting with 5% water and 5% formic acid in ethyl acetate. The residue obtained was treated with hydrochloric acid to give the title compound (100 mg). $^1H$ NMR ($CDCl_3$, 300 MHz) $\delta$ 0.98 (t. J = 7Hz, 3H), 1.21 (m, 2H), 3.55 (t, J = 7Hz, 2H), 4.23 (s, 2H), 4.60 (s, 2H), 6.75 (m, 2H), 7.09 (d, J = 8Hz, 2H), 7.25 (d, J = 8Hz, 2H), 7.40-7.60 (m, 3H), 7.85 (dd, 1H), 8.15 (dd, 1H).

Example 94

3-Methanesulfonamido-2-{N-propyl-N-[(2'-[1H-tetrazol-5-yl]biphenyl-4-yl)methyl]amino}pyridine

To the compound resulting from Example 93A (500 mg, 0.8 mmol) dissolved in 10 mL of methylene chloride containing 0.11 mL of triethylamine was added methanesulfonyl chloride (0.92 mL, 1.04 mmol). After 18 hours, the reaction mixture was washed with water, dried over magnesium sulfate and concentrated *in vacuo*. The residue obtained was chromatographed on silica gel eluting with 25% ethyl acetate in hexane to give the N-triphenylmethyl intermediate. This compound was refluxed in 12 mL of tetrahydrofuran containing 12 mL of acetic acid and 1 mL of water for 1 hour. The solvents were removed under reduced pressure and the residue obtained chromatographed on silica gel eluting with 10% methanol in methylene chloride to give the title compound. $^1H$ NMR ($CDCl_3$, 300 MHz) $\delta$ 0.95 (t, J = 8Hz, 3H), 1.45 (m, 2H), 3.05 (s, 3H), 3.15 (t J = 7Hz, 2H), 4.11 (s, 2H), 7.02 (d, J = 8Hz, 2H), 7.08 (d, J = 8Hz, 2H), 7.15 (dd, 1H), 7.42 (dd, J = 8Hz, 1Hz, 1H), 7.45-7.60 (m, 3H), 7.95 (dd, J = 8Hz, 1Hz, 1H), 8.19 (dd, 1H).

Example 95

Ethyl 4-{N-butyl-N-[(2'-[1H-tetrazol-5-yl]biphenyl-4-yl)methyl]amino}pyrimidine-5-acetate

Example 95A

Ethyl 4-{N-butyl-N-[(2'-[N-triphenylmethyl-1H-tetrazol-5-yl]biphenyl-4-yl)methyl]amino}pyrimidine-5-acetate

Ethyl 4-chloropyrimidine-5-acetate (300 mg, 1.5 mmol), prepared as described by G.G. Massarol and G. Signorelli, *Boll. Chim. Farm.* **1966**, *105(5)*: 400, N-triphenylmethyl-5-[2-(4''-butylaminomethyl-biphenyl)]-tetrazole (0.84 g, 1.5 mmol) and 1.5 mL of diisopropylethylamine in 3 mL of toluene were refluxed for 4 days. The product was chromatographed on silica gel to give 300 mg of the title compound.

Example 95B

Ethyl 4-{N-butyl-N-[(2'-[1H-tetrazol-5-yl]biphenyl-4-yl)methyl]amino}pyrimidine-5-acetate

The compound resulting from Example 95A (380 mg) was refluxed in 10 mL of tetrahydrofuran, 10 mL of acetic acid and 1 mL of water for 1 hour. The product was chromatographed on silica gel eluting with 10% ethanol in methylene chloride to give 130 mg of the title compound. $^1H$ NMR ($CDCl_3$, 300 MHz) $\delta$ 0.90 (t, J = 7Hz, 3H), 1.22 (t, J = 7Hz, 3H), 1.26 (m, 2H), 1.55 (m, 2H), 3.36 (t, J = 7Hz, 2H), 3.45 (s, 2H), 4.15 (q, J = 7Hz, 2H), 4.25 (s, 2H), 7.09 (d, J = 8Hz, 2H), 7.14 (d, J = 8Hz, 2H), 7.44 (dd, J = 8Hz, 1Hz, 1H), 7.50-7.65 (m, 2H), 7.72 (s, 1H), 8.00 (dd, J = 8Hz, 1Hz, 1H), 8.17 (s, 1H).

Example 96

Ethyl 2-methyl-4-{N-propyl-N-[(2'-[1H-tetrazol-5-yl]biphenyl-4-yl)methyl]amino}pyrimidine-5-carboxylate

Example 96A

N-Triphenylmethyl-5-[2-(4'-propylaminomethyl-biphenyl)]tetrazole

To N-triphenylmethyl-5-[2-(4'-bromomethyl-biphenyl)]tetrazole (2.8 g, 5 mmol) dissolved in tetrahydrofuran (50 mL) was added propylamine (2.5 mL). The reaction was stirred for 4 hours at ambient temperature and then concentrated *in vacuo*. The residue obtained was dissolved in ethyl acetate, washed with water and saturated sodium chloride, dried over sodium sulfate and concentrated under reduced pressure to afford the title compound.

Example 96B

Ethyl 2-methyl-4-{N-propyl-N-[(2'-[N-triphenylmethyl-1H-tetrazol-5-yl]biphenyl-4-yl)methyl]-amino}pyrimidine-5-carboxylate

The compound resulting from Example 96A (2.5 mmol) was dissolved in tetrahydrofuran (15 mL) containing N-methylmorpholine (1.1 mL). Ethyl 2-methyl-4-chloropyrimidine-5-carboxylate (502 mg, 2.5 mmol) was added and the reaction mixture stirred at ambient temperature for 16 hours. The reaction mixture was concentrated under reduced pressure and the residue obtained dissolved in ethyl acetate. This solution was washed with water and saturated brine, dried over sodium sulfate and concentrated under reduced pressure. The crude material was chromatographed on silica gel eluting with a gradient of ethyl acetate in toluene to afford the title compound as a light yellow oil which solidified on standing (1.2 g, 68%).

Example 96C

Ethyl 2-methyl-4-{N-propyl-N-[(2'-[1H-tetrazol-5-yl]biphenyl-4-yl)methyl]amino}pyrimidine-5-carboxylate

The compound resulting from Example 96B (200 mg, 0.286 mmol) was suspended in 5 mL of ethanol and hydrogen chloride saturated ethanol (500 µL) was added. After stirring for 2 hours at ambient temperature, the reaction mixture was concentrated under reduced pressure. The residue obtained was dissolved in ethyl acetate (50 mL), washed with water, dried over sodium sulfate and concentrated under reduced pressure to afford a light yellow oil. This oil was dissolved in ethyl acetate (20 mL) and hexane (200 mL) was added. The resulting solid was collected by filtration and dried under vacuum to afford the title compound as a colorless amorphous solid (75 mg, 57%). $^1H$ NMR ($CDCl_3$, 300 MHz) δ 0.84 (t, J = 7.5Hz, 3H), 1.33 (t, J = 7.5Hz, 3H), 1.60 (m, 2H), 2.35 (s, 3H), 3.39 (t, J = 7.5Hz, 2H), 4.30 (q, J = 7.5Hz, 2H), 4.78 (s, 2H), 7.04 (s, 4H), 7.45 (dd, J = 7.5Hz, 2Hz, 1H), 7.50-7.62 (m, 2H), 7.97 (dd, J = 7.5Hz, 2Hz, 1H), 8.16 (s, 1H).

Example 97

Ethyl 2-n-propyl-4-{N-propyl-N-[(2'-[1H-tetrazol-5-yl]biphenyl-4-yl)methyl]amino}pyrimidine-5-carboxylate

Example 97A

Ethyl 2-n-propyl-4-{N-propyl-N-[(2'-[N-triphenylmethyl-1H-tetrazol-5-yl]biphenyl-4-yl)methyl]-amino}pyrimidine-5-carboxylate

The compound resulting from Example 96A (1.2 g, 2.2 mmol) was dissolved in tetrahydrofuran (20 mL) containing N-methylmorpholine (1 mL). Ethyl 2-n-propyl-4-chloropyrimidine-5-carboxylate (563 mg, 2.5 mmol) was added and the reaction mixture stirred at ambient temperature for 60 hours. The reaction was worked up and purified by the procedure described in Example 96B to provide the title compound as a colorless amorphous solid (1.08 g, 60%).

Example 97B

Ethyl 2-n-propyl-4-{N-propyl-N-[(2'-[1H-tetrazol-5-yl]biphenyl-4-yl)methyl]amino}pyrimidine-5-carboxylate

The compound resulting from Example 97A (300 mg, 0.41 mmol) was deprotected by the procedure

described in Example 96C to afford the title compound as a colorless amorphous solid (120 mg, 60%). $^1$H NMR ($CDCl_3$, 300 MHz) δ 0.93 (t, J = 8Hz, 3H), 1.30 (t, J = 7.5Hz, 3H), 1.72 (m, 4H), 2.77 (t, J = 7.5Hz, 2H), 3.63 (bt, J = 8Hz, 2H), 4.26 (q, J = 7.5Hz, 2H), 4.86 (s, 2H), 7.00 (d, J = 9Hz, 2H), 7.08 (d, J = 9Hz, 2H), 7.40 (dd, J = 7.5Hz, 2Hz, 1H), 7.55 (m, 2H), 7.95 (dd, J = 7.5Hz, 2Hz, 1H), 8.39 (s, 1H).

Example 98

2-n-Propyl-4-{N-propyl-N-[(2'-[1H-tetrazol-5-yl]biphenyl-4-yl)methyl]amino}pyrimidine-5-carboxylic acid

To the compound resulting from Example 97 (630 mg, 1.3 mmol) dissolved in ethanol (25 mL) was added a solution of sodium hydroxide (520 mg, 13 mmol) in 2.5 mL of water. The mixture was refluxed for 2.5 hours, cooled to ambient temperature and concentrated under reduced pressure. The residue obtained was suspended in water (50 mL) and washed with ether. The aqueous phase was acidified with 12 *N* hydrochloric acid and extracted with methylene chloride. The combined organic extracts were washed with saturated brine, dried over sodium sulfate and concentrated under reduced pressure to afford the title compund as an amorphous solid (440 mg, 74%). $^1$H NMR (DMSO-$d_6$, 300 MHz) δ 0.76 (t, J = 7.5Hz, 3H), 0.85 (t, J = 7.5Hz, 3H), 1.55 (m, 2H), 1.65 (m, 2H), 2.65 (t, J = 7.5Hz, 2H), 3.38 (m, 2H), 4.85 (s, 2H), 7.04 (d, J = 8Hz, 2H), 7.18 (d, J = 8Hz, 2H), 7.50-7.70 (m, 4H), 8.50 (s, 1H).

Example 99

2-Methyl-4-{N-propyl-N-[(2'-[1H-tetrazol-5-yl]biphenyl-4-yl)methyl]amino}pyrimidine-5-carboxylic acid

The compound resulting from Example 96 (250 mg, 0.546 mmol) was hydrolyzed and worked up according to the procedure described in Example 98. The crude product was recrystallized from methylene chloride and hexane to afford the title compound as a colorless amorphous solid (140 mg, 55%). $^1$H NMR ($CD_3OD$, 300 MHz) δ 0.90 (t J = 7.5Hz, 3H), 1.72 (m, 2H), 2.59 (s, 3H), 3.70 (bt, J = 7.5Hz, 2H), 5.07 (s, 2H), 7.10 (d, J = 9Hz, 2H), 7.22 (bd, J = 9Hz, 2H), 7.57 (dt, J = 7.5Hz, 1Hz, 2H), 7.68 (m, 2H), 8.46 (s, 1H).

Example 100

Ethyl 2-methyl-4-{N-pentyl-N-[(2'-[1H-tetrazol-5-yl]biphenyl-4-yl)methyl]amino}pyrimidine-5-carboxylate

Example 100A

N-Triphenylmethyl-5-[2-(4'-pentylaminomethyl-bipheny)]tetrazole

Using the procedure described in Example 96A, N-triphenylmethyl-5-[2-(4'-bromomethyl-biphenyl)]-tetrazole (3.00 g, 5.4 mmol) was reacted with n-pentylamine (6 mL) in tetrahydrofuran (50 mL) to afford the title compound as a yellow oil (3.00 g, 98%).

Example 100B

Ethyl 2-methyl-4-{N-pentyl-N-[(2'-[N-triphenylmethyl-1H-tetrazol-5-yl]biphenyl-4-yl)methyl]-amino}pyrimidine-5-carboxylate

Using the procedure described in Example 96B, the compound resulting from Example 100A (2.00 g, 3.5 mmol), ethyl 2-methyl-4-chloropyrimidine-5-carboxylate (703 mg, 3.5 mmol), and N-methylmorpholine (2 mL) in tetrahydrofuran (30 mL) were stirred for 60 hours at ambient temperature. Work up and chromatography using ethyl acetate and toluene mixtures provided the title compound as a colorless amorphous solid (1.00 g, 47%).

Example 100C

Ethyl 2-methyl-4-{N-pentyl-N-[(2'-[1H-tetrazol-5-yl]biphenyl-4-yl)methyl]amino}pyrimidine-5-carboxylate

To the compound resulting from Example 100B (1.00 g, 1.3 mmol) dissolved in tetrahydrofuran (25 mL)

was added acetic acid (4 mL) and water (1 mL). The reaction mixture was refluxed for 24 hours and then the cooled reaction mixture was concentrated under reduced pressure. The residue obtained was dissolved in ethyl acetate (200 mL) and washed with water and saturated sodium chloride, dried over sodium sulfate and concentrated under reduced pressure. The residue obtained was triturated with ether and the resulting solid collected by filtration to afford the title compund as a colorless amorphous solid (390 mg, 62%). $^1$H NMR (DMSO-$d_6$, 300 MHz) δ 0.82 (t, J = 7.5Hz, 3H), 1.10-1.28 (m, 4H), 1.24 (t, J = 7.5Hz, 3H), 1.50 (m, 2H), 2.40 (s, 3H), 3.30 (m, 2H), 4.20 (q, J = 7.5Hz, 2H), 4.80 (s, 2H), 7.05 (d, J = 8Hz, 2H), 7.16 (d, J = 8Hz, 2H), 7.50-7.70 (m, 4H), 8.40 (s, 1H).

Example 101

2-Methyl-4-{N-pentyl-N-[(2'-[1H-tetrazol-5-yl]biphenyl-4-yl)methyl]amino}pyrimidine-5-carboxylic acid

The compound resulting from Example 99 (250 mg, 0.51 mmol) was hydrolyzed with sodium hydroxide (206 mg) using the procedure described in Example 98. The crude product was dissolved in methylene chloride and hexane was added. The resulting solid was collected by filtration to afford the title compound as an amorphous solid (150 mg, 59%). $^1$H NMR ($CD_3OD$, 300 MHz) δ 0.90 (t, J = 7.5Hz, 3H), 1.30 (m, 4H), 1.70 (m, 2H), 2.55 (s, 3H), 3.72 (t, J = 7.5Hz, 2H), 5.07 (s, 2H), 7.10 (d, J = 8Hz, 2H), 7.22 (d, J = 8Hz, 2H), 7.56 (m, 2H), 7.67 (m, 2H), 8.27 (s, 1H).

Example 102

Ethyl 2-methyl-4-{N-(2-methylpropyl)-N-[(2'-[1H-tetrazol-5-yl]biphenyl-4-yl)methyl]amino}pyrimidine-5-carboxylate

Example 102A

N-Triphenylmethyl-5-{2-[4'-(2-methylpropyl)aminomethyl]}tetrazole

Using the procedure described in Example 96A, N-triphenylmethyl-5-[2-(4'-bromomethyl-biphenyl)]-tetrazole (3.00 g, 5.4 mmol) in tetrahydrofuran (50 mL) was treated with isobutylamine (3.9 g) and worked up to afford the title compound as a light yellow amorphous solid (3.00 g).

Example 102B

Ethyl 2-methyl-4-{N-(2-methylpropyl)-N-[(2'-[N-triphenylmethyl-1H-tetrazol-5-yl]biphenyl-4-yl)methyl]-amino}pyrimidine-5-carboxylate

Using the procedure described in Example 100B, the compound resulting from Example 102A (1.5 g), ethyl 2-methyl-4-chloropyrimidine-5-carboxylate (543 mg, 2.7 mmol) and N-methylmorpholine (3 mL) were mixed in tetrahydrofuran (25 mL) and stirred for 18 hours at ambient temperature. Work up and chromatography eluting with ethyl acetate and toluene mixtures afforded the title compound as an amorphous solid (1.18 g, 81%).

Example 102C

Ethyl 2-methyl-4-{N-(2-methylpropyl)-N-[(2'-[1H-tetrazol-5-yl]biphenyl-4-yl)methyl]amino}pyrimidine-5-carboxylate

The compound resulting from Example 102B (1.15 g, 1.6 mmol) was dissolved in tetrahydrofuran (20 mL). Acetic acid (10 mL) and water (1 mL) were added and the reaction mixture was refluxed for 4 hours. The cooled reaction mixture was concentrated under reduced pressure and the residue obtained dissolved in ethyl acetate. The organic layer was washed with water and brine, dried over sodium sulfate and concentrated under reduced pressure. Trituration of the residue with ether/hexane afforded the title compound as a colorless amorphous solid (670 mg, 88%). $^1$H NMR ($CDCl_3$, 300 MHz) δ 0.80 (d, J = 7.5Hz, 6H), 1.38 (t, J = 7.5Hz, 3H), 1.95 (m, 1H), 2.20 (s, 3H), 3.18 (bd, J = 7.5Hz, 2H), 3.38 (q, J = 7.5Hz, 2H), 4.62 (s, 2H), 6.98 (d, J = 9Hz, 2H), 7.01 (d, J = 9Hz, 2H), 7.46 (dd, J = 7.5Hz, 2Hz, 2H), 7.52-7.63 (m, 4H), 8.00 (s, 1H), 8.13 (dd, J = 7.5Hz, 2Hz, 1H).

Example 103

2-Methyl-4-{N-(2-methylpropyl)-N-[(2'-[1H-tetrazol-5-yl]biphenyl-4-yl)methyl]amino}pyrimidine-5-carboxylic acid

The compund resulting from Example 102 (300 mg, 0.64 mmol) was hydrolyzed with sodium hydroxide (254 mg) by the procedure described in Example 98. The crude product was dissolved in tetrahydrofuran (50 mL) and hexane (100 mL) was added. The solid obtained was filtered to afford the title compound as a colorless amorphous solid (240 mg, 86%). $^1H$ NMR (DMSO-$d_6$, 300 MHz) $\delta$ 0.77 (d, J = 7Hz, 6H), 2.00 (m, 1H), 3.30 (d, J = 7.5Hz, 2H), 4.85 (s, 2H), 7.06 (d, J = 9Hz, 2H), 7.18 (d, J = 9Hz, 2H), 7.50-7.60 (m, 2H), 7.60-7.70 (m, 3H), 8.55 (s, 1H).

Example 104

Ethyl 2-methyl-4-{N-(3-methylbutyl)-N-[(2'-[1H-tetrazol-5-yl]biphenyl-4-yl)methyl]amino}pyrimidine-5-carboxylate

Example 104A

N-Triphenylmethyl-5-{2-[4'-(2-methylbutyl)aminomethyl-biphenyl]}tetrazole

Using the procedure described in Example 96A, N-triphenylmethyl-5-[2-(4'-bromomethyl-biphenyl)]-tetrazole (1.5 g, 2.7 mmol) and isoamylamine (1.7 mL) in tetrahydrofuran (25 mL) were reacted to afford the title compound as a pale yellow amorphous solid.

Example 104B

Ethyl 2-methyl-4-{N-(3-methylbutyl)-N-[(2'-[N-triphenylmethyl-1H-tetrazol-5-yl]biphenyl-4-yl)methyl]-amino}pyrimidine-5-carboxylate

Using the procedure described in Example 96B, the compound resulting from Example 104A was reacted with ethyl 2-methyl-4-chloropyrimidine-5-carboxylate and N-methylmorpholine (2 mL) in tetrahydrofuran (25 mL) for 72 hours. Normal work up and chromatography eluting with ethyl acetate/toluene mixtures afforded the title compound as a colorless oil (1.3 g, 87%).

Example 104C

Ethyl 2-methyl-4-{N-(3-methylbutyl)-N-[(2'-[1H-tetrazol-5-yl]biphenyl-4-yl)methyl]amino}pyrimidine-5-carboxylate

The compound resulting from Example 104B (1.3 g, 1.8 mmol) was deprotected using acetic acid (20 mL) and water (1 mL) in refluxing tetrahydrofuran (20 mL) by the procedure described in Example 102. The crude product was dissolved in methylene chloride and hexane was added. Filtration of the resulting solid afforded the title compound as an amorphous solid (640 mg, 74%). $^1H$ NMR ($CDCl_3$, 300 MHz) $\delta$ 0.82 (d, J = 6Hz, 6H), 1.32 (t, J = 6Hz, 3H), 1.42 (m, 3H), 2.27 (s, 3H), 3.41 (bt, J = 7.5Hz, 2H), 4.30 (q, J = 7.5Hz, 2H), 4.74 (s, 2H), 7.02 (d, J = 8Hz, 2H), 7.07 (d, J = 8Hz, 2H), 7.44 (dd, J = 7Hz, 2Hz, 1H), 7.51-7.62 (m, 2H), 8.05 (dd, J = 7Hz, 2Hz, 1H), 8.07 (s, 1H).

Example 105

2-Methyl-4-{N-(3-methylbutyl)-N-[(2'-[1H-tetrazol-5-yl]biphenyl-4-yl)methyl]amino}pyrimidine-5-carboxylic acid

Following the procedure described in Example 98, the compound resulting from Example 104C (270 mg, 0.56 mmol) was hydrolyzed using sodium hydroxide (230 mg). The crude product was dissolved in methylene chloride and hexane was added. Filtration of the solid obtained afforded the title compound as an amorphous solid (200 mg, 80%). $^1H$ NMR (DMSO-$d_6$, 300 MHz) $\delta$ 0.82 (d, J = 6Hz, 6H), 1.40 (m, 3H), 2.46 (s, 3H), 3.47 (bt, J = 6Hz, 2H), 4.88 (s, 2H), 7.06 (d, J = 8Hz, 2H), 7.20 (d, J = 8Hz, 2H), 7.50-7.70 (m,

4H), 8.52 (s, 1H).

Example 106

Ethyl 2-methyl-4-{N-[1-(2-butenyl)]-N-[(2'-[1H-tetrazol-5-yl]biphenyl-4-yl)methyl]amino}pyrimidine-5-carboxylate

The compound resulting from Example 65A (600 mg, 0.91 mmol) was dissolved in anhydrous dimethylformamide (12 mL) under a nitrogen atmosphere. The solution was cooled to 0 °C and crotyl bromide (500 µL, 4.5 mmol) was added followed by 60% oil dispersion sodium hydride (54 mg, 1.4 mmol) in portions. After 1 hour at 0 °C, the cooling bath was removed and the reaction was stirred at ambient temperature for 30 minutes. The reaction mixture was cautiously poured into saturated aqueous ammonium chloride solution (100 mL) and extracted with ethyl acetate (3 x 100 mL). The combined organic ectracts were washed with water and brine, dried over sodium sulfate and sodium carbonate, and concentrated under reduced pressure. Chromatography using ethyl acetate and toluene mixtures provided an orange foam (570 mg, 88%). The foam was deprotected using acetic acid (5 mL) and water (1 mL) in tetrahydrofuran (10 mL) according to the procedure described in Example 100C. Chromatography eluting with ethanol/methylene chloride mixtures afforded an off-white solid. Trituration with ethyl acetate/hexane (1:7) afforded the title compound as an amorphous solid (130 mg, 34%). MS ($DCl/NH_3$) m/e 470 $(M+H)^+$.

Example 107

Ethyl 2-methyl-4-{N-[1-(2-propenyl)]-N-[(2'-[1H-tetrazol-5-yl]biphenyl-4-yl)methyl]amino}pyrimidine-5-carboxylate

Example 107A

N-Triphenylmethyl-5-[2-(4'-[1-(2-propenyl)]aminomethyl-biphenyl)]tetrazole

Following the procedure described in Example 96A, N-triphenylmethyl-5-[2-(4'-bromomethyl-biphenyl)]-tetrazole (2.00 g, 3.6 mmol) and allylamine (3 mL) were reacted in tetrahydrofuran (10 mL). Normal work up afforded the title compound as an amorphous solid.

Example 107B

Ethyl 2-methyl-4-{N-[1-(2-propenyl)]-N-[(2'-[N-triphenylmethyl-1H-tetrazol-5-yl]biphenyl-4-yl)methyl]-amino}pyrimidine-5-carboxylate

The compound resulting from Example 107A was reacted with ethyl 2-methyl-4-chloropyrimidine-5-carboxylate (720 mg, 3.6 mmol) and N-methylmorpholine (2 mL) in tetrahydrofuran (15 mL). Normal work up and chromatography using ethyl acetate/tolune mixtures afforded the title compound as an amorphous solid (1.5 g, 80%).

Example 107C

Ethyl 2-methyl-4-{N-[1-(2-propenyl)]-N-[(2'-[1H-tetrazol-5-yl]biphenyl-4-yl)methyl]amino}pyrimidine-5-carboxylate

The compound resulting from Example 107B (1.5 g, 2.1 mmol) was deblocked with acetic acid (30 mL) and water (3 mL) in refluxing tetrahydrofuran (30 mL) for 3 hours according to the procedure described in Example 100C. Normal work up afforded a colorless solid which was triturated with hexane to afford a partially purified product. Recrystallization from methylcyclohexane afforded the title compound as an amorphous solid (260 mg, 27%). $^1$H NMR ($CDCl_3$, 300 MHz) δ 1.30 (t, J = 7.5Hz, 3H), 2.27 (s, 3H), 3.97 (d, J = 6Hz, 2H), 4.27 (q, J = 7.5Hz, 2H), 4.82 (s, 2H), 5.08-5.14 (m, 2H), 5.70-5.85 (m, 1H), 7.02 (d, J = 9Hz, 2H), 7.06 (d, J = 9Hz, 2H), 7.46 (dd, J = 7.5Hz, 2Hz, 1H), 7.52-7.63 (m, 2H), 8.03 (dd, J = 7.5Hz, 2Hz, 1H), 8.12 (s, 1H).

Example 108

2-Methyl-4-{N-[1-(2-propenyl)]-N-[(2'-[1H-tetrazol-5-yl]biphenyl-4-yl)methyl]amino}pyrimidine-5-carboxylic acid

The compound resulting from Example 107 (660 mg, 1.45 mmol) was hydrolyzed with sodium hydroxide (580 mg, 14.5 mmol) by the procedure described in Example 98. The crude product was dissolved in ethanol and ether was added. Filtration of the solid afforded the title compound as an amorphous solid (210 mg, 34%). $^1$H NMR (DMSO-$d_6$, 300 MHz) δ 2.50 (s, 3H), 4.10 (d, J = 6Hz, 2H), 4.87 (s, 2H), 5.11-5.25 (m, 2H), 5.70-5.85 (m, 1H), 7.05 (d, J = 7.5Hz, 2H), 7.21 (d, J = 7.5Hz, 2H), 7.52-7.70 (m, 4H), 8.60 (s, 1H).

Example 109

Ethyl 2-methyl-4-{N-ethyl-N-[(2'-[1H-tetrazol-5-yl]biphenyl-4-yl)methyl]amino}pyrimidine-5-carboxylate

Example 109A

N-Triphenylmethyl-5-[2-(4'-ethylaminomethyl-biphenyl)]tetrazole

Following the procedure described in Example 96A, N-triphenylmethyl-5-[2-(4'-bromomethyl-biphenyl)]-tetrazole (3.00 g, 5.4 mmol) in tetrahydrofuran (50 mL) was treated with 70% ethylamine in water (5 mL). Normal work up afforded the title compound as an amorphous solid.

Example 109B

Ethyl 2-methyl-4-{N-ethyl-N-[(2'-[N-triphenylmethyl-1H-tetrazol-5-yl]biphenyl-4-yl)methyl]amino}pyrimidine-5-carboxylate

Following the procedure described in Example 96B, the compound resulting from Example 109A was dissolved in tetrahydrofuran (25 mL) and treated with N-methylmorpholine (2 mL) and ethyl 2-methyl-4-chloropyrimidine-5-carboxylate (914 mg). After stirring overnight at ambient temperature, normal work up and chromatography eluting with ethyl acetate and toluene mixtures provided the title compound as a light yellow semi-solid (2.00 g, 67%).

Example 109C

Ethyl 2-methyl-4-{N-ethyl-N-[(2'-[1H-tetrazol-5-yl]biphenyl-4-yl)methyl]amino}pyrimidine-5-carboxylate

Following the procedure described in Example 100C, the compound resulting from Example 109B (1.25 g, 1.8 mmol) was deblocked using acetic acid (20 mL) and water (2 mL) in refluxing tetrahydrofuran (20 mL) for 3.5 hours. Normal work up afforded a crude product which was triturated with hexane to afford a partially purified product. Recrystallization from methylene chloride/methylcyclohexane afforded the title compound as an amorphous solid (450 mg, 56%). $^1$H NMR ($CDCl_3$, 300 MHz) δ 1.14 (t, J = 7.5Hz, 3H), 1.32 (t, J = 7.5Hz, 3H), 2.25 (s, 3H), 3.40 (q, J = 7.5Hz, 2H), 4.28 (q, J = 7.5Hz, 2H), 4.72 (s, 2H), 7.01 (d, J = 9Hz, 2H), 7.06 (d, J = 9Hz, 2H), 7.46 (dd, J = 7.5Hz, 1Hz, 1H), 7.50-7.62 (m, 2H), 8.02 (dd, J = 7.5Hz, 1Hz, 1H), 8.06 (s, 1H).

Example 110

2-Methyl-4-{N-ethyl-N-[(2'-[1H-tetrazol-5-yl]biphenyl-4-yl)methyl]amino}pyrimidine-5-carboxylic acid

Following the procedure described in Example 98, the compound resulting from Example 109 (250 mg, 0.56 mmol) was hydrolyzed with sodium hydroxide (225 mg, 5.6 mmol). Normal work up afforded an off-white solid which was dissolved in ethanol (5 mL) and ether (200 mL) was added. Filtration afforded the title compound as an amorphous solid (36 mg, 15%). $^1$H NMR (DMSO-$d_6$, 300 MHz) δ 1.10 (t, J = 7.5Hz, 3H), 2.25 (s, 3H), 3.45 (q, J = 7.5Hz, 2H), 4.87 (s, 2H), 7.05 (d, J = 9Hz, 2H), 7.21 (d, J = 9Hz, 2H), 7.52-7.70 (m, 4H), 8.50 (s, 1H).

Example 111

Ethyl 4-{N-[(2'-[1H-tetrazol-5-yl]biphenyl-4-yl)methyl]amino}pyrimidine-5-carboxylate

Example 111A

Ethyl 4-{N-[(2'-[N-triphenylmethyl-1H-tetrazol-5-yl]biphenyl-4-yl)methyl]amino}pyrimidine-5-carboxylate

The compound resulting from Example 1B (7.2 mmol) and ethyl 2-methyl-4-chloropyrimidine-5-carboxylate (1.35 g, 7.2 mmol) were reacted by the method described in Example 96B to afford crude product. Chromatography eluting with ethyl acetate/toluene mixtures provided the title compund as an amorphous solid (3.00 g, 81%).

Example 111B

Ethyl 4-{N-[(2'-[1H-tetrazol-5-yl]biphenyl-4-yl)methyl]amino}pyrimidine-5-carboxylate

Following the procedure described in Example 100C, the compound resulting from Example 111A (1.5 g, 2.3 mmol) was treated with acetic acid (15 mL) and water (2 mL) in tetrahydrofuran (15 mL). Normal work up afforded a colorless solid. Recrystallization from ethyl acetate/hexane followed by methylene chloride/hexane provided the title compound as an amorphous solid (670 mg, 62%). $^1H$ NMR (DMSO-$d_6$, 300 MHz) $\delta$ 1.32 (t, J = 7Hz, 3H), 4.33 (q, J = 7Hz, 2H), 4.72 (d, J = 6Hz, 2H), 7.04 (d, J = 8Hz, 2H), 7.25 (d, J = 8Hz, 2H), 7.50-7.70 (m, 4H), 8.60 (s, 1H), 8.70 (bt, J = 6Hz, 1H), 8.77 (s, 1H).

Example 112

4-{N-[(2'-[1H-tetrazol-5-yl]biphenyl-4-yl)methyl]amino}pyrimidine-5-carboxylic acid

Following the procedure described in Example 98, the compound resulting from Example 111 (300 mg, 0.75 mmol) was hydrolyzed using sodium hydroxide. The crude reaction mixture was dissolved and acidified with acetic acid. The product was filtered and redissolved in warm methanol/ethanol and filtered. The filtrate was concentrated under reduced pressure to afford the title compound as an amorphous solid (245 mg, 88%). $^1H$ NMR (DMSO-$d_6$, 300 MHz) $\delta$ 4.73 (d, J = 6Hz, 2H), 7.05 (d, J = 9Hz, 2H), 7.25 (d, J = 9Hz, 2H), 7.52-7.70 (m, 4H), 8.60 (s, 1H), 8.72 (s, 1H), 8.90 (bt, J = 6Hz, 1H).

Example 113

Ethyl 2-trifluoromethyl-4-{N-propyl-N-[(2'-[1H-tetrazol-5-yl]biphenyl-4-yl)methyl]amino}pyrimidine-5-carboxylate

Example 113A

Ethyl 2-trifluoromethyl-4-{N- propyl-N-[(2'-[N-triphenylmethyl-1H-tetrazol-5-yl]biphenyl-4-yl)methyl]-amino}pyrimidine-5-carboxylate

Following the procedure described in Example 96B, the compound resulting from Example 96A (5 mmol) was treated with ethyl 2-trifluoromethyl-4-chloropyrimidine-5-carboxylate (1.3 g, 5 mmol), prepared according to Barane *et al. J. Org. Chem.* **1959**, *24*: 198. Normal work up and chromatography using ethyl acetate/hexane mixtures provided an off-white foam. Recrystallization from ethyl acetate/hexane provided the title compound as colorless needles (2.2 g, 70%).

Example 113B

Ethyl 2-trifluoromethyl-4-{N-propyl-N-[(2'-[1H-tetrazol-5-yl]biphenyl-4-yl)methyl]amino}pyrimidine-5-carboxylate

Following the procedure described in Example 100, the compound resulting from Example 113A was deblocked using acetic acid (20 mL) and water (2 mL) in refluxing tetrahydrofuran for 2 hours. Chromatography eluting with ethanol in methylene chloride provided a partially purified product. Recrystallization from methylcyclohexane provided the title compound as an amorphous solid (590 mg, 68%). $^1H$ NMR ($CDCl_3$,

300 MHz) δ 0.93 (t, J = 7.5Hz, 3H), 1.44 (t, J = 7.5Hz, 3H), 1.72 (m, 2H), 3.60 (t, J = 7.5Hz, 2H), 4.31 (q, J = 7.5Hz, 2H), 4.90 (s, 2H), 7.17-7.20 (m, 4H), 7.42 (dd, J = 7Hz, 1Hz, 1H), 7.52-7.63 (m, 2H), 8.20 (dd, J = 7Hz, 1Hz, 1H), 8.60 (s, 1H).

Example 114

2-Trifluoromethyl-4-{N-propyl-N-[(2'-[1H-tetrazol-5-yl]biphenyl-4-yl)methyl]amino}pyrimidine-5-carboxylic acid

Following the procedure described in Example 98, the compound resulting from Example 113 (350 mg, 0.69 mmol) was suspended in water (10 mL). Solid sodium hydroxide (2.3 g) was added followed by ethanol (4 mL). After stirring at ambient temperature for 1 hour, the reaction was worked up. Chromatography of the residue using water, acetic acid and ethyl acetate mixtures provided a partially purified product. The residue was dissolved in ethyl acetate and hexane was added. Filtration afforded the title compound as an amorphous solid (230 mg, 67%). $^1$H NMR (DMSO-$d_6$, 300 MHz) δ 0.78 (t, J = 8Hz, 3H), 1.50-1.61 (m, 2H), 3.40 (m, 2H), 4.84 (m, 2H), 7.05 (d, J = 9Hz, 2H), 7.23 (d, J = 9Hz, 2H), 7.51-7.70 (m, 4H), 8.62 (s, 1H).

Example 115

Ethyl 2-trifluoromethyl-4-{N-butyl-N-[(2'-[1H-tetrazol-5-yl]biphenyl-4-yl)methyl]amino}pyrimidine-5-carboxylate

Example 115A

Ethyl 2-trifluoromethyl-4-{N-butyl-N-[(2'-[N-triphenylmethyl-1H-tetrazol-5-yl]biphenyl-4-yl)methyl]-amino}pyrimidine-5-carboxylate

Following the procedure described in Example 96B, the compound resulting from Example 19 (5.6 mmol) was reacted with ethyl 2-trifluoromethyl-4-chloropyrimidine-5-carboxylate (1.1 g, 5.9 mmol). Normal work up and chromatography using ethyl acetate/hexane mixtures afforded the title compound as an amorphous solid (3.1 g, 72%).

Example 115B

Ethyl 2-trifluoromethyl-4-{N-butyl-N-[(2'-[1H-tetrazol-5-yl]biphenyl-4-yl)methyl]amino}pyrimidine-5-carboxylate

Following the procedure described in Example 100, the compound resulting from Example 115A (3.1 g, 4 mmol) was deblocked using acetic acid (50 mL) and water (3 mL) in tetrahydrofuran (50 mL) at reflux for 2 hours. Chromatography using ethanol in methylene chloride containing acetic acid provided a substantially purified product. Chromatography using ethanol in methylene chloride mixtures provided the title compound as an amorphous solid (900 mg, 42%). $^1$H NMR ($CDCl_3$, 300 MHz) δ 0.95 (t, J = 7Hz, 3H), 1.33 (m, 2H), 1.34 (t, J = 6Hz, 3H), 1.67 (m, 2H), 3.65 (t, J = 8Hz, 2H), 4.30 (t, J = 7Hz, 3H), 4.88 (s, 2H), 7.20 (m, 4H), 7.40-7.65 (m, 3H), 8.20 (bd, J = 8Hz, 1H), 8.60 (s, 1H).

Example 116

2-Trifluoromethyl-4-{N-butyl-N-[(2'-[1H-tetrazol-5-yl]biphenyl-4-yl)methyl]amino}pyrimidine-5-carboxylic acid

Following the procedure described in Example 98, the compound resulting from Example 115 (850 mg, 1.6 mmol) was hydrolyzed using sodium hydroxide (850 mg). Normal work up afforded a colorless solid. The crude product was dissolved in ether and hexane was added. The solid was collected by filtration and the process was repeated. Filtration afforded the title compound as an amorphous solid (600 mg, 75%). $^1$H NMR (DMSO-$d_6$, 300 MHz) δ 0.83 (t, J = 7.5Hz, 3H), 1.20 (m, 2H), 1.52 (m, 2H), 3.45 (bt, J = 7.5Hz, 2H), 4.82 (s, 2H), 7.05 (d, J = 8Hz, 2H), 7.23 (d, J = 8Hz, 2H), 7.51-7.70 (m, 4H), 8.62 (s, 1H).

Example 117

Ethyl 1-{N-butyl-N-[(2'-[1H-tetrazol-5-yl]biphenyl-4-yl)methyl]amino}benzene-2-carboxylate

Example 117A

Ethyl 1-(N-butylamino)benzene-2-carboxylate

A mixture of ethyl anthranilate (4.96 g, 30 mmol), potassium carbonate (13.8 g, 100 mmol), and n-butyl iodide (25 mL) was stirred at ambient temperature for 48 hours and refluxed for 8 hours. The cooled reaction mixture was diluted with ethyl acetate and filtered. The filtrate was concentrated under reduced pressure and the residue obtained chromatographed eluting with ethyl acetate/hexane mixtures to afford the title comound as a light yellow liquid (2.2 g, 33%).

Example 117B

Ethyl 1-{N-butyl-N-[(2'-[N-triphenylmethyl-1H-tetrazol-5-yl]biphenyl-4-yl)methyl]amino}benzene-2-carboxylate

The compound resulting from Example 117A (2.00 g, 9 mmol), potassium carbonate (1.38 g, 10 mmol), and N-triphenylmethyl-5-[2-(4'-bromomethyl-biphenyl)]tetrazole (2.00 g, 3 mmol) in anhydrous dimethylformamide (3 mL) were stirred at 55 °C for 20 hours. The cooled reaction mixture was poured into ethyl acetate and washed with saturated brine, dried over sodium carbonate and sodium sulfate, and concentrated under reduced pressure. Flash chromatography eluting with ethyl acetate/hexane mixtures provided the title compound as an amorphous solid (800 mg, 46%).

Example 117C

Ethyl 1-{N-butyl-N-[(2'-[1H-tetrazol-5-yl]biphenyl-4-yl)methyl]amino}benzene-2-carboxylate

The compound resulting from Example 117A (790 mg, 1.2 mmol) was deblocked following the procedure described in Example 100C. Chromatography eluting with ethanol in methylene chloride provided the title compound as an amorphous solid (370 mg, 70%). $^1H$ NMR ($CDCl_3$, 300 MHz) $\delta$ 0.91 (t, J = 7.5Hz, 3H), 1.36 (t, J = 7Hz, 3H), 1.40 (m, 2H), 1.58 (m, 2H), 3.15 (t, J = 7.5 Hz, 2H), 4.07 (s, 2H), 4.25 (q, J = 7.5Hz, 2H), 6.97-7.17 (m, 5H), 7.32-7.61 (m, 6H), 8.21 (dd, J = 7Hz, 2Hz, 1H).

Example 118

1-{N-Butyl-N-[(2'-[1H-tetrazol-5-yl]biphenyl-4-yl)methyl]amino}benzene-2-carboxylic acid

The compound resulting from Example 117 (300 mg, 0.66 mmol) was hydrolyzed following the procedure described in Example 98. The crude product was dissolved in methylene chloride and hexane was added. Filtration afforded the title compound as an amorphous solid (230 mg, 79%). $^1H$ NMR (DMSO-$d_6$, 300 MHz) $\delta$ 0.75 (t, J = 7.5Hz, 3H), 1.10-1.28 (m, 2H), 3.07 (t, J = 7Hz, 2H), 4.27 (s, 2H), 7.05 (d, J = 9Hz, 2H), 7.22 (d, J = 9Hz, 2H), 7.40-7.55 (m, 2H), 7.95 (dd, J = 9Hz, 2Hz, 1H).

Example 119

1-(Ethyloxycarbonyloxy)ethyl 4-{N-butyl-N-[(2'-[1H-tetrazol-5-yl]biphenyl-4-yl)methyl]amino}pyrimidine-5-carboxylate

Example 119A

4-{N-butyl-N-[(2'-[N-triphenylmethyl-1H-tetrazol-5-yl]biphenyl-4-yl)methyl]amino}pyrimidine-5-carboxylic acid

The compound resulting from Example 83A (3.25 g, 4.6 mmol) was dissolved in a 2:3 mixture of ethanol/tetrahydrofuran (50 mL). To this was added a solution of sodium hydroxide (3.25 g) in water (8 mL). After stirring at ambient temperature for 48 hours, an additional aliquot of sodium hydroxide (1.00 g) was added and stirring was continued for an additional 24 hours. The reaction mixture was concentrated under

reduced pressure and the residue obtained suspended in water (100 mL). Concentrated hydrochloric acid was added and the suspension was extracted with ethyl acetate. The combined organic extracts were dried over sodium sulfate and concentrated under reduced pressure to afford the title compound as an amorphous solid (3.00 g, 98%).

Example 119B

1-(Ethyloxycarbonyloxy)ethyl 4-{N-butyl-N-[(2'-[N-triphenylmethyl-1H-tetrazol-5-yl]biphenyl-4-yl)methyl]-amino}pyrimidine-5-carboxylate

The compound resulting from Example 119A (1.00 g, 1.5 mmol) was dissolved in anhydrous dimethylformamide (3 mL) and potassium carbonate (616 mg, 4.5 mmol), 1-chloroethyl ethyl carbonate (460 mg, 3 mmol), sodium iodide (450 mg) and triethylamine (5 drops) were added. After stirring at ambient temperature for 30 hours, the reaction mixture was poured into saturated aqueous ammonium chloride and extracted with ethyl acetate. The combined organic extracts were washed with brine, dried over sodium sulfate and sodium carbonate, and concentrated under reduced pressure. Chromatography eluting with ethyl acetate/hexane mixtures provided the title compound as an amorphous solid (470 mg, 40%).

Example 119C

1-(Ethyloxycarbonyloxy)ethyl 4-{N-butyl-N-[(2'-[1H-tetrazol-5-yl]biphenyl-4-yl)methyl]amino}pyrimidine-5-carboxylate

Following the procedure described in Example 82B, the compound resulting from Example 119B (430 mg, 0.54 mmol) was deblocked using aqueous acetic acid in refluxing tetrahydrofuran. Chromatography eluting with ethanol in methylene chloride produced an off-white amorphous solid. Recrystallization from ether/hexane afforded the title compound as an amorphous solid (119 mg, 40%). $^1$H NMR ($CDCl_3$, 300 MHz) $\delta$ 0.88 (t, J = 7.5Hz, 3H), 1.27 (m, 2H), 1.27 (t, J = 6Hz, 3H), 1.60 (m, 2H), 1.60 (d, J = 6Hz, 3H), 3.48 (m, 2H), 4.18 (m, 2H), 4.85 (s, 2H), 7.00 (q, J = 6Hz, 1H), 7.13 (d, J = 9Hz, 2H), 7.19 (d, J = 9Hz, 2H), 7.46 (dd, J = 7.5Hz, 2Hz, 1H), 7.58 (m, 2H), 8.12 (dd, J = 7.5Hz, 2Hz, 1H), 8.33 (s, 1H), 8.47 (s, 1H).

Example 120

1-(Cyclohexyloxycarbonyloxy)ethyl 4-{N-butyl-N-[(2'-[1H-tetrazol-5-yl]biphenyl-4-yl)methyl]-amino}pyrimidine-5-carboxylate

Example 120A

1-(Cyclohexyloxycarbonyloxy)ethyl 4-{N-butyl-N-[(2'-[N-triphenylmethyl-1H-tetrazol-5-yl]biphenyl-4-yl)-methyl]amino}pyrimidine-5-carboxylate

Following the procedure described in Example 119B, the compound resulting from Example 119A (1.00 g, 1.5 mmol), triethylamine (450 mg), sodium iodide (100 mg) and 1-chloroethyl cyclohexylcarbonate (775 mg, 3.8 mmol), prepared according to Yoshimura et al., *J. Antitiotics*. **1987**, Vol. XL, No. 1, 81-90, were mixed in dimethylformamide (3 mL). After stirring for 90 hours at ambient temperature, normal work up and chromatography eluting with ethyl acetate/hexane mixtures provided the title compound as an amorphous solid (510 mg, 41%).

Example 120B

1-(Cyclohexyloxycarbonyloxy)ethyl 4-{N-butyl-N-[(2'-[1H-tetrazol-5-yl]biphenyl-4-yl)methyl]-amino}pyrimidine-5-carboxylate

Following the procedure described in Example 821B, the compound resulting from Example 120A (510 mg, 0.62 mmol) was deblocked. Chromatography eluting with ethanol in methylene chloride provided a light yellow oil. Trituration with ether/hexane provided the title compound as an off-white amorphous solid (100 mg, 28%). $^1$H NMR ($CDCl_3$, 300 MHz) $\delta$ 0.88 (t, J = 7Hz, 3H), 1.20-1.93 (m, 14H), 1.60 (d, J = 6Hz, 3H), 3.48 (m, 2H), 4.60 (m, 1H), 4.85 (s, 2H), 6.88 (q, J = 6Hz, 1H), 7.13 (d, J = 9Hz, 2H), 7.17 (d, J = 9Hz,

2H), 7.42 (dd, J = 7Hz, 1Hz, 1H), 7.58 (m, 4H), 8.10 (dd, J = 7Hz 1Hz, 1H), 8.35 (s, 1H), 8.48 (s 1H).

Example 121

1-(1-Methylpiperidin-4-ylcarbonyloxy)ethyl 4-{N-butyl-N-[(2'-[1H-tetrazol-5-yl]biphenyl-4-yl)methyl]-amino}pyrimidine-5-carboxylate

Example 121A

1-(1-Methylpiperidin-4-ylcarbonyloxy)ethyl 4-{N-butyl-N-[(2'-[N-triphenylmethyl-1H-tetrazol-5-yl]biphenyl-4-yl)methyl]amino}pyrimidine-5-carboxylate

Following the procedure described in Example 119B, the compound resulting from Example 119A (1.6 g, 2.4 mmol), sodium iodide (200 mg), potassium carbonate (1.38 g) and 1-chloroethyl (1-methylpiperidin-4-yl)carbonate (1.9 g, 8.6 mmol) were reacted in anhydrous dimethylformamide (3 mL). Column chromatography eluting with ethyl acetate/pyridine/acetic acid mixtures afforded the title compound as an amorphous solid (700 mg, 34%).

Example 121B

1-(1-Methylpiperidin-4-ylcarbonyloxy)ethyl 4-{N-butyl-N-[(2'-[1H-tetrazol-5-yl]biphenyl-4-yl)methyl]-amino}pyrimidine-5-carboxylate

Following the procedure described in Example 82B, the compound resulting from Example 121A (650 mg, 0.76 mmol) was deblocked to afford the title compound as an off-white amorphous solid (250 mg, 54%). MS (FAB) m/e 615 $(M+H)^+$.

Example 122

(N,N-Diethylaminocarbonyl)methyl 4-{N-butyl-N-[(2'-[1H-tetrazol-5-yl]biphenyl-4-yl)methyl]-amino}pyrimidine-5-carboxylate

Example 122A

(N,N-Diethylaminocarbonyl)methyl 4-{N-butyl-N-[(2'-[N-triphenylmethyl-1H-tetrazol-5-yl]biphenyl-4-yl)-methyl]amino}pyrimidine-5-carboxylate

Following the procedure described in Example 119B, the compound resulting from Example 119B (1.00 g, 1.5 mmol) was reacted with 2-chloro-N,N-diethylacetamide (335 mg, 2.2 mmol) and triethylamine (3 mmol) in anhydrous dimethylformamide (3 mL). Chromatography eluting with ethyl acetate/hexane mixtures provided the title compound as an off-white amorphous solid (800 mg, 67%).

Example 122B

(N,N-Diethylaminocarbonyl)methyl 4-{N-butyl-N-[(2'-[1H-tetrazol-5-yl]biphenyl-4-yl)methyl]-amino}pyrimidine-5-carboxylate

Following the procedure described in Example 82B, the compound resulting from Example 122A (785 mg, 1 mmol) was deblocked. Chromatography eluting with ethanol in methylene chloride provided the title compound as an off-white amorphous solid (490 mg, 90%). $^1H$ NMR ($CDCl_3$, 300 MHz) δ 0.90-1.00 (m, 6H), 1.22 (t, J = 7Hz, 3H), 1.40 (m, 2H), 1.72 (m, 2H), 3.25 (m, 4H), 3.83 (t, J = 7Hz, 2H), 4.75 (s, 2H), 4.80 (s, 2H), 6.88 (d, J = 9Hz, 2H), 6.95 (d, J = 9Hz, 2H), 7.43 (dd, J = 7Hz, 2Hz, 1H), 7.48-7.60 (m, 2H), 7.90 (dd, J = 7Hz, 2Hz, 1H), 8.63 (s, 1H), 8.66 (s, 1H).

Example 123

(5-Methyl-2-oxo-1,3-dioxolen-4-yl)methyl 4-{N-butyl-N-[(2'-[1H-tetrazol-5-yl]biphenyl-4-yl)methyl]-amino}pyrimidine-5-carboxylate

Example 123A

(5-Methyl-2-oxo-1,3-dioxolen-4-yl)methyl 4-{N-butyl-N-[(2'-[N-triphenylmethyl-1H-tetrazol-5-yl]biphenyl-4-yl)-methyl]amino}pyrimidine-5-carboxylate

4-{N-Butyl-N-[(2'-[N-triphenylmethyl-1H-tetrazol-5-yl]biphenyl-4-yl)methyl]amino}pyrimidine-5-carboxylylic acid (537.8 mg, 0.80 mmol) in dimethylformamide (3 mL) was treated with 4-bromomethyl-5-methyl-2-oxo-1,3-dioxolene (340 mg, 1.8 mmol, Sakamoto, F. *et al. Chem. Pharm. Bull.* **1984**, *32*: 2241) and diisopropylethylamine (0.31 mL, 1.8 mmol). After 18 hours at ambient temperature, the mixture was diluted with ethyl acetate. The ethyl acetate solution was washed with water and brine, dried over sodium sulfate and evaporated under reduced pressure. Chromatography of the residue on silica gel eluting with 50% ethyl acetate in hexane afforded 414.3 mg (66%) of the desired product as a foam: TLC (50% ethyl acetate/50% hexane) $R_f$ = 0.18; $^1$H NMR ($CDCl_3$, 300 MHz) δ 0.84 (t, 3H), 1.25-1.10 (m, 2H), 1.60-1.45 (m, 2H), 2.13 (s, 3H), 3.38 (t, 2H), 4.66 (s, 2H), 5.40 (s, 2H), 7.55-6.87 (multiplets, total 22H), 7.93-7.87 (m, 1H), 8.62 (s, 2H).

Example 123B

(5-Methyl-2-oxo-1,3-dioxolen-4-yl)methyl 4-{N-butyl-N-[(2'-[1H-tetrazol-5-yll]biphenyl-4-yl)methyl]-amino}pyrimidine-5-carboxylate

The resultant compound from Example 123A (404.0 mg, 0.515 mmol) in 15:15:1 acetic acid/tetrahydrofuran/water (10 mL) was heated at reflux for 1 hour. The mixture was evaporated under reduced pressure and chased with several portions of toluene. Chromatography of the residue on silica gel eluting with 3-7% methanol in chloroform afforded 94.7 mg (81%) of the title compound as a foam: TLC (10% methanol/90% chloroform) $R_f$ = 0.29; $^1$H NMR ($CDCl_3$, 300 MHz) δ 0.88 (t, 3H), 1.34-1.18 (m, 2H), 1.66-1.52 (m, 2H), 2.22 (s, 3H), 3.44 (t, 2H), 4.82 (s, 2H), 5.01 (s, 2H), 7.22-7.13 (m, 4H), 7.41-7.47 (m, 1H), 7.66-7.51 (m, 2H), 8.16-8.06 (m, 1H), 8.53 (s, 1H), 8.54 (s, 1H).

Example 124

(5-*tert*-Butyl-2-oxo-1,3-dioxolen-4-yl)methyl 4-{N-butyl-N-[(2'-[1H-tetrazol-5-yl]biphenyl-4-yl)methyl]-amino}pyrimidine-5-carboxylate

Example 124A

(5-*tert*-Butyl-2-oxo-1,3-dioxolen-4-yl)methyl 4-{N-Butyl-N-[(2'-[N-triphenylmethyl-1H-tetrazol-5-yl]biphenyl-4-yl)methyl]amino}pyrimidine-5-carboxylate

Using the procedure of Example 123A and replacing 4-bromomethyl-5-methyl-2-oxo-1,3-dioxolene with 4-bromomethyl-5-*tert*-butyl-2-oxo-1,3-dioxolene (Sakamoto, F. *et al. Chem. Pharm. Bull.* **1984**, *32*: 2241) gives the title compound.

Example 124B

(5-*tert*-Butyl-2-oxo-1,3-dioxolen-4-yl)methyl 4-{N-butyl-N-[(2'-[1H-tetrazol-5-yl]biphenyl-4-yl)methyl]-amino}pyrimidine-5-carboxylate

The resultant compound from Example 124A is treated in the manner described in Example 123B to give the title compound.

Example 125

(5-Phenyl-2-oxo-1,3-dioxolen-4-yl)methyl 4-{N-butyl-N-[(2'-[1H-tetrazol-5-yl]biphenyl-4-yl)methyl]-amino}pyrimidine-5-carboxylate

Example 125A

(5-Phenyl-2-oxo-1,3-dioxolen-4-yl)methyl 4-{N-butyl-N-[(2'-[N-triphenylmethyl-1H-tetrazol-5-yl]biphenyl-4-yl)-methyl]amino}pyrimidine-5-carboxylate

Using the procedure described in Example 123A and replacing 4-bromomethyl-5-methyl-2-oxo-1,3-dioxolene with 4-bromomethyl-5-phenyl-2-oxo-1,3-dioxolene (Sakamoto, F. *et al. Chem. Pharm. Bull.* **1984**, *32*, 2241) gives the title compound.

Example 125B

(5-Phenyl-2-oxo-1,3-dioxolen-4-yl)methyl 4-{N-butyl-N-[(2'-[1H-tetrazol-5-yl]biphenyl-4-yl)methyl]-amino}pyrimidine-5-carboxylate

The resultant compound from Example 125A is treated in the manner described in Example 123B to give the title compound.

Example 126

Acetoxymethyl 4-{N-butyl-N-[(2'-[1H-tetrazol-5-yl]biphenyl-4-yl)methyl]amino}pyrimidine-5-carboxylate

Example 126A

4-{N-Butyl-N-[(2'-[1H-tetrazol-5-yl]biphenyl-4-yl)methyl]amino}pyrimidine-5-carboxylic acid

To the compound resulting from Example 52A (1.44 g, 2.06 mmol) dissolved in 25 mL of tetrahydrofuran and 25 mL of absolute ethanol was added a solution of 2.7 g of sodium hydroxide dissolved in 5 mL of water. The reaction mixture was stirred at ambient temperature for 24 hours. The solvents were removed under reduced pressure, and 50 mL of water was added. The resulting aqueous solution was acidified to pH ~3 and extracted with ethyl acetate (2 x 75 mL). The combined organic extracts were dried over magnesium sulfate and concentrated *in vacuo* to give the title compound (1.28 g, 93%).

Example 126B

Acetoxymethyl 4-{N-butyl-N-[(2'-[N-triphenylmethyl-1H-tetrazol-5-yl]biphenyl-4-yl)methyl]amino}pyrimidine-5-carboxylate

To the compound resulting from Example 126A (950 mg, 1.41 mmol) dissolved in 3 mL of dimethylformamaide was added chloromethyl acetate (310 mg, 2.83 mmol), prepared as described by L.N. Ulich and R. Adams in *J. Am. Chem. Soc.*, **1921**, *43*: 660, and triethylamine (400 µL, 2.83 mmol). The reaction was stirred at ambient temperature for 18 hours and then partitioned between ethyl acetate (200 mL) and 2:1 water/brine (100 mL). The organic phase was washed with 2:1 water/brine (100 mL) and brine (50 mL), dried over magnesium sulfate and concentrated *in vacuo*. Chromatography on silica gel eluting with 2:1 hexane/ethyl acetate afforded 300 mg (29%) of the title compound. $^1H$ NMR ($CDCl_3$, 300 MHz) δ 0.85 (t, J = 7Hz, 3H), 1.19 (m, 2H), 1.52 (m, 2H), 2.08 (s, 3H), 3.35 (t, J = 7Hz, 2H), 4.67 (s, 2H), 5.88 (s, 2H), 6.91 (m, 6H), 6.95 (d, J = 8Hz, 2H), 7.09 (d, J = 8Hz, 2H), 7.20-7.35 (m, 9H), 7.38 (dd, 1H), 7.44 (dt, 1H), 7.49 (dt, 1H), 7.92 (dd, 1H), 8.60 (s, 1H), 8.69 (s, 1H).

Example 126C

Acetoxymethyl 4-{N-butyl-N-[(2'-[1H-tetrazol-5-yl]biphenyl-4-yl)methyl]amino}pyrimidine-5-carboxylate

To the compound resulting from Example 126B (300 mg, 0.40 mmol) dissolved in 7 mL of tetrahydrofuran was added a solution of 560 µL of water in 7 mL of acetic acid. The reaction was heated at 95 °C for 1 hour, cooled to ambient temperature, and then concentrated *in vacuo* and chased with toluene (2 x 50 mL). The resulting residue was chromatographed on silica gel eluting with a gradient (3%,5%,7%,10%) of ethanol in methylene chloride to provide the title compound (100 mg, 50%) as an amorphous solid. m.p. 65-69 °C. $^1H$ NMR ($CDCl_3$, 300 MHz) δ 0.90 (t, J = 7Hz, 3H), 1.27 (m, 2H), 1.61 (m, 2H), 2.12 (s, 3H), 3.46 (t, J = 7Hz, 2H), 4.83 (s, 2H), 5.83 (s, 2H), 7.17 (dd, 4H), 7.45 (dd, 1H), 7.52-7.64 (m, 2H), 8.17 (dd, 1H), 8.52 (bs, 2H).

Example 127

1-(Benzoyloxy)ethyl 4-{N-butyl-N-[(2'-[1H-tetrazol-5-yl]biphenyl-4-yl)methyl]amino}pyrimidine-5-carboxylate

Example 127A

1-(Benzoyloxy)ethyl 4-{N-butyl-N-[(2'-[N-triphenylmethyl-1H-tetrazol-5-yl]biphenyl-4-yl)methyl]-amino}pyrimidine-5-carboxylate

By the procedure described in Example 126B, the compound resulting from Example 126A (1.00 g, 1.49 mmol) was reacted with 1-chloroethyl benzoate (1.10 g, 5.96 mmol), prepared as described by L.N. Ulich and R. Adams in *J. Am. Chem. Soc.*, **1921**, *43*: 660, to give the title compound (800 mg, 66%). $^1$H NMR ($CDCl_3$, 300 MHz) $\delta$ 0.80 (t, J = 7Hz, 3H), 1.15 (m, 2H), 1.50 (m, 2H), 1.68 (s, J = 6Hz, 3H), 3.22-3.48 (m, 2H), 4.64 (d, J = 16Hz, 1H), 4.73 (d, J = 16Hz, 1H), 6.89 (m, 6H), 6.92 (d, J = 8Hz, 2H), 7.04 (d, J = 8Hz, 2H), 7.19-7.59 (m, 16H), 7.92 (dd, 1H), 8.02 (m, 2H), 8.58 (s, 1H), 8.67 (s, 1H).

Example 127B

1-(Benzoyloxy)ethyl 4-{N-butyl-N-[(2'-[1H-tetrazol-5-yl]biphenyl-4-yl)methyl]amino}pyrimidine-5-carboxylate

The compound resulting from Example 127A (800 mg, 0.98 mmol) was treated with 25 mL of tetrahydrofuran, 2 mL of water, and 25 mL of acetic acid by the procedure described in Example 126C to give, after chromatography, the title compound (370 mg, 66%) as an amorphous solid. m.p. 79-88 °C. $^1$H NMR ($CDCl_3$, 300 MHz) $\delta$ 0.82 (t, J = 7Hz, 3H), 1.20 (m, 2H), 1.53 (m, 2H), 1.70 (d, J = 6Hz, 3H), 3.41 (m, 2H), 4.78 (d, J = 2Hz, 2H), 7.07 (dd, 4H), 7.28 (q, J = 6Hz, 1H), 7.37-7.46 (m, 3H), 7.49-7.62 (m, 3H), 7.99 (dd, 2H), 8.04 (dd, 1H), 8.20 (s, 1H), 8.41 (s, 1H).

Example 128

1-(*tert*-Butylcarbonyloxy)ethyl 4-{N-butyl-N-[(2'-[1H-tetrazol-5-yl]biphenyl-4-yl)methyl]amino}pyrimidine-5-carboxylate

Example 128A

$\alpha$-Chloroethyl trimethylacetate

Acetaldehyde (5.6 mL, 0.10 mmol) was added to a flask containing trimethylacetyl chloride (12.3 mL, 0.10 mmol) and a catalytic amount of zinc chloride. After heating at 90 °C for 2 hours, the reaction was cooled to ambient temperature and diluted with 200 mL of ether. This solution was washed with saturated sodium carbonate solution, dried over calcium chloride and distilled (b.p. 53-57 °C at 24 torr) to provide the title compound as a colorless liquid. $^1$H NMR ($CDCl_3$, 300 MHz) $\delta$ 1.22 (s, 9H), 1.79 (d, J = 6Hz, 3H), 6.54 (q, J = 6Hz, 1H).

Example 128B

1-*tert*-Butylcarbonyloxy)ethyl 4-{N-butyl-N-[(2'-[N-triphenylmethyl-1H-tetrazol-5-yl]biphenyl-4-yl)methyl]-amino}pyrimidine-5-carboxylate

The compound resulting from Example 128A (1.00 g, 1.49 mmol) was reacted with $\alpha$-chloroethyl trimethylacetate (910 mg, 5.95 mmol) by the procedure described in Example 126B to give, after chromatography on silica gel eluting with 2:1 hexane/ethyl acetate, the title compound (760 mg, 64%). $^1$H NMR ($CDCl_3$, 300 MHz) $\delta$ 0.84 (t, J = 7Hz, 3H), 1.11-1.29 (m, 2H), 1.18 (s, 9H), 1.44-1.57 (m, 2H), 1.52 (d, J = 6Hz, 3H), 3.21-3.49 (m, 2H), 4.65 (d, J = 15Hz, 1H), 4.73 (d, J = 15Hz, 1H), 6.90 (m, 6H), 6.94 (d, J =8Hz, 2H), 7.00 (q, J = 6Hz, 1H), 7.07 (d, J = 8Hz, 2H), 7.20-7.40 (m, 10H), 7.41-7.53 (m, 2H), 7.92 (dd, 1H), 8.59 (s, 1H), 8.62 (s, 1H).

Example 128C

1-(*tert*-Butylcarbonyloxy)ethyl 4-{N-butyl-N-[(2'-[1H-tetrazol-5-yl]biphenyl-4-yl)methyl]amino}pyrimidine-5-carboxylate

The compound resulting from Example 128B (760 mg, 0.95 mmol) was treated with 25 mL of tetrahydrofuran, 2 mL of water, and 25 mL of acetic acid by the procedure described in Example 126C to give, after chromatography, the title compound (380 mg, 72%) as an amorphous solid. m.p. 71-85 °C. $^1H$ NMR ($CDCl_3$, 300 MHz) δ 0.86 (t, J = 7Hz, 3H), 1.17 (s, 9H), 1.22 (m, 2H), 1.49-1.60 (m, 2H), 1.55 (d, J = 6Hz, 3H), 3.40 (m, 2H), 4.81 (d, J = 2Hz, 2H), 6.97 (q, J = 6Hz, 1H), 7.13 (dd, 4H), 7.45 (dd, 1H), 7.54 (dt, 1H), 7.61 (dt, 1H), 8.03 (dd, 1H), 8.12 (s, 1H), 8.36 (s, 1H).

Example 129

Methoxycarbonylmethyl 4-{N-butyl-N-[(2'-[1H-tetrazol-5-yl]biphenyl-4-yl)methyl]amino}pyrimidine-5-carboxylate

Example 129A

Methoxycarbonylmethyl 4-{N-butyl-N-[(2'-[N-triphenylmethyl-1H-tetrazol-5-yl]biphenyl-4-yl)methyl]-amino}pyrimidine-5-carboxylate

The compound resulting from Example 126A (1.28 g, 1.91 mmol) was reacted with methyl bromoacetate (270 µL, 2.85 mmol) by the procedure described in Example 126B to give, after chromatography on silica gel eluting with 2:1 hexane/ethyl acetate, the title compound (800 mg, 56%). $^1H$ NMR ($CDCl_3$, 300 MHz) δ 0.84 (t, J = 7Hz, 3H), 1.18 (m, 2H), 1.53 (m, 2H), 3.36 (t, J = 7Hz, 2H), 3.74 (s, 3H), 4.69 (s, 2H), 4.72 (s, 2H), 6.91 (m, 6H), 6.96 (d, J = 8Hz, 2H), 7.08 (d, J = 8Hz, 2H), 7.21-7.34 (m, 9H), 7.37 (dd, 1H), 7.44 (dt, 1H), 7.49 (dt, 1H), 7.91 (dd, 1H), 8.60 (s, 1H), 8.76 (s, 1H).

Example 129B

Methoxycarbonylmethyl 4-{N-butyl-N-[(2'-[1H-tetrazol-5-yl]biphenyl-4-yl)methyl]amino}pyrimidine-5-carboxylate

The compound resulting from Example 129A (780 mg, 1.05 mmol) was treated with 25 mL of tetrahydrofuran, 2 mL of water, and 25 mL of acetic acid by the procedure described in Example 126C to give, after chromatography, the title compound (370 mg, 70%) as an amorphous solid. m.p. 65-80 °C. $^1H$ NMR ($CDCl_3$, 300 MHz) δ 0.89 (t, J = 7Hz, 3H), 1.28 (m, 2H), 1.62 (m, 2H), 1.53 (t, J = 7Hz, 2H), 3.75 (s, 3H), 4.74 (s, 2H), 4.82 (s, 2H), 7.09 (dt, 1H), 8.02 (dd, 1H), 8.32 (bs, 1H), 8.53 (bs, 1H).

In addition, the following compounds can be prepared according to the methods outlined above:
2-{N-Butyl-N-[(2'-[1H-tetrazol-5-yl]biphenyl-4-yl)methyl]amino}pyridine-3-carboxylic acid;
2-{N-Ethyl-N-[(2'-[1H-tetrazol-5-yl]biphenyl-4-yl)methyl]amino}pyridine-3-carboxylic acid;
2-{N-Allyl-N-[(2'-[1H-tetrazol-5-yl]biphenyl-4-yl)methyl]amino}pyridine-3-carboxylic acid;
2-{N-(2-Propynyl)-N-[(2'-[1H-tetrazol-5-yl]biphenyl-4-yl)methyl]amino}pyridine-3-carboxylic acid;
2-{N-Cyclopropylmethyl-N-[(2'-[1H-tetrazol-5-yl]biphenyl-4-yl)methyl]amino}pyridine-3-carboxylic acid;
2-{N-(2-Butyl)-N-[(2'-[1H-tetrazol-5-yl]biphenyl-4-yl)methyl]amino}pyridine-3-carboxylic acid;
2-{N-(3,3,3-Trifluoropropyl)-N-[(2'-[1H-tetrazol-5-yl]biphenyl-4-yl)methyl]amino}pyridine-3-carboxylic acid;
2-{N-(3-Methylbutyl)-N-[(2'-[1H-tetrazol-5-yl]biphenyl-4-yl)methyl]amino}pyridine-3-carboxylic acid;
2-{N-(3,3-Dimethylbutyl)-N-[(2'-[1H-tetrazol-5-yl]biphenyl-4-yl)methyl]amino}pyridine-3-carboxylic acid;
4-Methyl-2-{N-propyl-N-[(2'-[1H-tetrazol-5-yl]biphenyl-4-yl)methyl]amino}pyridine-3-carboxylic acid;
4-Trifluoromethyl-2-{N-propyl-N-[(2'-[1H-tetrazol-5-yl]biphenyl-4-yl)methyl]amino}pyridine-3-carboxylic acid;
6-Methyl-4-trifluoromethyl-2-{N-propyl-N-[(2'-[1H-tetrazol-5-yl]biphenyl-4-yl)methyl]amino}pyridine-3-carboxylic acid;
4,6-Dimethyl-2-{N-propyl-N-[(2'-[1H-tetrazol-5-yl]biphenyl-4-yl)methyl]amino}pyridine-3-carboxylic acid;
5-Methyl-2-{N-propyl-N-[(2'-[1H-tetrazol-5-yl]biphenyl-4-yl)methyl]amino}pyridine-3-carboxylic acid;
6-Dimethylamino-2-{N-propyl-N-[(2'-[1H-tetrazol-5-yl]biphenyl-4-yl)methyl]amino}pyridine-3-carboxylic acid;
6-Hydroxy-2-{N-propyl-N-[(2'-[1H-tetrazol-5-yl]biphenyl-4-yl)methyl]amino}pyridine-3-carboxylic acid;
3-{N-propyl-N-[(2'-[1H-tetrazol-5-yl]biphenyl-4-yl)methyl]amino}pyridine-4-carboxylic acid;
4-{N-propyl-N-[(2'-[1H-tetrazol-5-yl]biphenyl-4-yl)methyl]amino}pyridine-3-carboxylic acid;
3-{N-propyl-N-[(2'-[1H-tetrazol-5-yl]biphenyl-4-yl)methyl]amino}pyridine-2-carboxylic acid;

2-{N-propyl-N-[(2'-[1H-tetrazol-5-yl]biphenyl-4-yl)methyl]amino}pyridine-4-carboxylic acid;
2-{N-propyl-N-[(2'-[1H-tetrazol-5-yl]biphenyl-4-yl)methyl]amino}pyridine-5-carboxylic acid;
2-{N-propyl-N-[(2'-[1H-tetrazol-5-yl]biphenyl-4-yl)methyl]amino}pyridine-6-carboxylic acid;
3-Trifluoromethanesulfonamido-2-{N-propyl-N-[(2'-[1H-tetrazol-5-yl]biphenyl-4-yl)methyl]amino}pyridine;
3-Acetylamino-2-{N-propyl-N-[(2'-[1H-tetrazol-5-yl]biphenyl-4-yl)methyl]amino}pyridine;
3-Methylaminocarbonylamino-2-{N-propyl-N-[(2'-[1H-tetrazol-5-yl]biphenyl-4-yl)methyl]amino}pyridine;
3-Trifluoroacetylamino-2-{N-propyl-N-[(2'-[1H-tetrazol-5-yl]biphenyl-4-yl)methyl]amino}pyridine;
2-{N-propyl-N-[(2'-[1H-tetrazol-5-yl]biphenyl-4-yl)methyl]amino}pyridine-3-carbonitrile;
2-{N-propyl-N-[(2'-[1H-tetrazol-5-yl]biphenyl-4-yl)methyl]amino}pyridine-3-carboxamide;
3-Aminomethyl-2-{N-propyl-N-[(2'-[1H-tetrazol-5-yl]biphenyl-4-yl)methyl]amino}pyridine;
3-Methanesulfonamidomethyl-2-{N-propyl-N-[(2'-[1H-tetrazol-5-yl]biphenyl-4-yl)methyl]amino}pyridine;
3-Trifluoromethanesulfonamidomethyl-2-{N-propyl-N-[(2'-[1H-tetrazol-5-yl]biphenyl-4-yl)methyl]-amino}pyridine;
3-Acetylaminomethyl-2-{N-propyl-N-[(2'-[1H-tetrazol-5-yl]biphenyl-4-yl)methyl]amino}pyridine;
3-Methylaminocarbonylaminomethyl-2-{N-propyl-N-[(2'-[1H-tetrazol-5-yl]biphenyl-4-yl)methyl]-amino}pyridine;
3-Trifluoroacetylaminomethyl-2-{N-propyl-N-[(2'-[1H-tetrazol-5-yl]biphenyl-4-yl)methyl]amino}pyridine;
3-Trifluoromethyl-2-{N-propyl-N-[(2'-[1H-tetrazol-5-yl]biphenyl-4-yl)methyl]amino}pyridine;
2-Chloro-3-{N-propyl-N-[(2'-[1H-tetrazol-5-yl]biphenyl-4-yl)methyl]amino}pyridine;
2-Methoxy-5-{N-propyl-N-[(2'-[1H-tetrazol-5-yl]biphenyl-4-yl)methyl]amino}pyridine;
2-Phenoxy-5-{N-propyl-N-[(2'-[1H-tetrazol-5-yl]biphenyl-4-yl)methyl]amino}pyridine;
3,6-Dimethyl-2-{N-propyl-N-[(2'-[1H-tetrazol-5-yl]biphenyl-4-yl)methyl]amino}pyridine;
Trimethylacetoxymethyl 2-{N-propyl-N-[(2'-[1H-tetrazol-5-yl]biphenyl-4-yl)methyl]amino}pyridine-3-carboxylate;
Cyclohexyloxycarbonyloxymethyl 2-{N-propyl-N-[(2'-[1H-tetrazol-5-yl]biphenyl-4-yl)methyl]amino}pyridine-3-carboxylate;
Methoxycarbonylmethyl 2-{N-propyl-N-[(2'-[1H-tetrazol-5-yl]biphenyl-4-yl)methyl]amino}pyridine-3-carboxylate;
Methoxycarbonylaminomethyl 2-{N-propyl-N-[(2'-[1H-tetrazol-5-yl]biphenyl-4-yl)methyl]amino}pyridine-3-carboxylate;
Acetamidomethyl 2-{N-propyl-N-[(2'-[1H-tetrazol-5-yl]biphenyl-4-yl)methyl]amino}pyridine-3-carboxylate;
Methylaminocarbonylaminomethyl 2-{N-propyl-N-[(2'-[1H-tetrazol-5-yl]biphenyl-4-yl)methyl]amino}pyridine-3-carboxylate;
2-{N-Propyl-N-[(2'-[trifluoromethanesulfonamido]biphenyl-4-yl)methyl]amino}pyridine-3-carboxylic acid;
2-{N-Propyl-N-[(2'-[trifluoromethanesulfonamidomethyl]biphenyl-4-yl)methyl]amino}pyridine-3-carboxylic acid;
2-{N-Propyl-N-[(2'-[1H-tetrazol-5-yl]biphenyl-4-yl)methyl]amino}pyridine-3-sulfonic acid;
2-{N-Butyl-N-[(2'-[1H-tetrazol-5-yl]biphenyl-4-yl)methyl]amino}pyridine-3-sulfonic acid;
3-Sulfonamido-2-{N-propyl-N-[(2'-[1H-tetrazol-5-yl]biphenyl-4-yl)methyl]amino}pyridine;
2-{N-Propyl-N-[(2'-[1H-tetrazol-5-yl]biphenyl-4-yl)methyl]amino}pyridine-6-sulfonic acid;
6-Methyl-2-{N-propyl-N-[(2'-[1H-tetrazol-5-yl]biphenyl-4-yl)methyl]amino}pyrimidine-4-carboxylic acid;
4-{N-Propyl-N-[(2'-[1H-tetrazol-5-yl]biphenyl-4-yl)methyl]amino}-2-(2-pyridylmethylamino)-5-methylpyrimidine;
6-{N-Propyl-N-[(2'-[1H-tetrazol-5-yl]biphenyl-4-yl)methyl]amino}pyrimidine-4-carboxylic acid;
Methoxycarbonylaminomethyl 4-{N-propyl-N-[(2'-[1H-tetrazol-5-yl]biphenyl-4-yl)methyl]amino}pyrimidine-4-carboxylate;
Acetamidomethyl 4-{N-propyl-N-[(2'-[1H-tetrazol-5-yl]biphenyl-4-yl)methyl]amino}pyrimidine-4-carboxylate;
Methylaminocarbonylaminomethyl 4-{N-propyl-N-[(2'-[1H-tetrazol-5-yl]biphenyl-4-yl)methyl]-amino}pyrimidine-4-carboxylate;
2-{N-Propyl-N-[(2'-[1H-tetrazol-5-yl]biphenyl-4-yl)methyl]amino}pyrimidine-5-carboxylic acid;
3-{N-Propyl-N-[(2'-[1H-tetrazol-5-yl]biphenyl-4-yl)methyl]amino}pyrazine-2-carboxylic acid;
3-{N-Propyl-N-[(2'-[1H-tetrazol-5-yl]biphenyl-4-yl)methyl]amino}pyridazine-4-carboxylic acid;
4-{N-Propyl-N-[(2'-[1H-tetrazol-5-yl]biphenyl-4-yl)methyl]amino}pyridazine-5-carboxylic acid; and
4-{N-Propyl-N-[(2'-[1H-tetrazol-5-yl]biphenyl-4-yl)methyl]amino}pyridazine-3-carboxylic acid.

ANGIOTENSIN II RECEPTOR ASSAYS

RECEPTOR BINDING ASSAYS

The hormone angiotensin II produces biological responses such as vasoconstriction through stimulation of its receptors on cell membranes. For the purpose of identifying compounds such as angiotensin II antagonists which are capable of interacting with the angiotensin II receptor, a ligand-receptor binding assay was carried out as an initial screen. The affinity of the antagonist for the receptor is expressed as an inhibitory affinity constant (Ki) obtained by calculating the inhibitor concentration which blocks binding of the isotopically labeled ligand by 50% and converting this $IC_{50}$ value to the Ki value as described by Y.C. Cheng and W.H. Prusoff in *Biochemical Pharmacology*, 22: 3099, **1973**.

Rat liver was homogenized in pH 7.4 50 m*M* tris-HCl buffer containing 0.3 *M* sucrose, 0.2% heat-inactivated bovine serum albumin (BSA), 0.3 trypsin inhibitory units/mL of aprotinin (a protease inhibitor) and 100 μg/mL 1,10-o-phenanthroline (a protease inhibitor). The homogenized tissue was centrifuged at 48,000 X g for 30 minutes. The pellets were resuspended in the above buffer and centrifuged at 48,000 X g for 20 minutes. The resulting pellet was rehomogenized in 6.25 volumes of assay buffer. The rehomogenized tissues (in 2 mL aliquots) were frozen in liquid nitrogen and stored at -60 °C.

The binding assays were carried out as described by S.J. Fluharty and L.P. Reagan in *J. Neurochemistry*, **52**, 1393-1400 (1989). The binding reaction was initiated by adding 150 μL of thawed membranes to 100 μL of assay buffer containing $^{125}$I-SARILE (obtained from DuPont-New England Nuclear, Boston, MA with a specific activity of 2,200 Ci/mmol) and various concentrations of unlabeled test compounds, as needed.

| Buffer Component | Assay Buffer Concentration |
| --- | --- |
| tris-HCl | 50 m*M* |
| sodium chloride | 150 m*M* |
| magnesium chloride | 5 m*M* |
| aprotinin* | 0.3 trypsin inhibitory units/mL |
| 1,10-o-phenanthroline* | 100 μg/mL |
| heat-inactivated BSA* | 0.2% |

The pH of the buffer was 7.4,

* from Sigma Chemical Company, St. Louis, MO

Membrane preparations (2.66 mg/mL tissue in a final volume of 0.25 mL) were incubated for 1 h at 25 °C. The assay was terminated by diluting the reaction mixture and rinsing the assay tubes three times with wash buffer (150 m*M* sodium chloride, 5 m*M* Tris, pH 7.4) followed by vacuum filtration through glass fiber filters using a cell harvester. After filtration the filters were placed in test tubes and counted for 5 minutes in an LKB gamma counter at 76% efficiency.

Competitive studies were performed using 200-300 p*M* $^{125}$I-SARILE and 7 concentrations of cold competitor spanning at least five orders of magnitude. The Ki values were calculated from the relationship $Ki = IC_{50}([S]/K_D)$ (Y.C. Cheng and W.H. Prusoff in *Biochemical Pharmacology*, 22: 3099, **1973**) using a computerized log-logit analysis. In this expression [S] is the concentration of the isotopically labeled ligand in the assay; and $K_D$ is the affinity of the isotopically labeled ligand for the receptor. The data in Table 5 show that compounds of the invention have a very high affinity for the angiotensin receptor.

Table 5: Angiotensin II Receptor Binding

| Example Number | Ki (n*M*) |
|---|---|
| 53 | 3.54 |
| 63 | 68.1 |
| 69 | 44.8 |
| 71 | 3.28 |
| 76 | 15.3 |
| 81 | 30.2 |
| 98 | 69.7 |
| 99 | 2.78 |
| 101 | 25.5 |
| angiotensin II | 0.3 |

ANGIOTENSIN II FUNCTIONAL ASSAY: Antagonism of Contraction of Rabbit Aorta

The protocol reported by A.T Chiu and P.Timmermans (P.C. Wong, *et al. Hypertension*, **13**, 489-497 (1989)) was followed with a few modifications. Female New Zealand White rabbits weighing 2-5 kg were sedated with carbon dioxide and then sacrificed. Main abdominal aortas were removed and placed in Krebs-Henseleit buffer at room temperature.

Krebs-Henseleit buffer

| Buffer Component | m*M* Concentration |
|---|---|
| sodium chloride | 119.00 |
| potassium chloride | 4.70 |
| potassium dihydrogen phosphate | 1.20 |
| calcium chloride | 2.50 |
| sodium bicarbonate | 20.00 |
| magnesium sulfate | 1.50 |
| dextrose | 11.00 |
| EDTA* disodium calcium salt | 0.01 |

* EDTA = ethylenediamine tetraacetic acid

The buffer contained no cocaine, propanolol or steroid.

The pH of the buffer was 7.40 at 37°C when saturated with 5% carbon dioxide/95% oxygen.

The tissues were cleaned of extraneous connective tissue, cut into 3 mm rings, and suspended within a 10 mL tissue bath. All dilutions of peptide preparations were made with 0.3% aqueous BSA. The tissues were primed with 55 m*M* potassium chloride. Tissues were pre-loaded with 1 g of tension. Tension was recorded on a model 7 Grass polygraph using FT03 transducers. At the end of the equilibrium period, a control cumulative concentration-contractile response curve for angiotensin II (A II: 1 X $10^{-10}$ - $10^{-8}$ M) was

obtained. The tissue was washed several times until the baseline was reached. Forty five minutes later, test compound (antagonist) was added and the tissue was incubated for 30 minutes. The concentration-response curve for A II was then repeated in the presence of the test compound. One dose of antagonist was tested per tissue only. For single dose shift experiments a dose of 1 m*M* of test compound was used, for a full $pA_2$ experiment multiple doses were used depending upon the potency of the antagonist.

All responses to the control agonist were calculated as a percentage of the maximum response. These points in duplicate were plotted and analyzed according to standard Schild analysis (H.O. Schild, *British J Pharmacology and Chemotherapy,* **2**, 189-206 (1947). The $pA_2$ values calculated for the compounds of the invention are shown in Table 6. The $pA_2$ value is the negative logarithm of the $[A]_2$ value. $[A]_2$ is the concentration of antagonist which necessitates doubling the agonist concentration in order to achieve the agonist effect which was measured in the absence of antagonist.

The $pA_2$ value, therefore is a measure of the effectiveness of the compound as an antagonist. The data in Table 6 show that the compounds of the invention are potent antagonists at the angiotensin II receptor.

Table 6: $pA_2$ Values from Isolated Rabbit Aorta Assay

| Example | $pA_2$ |
|---|---|
| 53 | 9.90 |
| 54 | 8.27 |
| 63 | 8.30 |
| 69 | 8.38 |
| 71 | 9.68 |
| 73 | 8.26 |
| 76 | 8.81 |
| 81 | 8.50 |
| 82 | 9.48 |
| 84 | 8.00 |
| 86 | 8.15 |
| 88 | 10.1 |
| 98 | 8.52 |
| 99 | 9.60 |
| 101 | 8.73 |
| 105 | 8.85 |
| 108 | 8.16 |
| 114 | 8.79 |
| 116 | 8.70 |
| Sar,-1, Thr-8 AII (SARILE) | 9.02 |

The ability of the compounds of the invention to lower blood pressure in vivo can be demonstrated according to the method outlined below.

In Vivo Blood Pressure Lowering

When administered at a dose of 30 mg/kg to renal artery ligated rats (see Cangiano, et al., J. Pharmacol. Exp. Ther. 208 310 (1979)), the compound of Example 88 caused a reduction in blood pressure

of approximately 20% that persisted for at least four hours.

The foregoing is merely illustrative of the invention and is not intended to limit the invention to the disclosed compounds. Variations and changes which are obvious to one skilled in the art are intended to be within the scope and nature of the invention which are defined in the appended claims.

## Claims

**1.** A compound of the formula:

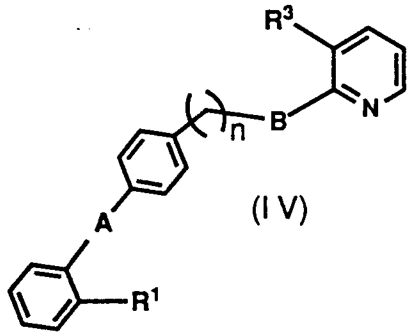

(I V)

wherein n is 0 or 1;
A is selected from
(i) a covalent bond,
(ii) -O- and
(iii) -C(O)-;
B is selected from
(i) $-N(R^4)-$ wherein $R^4$ is hydrogen, lower alkyl, loweralkoxy-substituted lower alkyl, lower alkenyl, lower alkynyl, cycloalkyl or cycloalkylalkyl,
(ii) -O- and
(iii) -S-;
$R^1$ is selected from
(i) tetrazolyl,
(ii) $-COOR^5$ or $-CH_2COOR^5$ wherein $R^5$ is hydrogen or a carboxy-protecting group; and
(iii) $-NHS(O)_2R^6$ or $-CH_2NHS(O)_2R^6$ wherein $R^6$ is selected from lower alkyl and halo-substituted lower alkyl; and
$R^3$ is selected from
(i) hydrogen,
(ii) loweralkyl,
(iii) halo-substituted loweralkyl,
(iv) -CN,
(v) $-NO_2$,
(vi) $-NH_2$,
(vii) -CHO,
(viii) tetrazolyl,
(ix) $-NHS(O)_2R^9$ or $-CH_2NHS(O)_2R^9$ or $-NHC(O)R^9$ or $-CH_2NHS(O)_2R^9$ wherein $R^9$ is selected from lower alkyl and halo-substituted lower alkyl,
(x) $-COOR^{10}$ or $-CH_2COOR^{10}$ wherein $R^{10}$ is hydrogen or a carboxy- protecting group,
(xi) $-C(O)NR^{11}R^{12}$ or $-CH_2C(O)NR^{11}R^{12}$ or $-NHC(O)NR^{11}R^{12}$ or $-CH_2NHC(O)NR^{11}R^{12}$ wherein $R^{11}$ and $R^{12}$ are independently selected from hydrogen, lower alkyl, hydroxy, lower alkoxy, hydroxy- substituted lower alkyl, loweralkoxy-substituted lower alkyl and loweralkoxy-substituted loweralkoxy, or
$R^{11}$ and $R^{12}$ taken together with the nitrogen atom to which they are attached form a 5- to 7-membered aliphatic heterocycle;
(xii) $-CH_2OR^{13}$ wherein $R^{13}$ is selected from hydrogen, lower alkyl and $-C(O)R^{14}$ wherein $R^{14}$ is hydrogen, lower alkyl or aryl;
(xiii) $-CH_2NR^{15}R^{15*}$ wherein $R^{15}$ is selected from hydrogen, lower alkyl, $-C(O)R^{16}$, $-C(O)NR^{16}R^{16*}$ and $-S(O)_2R^{17}$ wherein

$R^{16}$ is selected from hydrogen, lower alkyl and aryl and
$R^{17}$ is selected from lower alkyl and halo-substituted lower alkyl and wherein $R^{15*}$ and $R^{16*}$ are independently selected from hydrogen, loweralkyl, hydroxy and lower alkoxy;
(xiv) $-SO_3H$ or $-CH_2SO_3H$ and
(xv) $-SO_2NR^{11}R^{12}$ or $-CH_2SO_2NR^{11}R^{12}$ wherein $R^{11}$ and $R^{12}$ are defined as above;
or a pharmaceutically acceptable salt or prodrug thereof.

**2.** The compound of Claim 1 wherein $R^1$ is tetrazolyl, n is 1, A is a bond, B is $NR^4$ and $R^3$ is $-COOR^{10}$ or $-C(O)NR^{11}R^{12}$.

**3.** A compound of the formula:

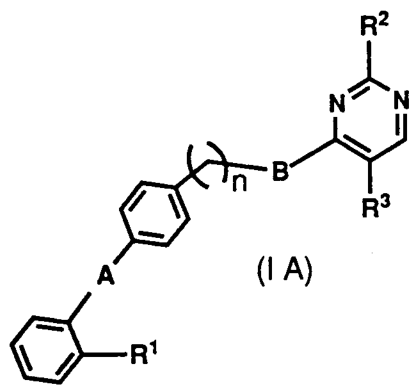

(I A)

wherein n is 0 or 1;
A is selected from
(i) a covalent bond,
(ii) -O- and
(iii) -C(O)-;
B is selected from
(i) $-N(R^4)-$ wherein $R^4$ is hydrogen, lower alkyl, loweralkoxy-substituted lower alkyl, lower alkenyl, lower alkynyl, cycloalkyl or cycloalkylalkyl,
(ii) -O- and
(iii) -S-;
$R^1$ is selected from
(i) tetrazolyl,
(ii) $-COOR^5$ or $-CH_2COOR^5$ wherein $R^5$ is hydrogen or a carboxy-protecting group; and
(iii) $-NHS(O)_2R^6$ or $-CH_2NHS(O)_2R^6$ wherein $R^6$ is selected from lower alkyl and halo-substituted lower alkyl;
$R^2$ is selected from
(i) lower alkyl,
(ii) halo,
(iii) halo-substituted loweralkyl,
(iv) lower alkylthio,
(v) loweralkoxy-substituted lower alkyl,
(vi) loweralkylthio-substituted lower alkyl,
(vii) arylalkyl,
(viii) $-OR^a$ wherein Ra is loweralkyl, halo-substituted loweralkyl or aryl and
(ix) $-NR^7R^8$ or $-CH_2NR^7R^8$ wherein $R^7$ and $R^8$ are independently selected from hydrogen and lower alkyl, or $R^7$ and $R^8$ taken together with the nitrogen atom to which they are attached form a 5- to 7-membered aliphatic heterocycle and
$R^3$ is selected from
(i) hydrogen,
(ii) loweralkyl,
(iii) halo-substituted loweralkyl,
(iv) -CN,

(v) $-NO_2$,
(vi) $-NH_2$,
(vii) -CHO,
(viii) tetrazolyl,
(ix) $-NHS(O)_2R^9$ or $-CH_2NHS(O)_2R^9$ or $-NHC(O)R^9$ or $-CH_2NHS(O)_2R^9$ wherein $R^9$ is selected from lower alkyl and halo-substituted lower alkyl,
(x) $-COOR^{10}$ or $-CH_2COOR^{10}$ wherein $R^{10}$ is hydrogen or a carboxy- protecting group,
(xi) $-C(O)NR^{11}R^{12}$ or $-CH_2C(O)NR^{11}R^{12}$ or $-NHC(O)NR^{11}R^{12}$ or $-CH_2NHC(O)NR^{11}R^{12}$ wherein $R^{11}$ and $R^{12}$ are independently selected from hydrogen, lower alkyl, hydroxy, lower alkoxy, hydroxy- substituted lower alkyl, loweralkoxy-substituted lower alkyl and loweralkoxy-substituted loweralkoxy, or $R^{11}$ and $R^{12}$ taken together with the nitrogen atom to which they are attached form a 5- to 7-membered aliphatic heterocycle;
(xii) $-CH_2OR^{13}$ wherein $R^{13}$ is selected from hydrogen, lower alkyl and $-C(O)R^{14}$ wherein $R^{14}$ is hydrogen, lower alkyl or aryl;
(xiii) $-CH_2NR^{15}R^{15*}$ wherein $R^{15}$ is selected from hydrogen, lower alkyl, $-C(O)R^{16}$, $-C(O)NR^{16}R^{16*}$ and $-S(O)_2R^{17}$ wherein
$R^{16}$ is selected from hydrogen, lower alkyl and aryl and
$R^{17}$ is selected from lower alkyl and halo-substituted lower alkyl and wherein $R^{15*}$ and $R^{16*}$ are independently selected from hydrogen, loweralkyl, hydroxy and lower alkoxy;
(xiv) $-SO_3H$ or $-CH_2SO_3H$ and
(xv) $-SO_2NR^{11}R^{12}$ or $-CH_2SO_2NR^{11}R^{12}$ wherein $R^{11}$ and $R^{12}$ are defined as above;
or a pharmaceutically acceptable salt or prodrug thereof.

**4.** The compound of Claim 3 wherein $R^1$ is tetrazolyl, n is 1, A is a bond, B is $NR^4$, $R^2$ is loweralkyl and $R^3$ is $-COOR^{10}$ or $-C(O)NR^{11}R^{12}$.

**5.** A compound selected from the group consisting of:
4-{N-butyl-N-((2'-(1H-tetrazol-5-yl)biphenyl-4-yl)methyl)amino}pyrimidine-5-carboxylic acid ;
Pivaloyloxymethyl 4-{N-butyl-N-((2'-(1H-tetrazol-5-yl)biphenyl-4-yl)methyl)amino}pyrimidine-5-carboxylate;
2-{N-Propyl-N-((2'-(1H-tetrazol-5-yl)biphenyl-4-yl)methyl)amino}pyridine-3-carboxylic acid;
1-(Ethyloxycarbonyloxy)ethyl 4-{N-butyl-N-((2'-(1H-tetrazol-5-yl)biphenyl-4-yl)methyl)amino}pyrimidine-5-carboxylate;
2-{N-Butyl-N-((2'-(1H-tetrazol-5-yl)biphenyl-4-yl)methyl)amino}pyridine-3-carboxylic acid;
2-{N-Allyl-N-((2'-(1H-tetrazol-5-yl)biphenyl-4-yl)methyl)amino}pyridine-3-carboxylic acid; and
2-{N-(3-Methylbutyl)-N-((2'-(1H-tetrazol-5-yl)biphenyl-4-yl)methyl)amino}pyridine-3-carboxylic acid;
or a pharmaceutically acceptable salt or ester thereof.

**6.** A compound having the formula:

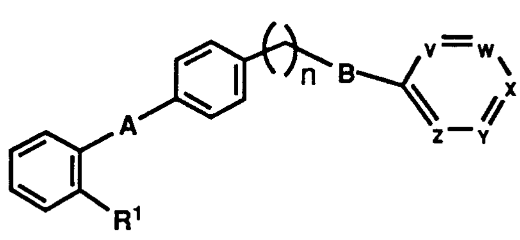
(I)

wherein n is 0 or 1;
A is selected from
(i) a covalent bond,
(ii) -O- and
(iii) -C(O)-;
B is selected from
(i) $-N(R^4)-$ wherein $R^4$ is hydrogen, lower alkyl, loweralkoxy-substituted lower alkyl, lower alkenyl, lower alkynyl, cycloalkyl or cycloalkylalkyl,
(ii) -O- and

(iii) -S-;
$R^1$ is selected from
(i) tetrazolyl,
(ii) $-COOR^5$ or $-CH_2COOR^5$ wherein $R^5$ is hydrogen or a carboxy-protecting group; and
(iii) $-NHS(O)_2R^6$ or $-CH_2NHS(O)_2R^6$ wherein $R^6$ is selected from lower alkyl and halo-substituted lower alkyl;
V, W, X, Y and Z are independently selected from N, -N(O)-, CH, $CR^2$ and $CR^3$, with the proviso that
(1) not more than one of V, W, X, Y and Z is $CR^2$,
(2) one of V, W, X, Y and Z is $CR^3$, and
(3) not more than three of V, W, X, Y and Z are N, wherein
$R^2$ is selected from
(i) lower alkyl,
(ii) halo,
(iii) halo-substituted loweralkyl,
(iv) lower alkylthio,
(v) loweralkoxy-substituted lower alkyl,
(vi) loweralkylthio-substituted lower alkyl,
(vii) arylalkyl,
(viii) $-OR^a$ wherein Ra is loweralkyl, halo-substituted loweralkyl or aryl and
(ix) $-NR^7R^8$ or $-CH_2NR^7R^8$ wherein $R^7$ and $R^8$ are independently selected from hydrogen and lower alkyl, or $R^7$ and $R^8$ taken together with the nitrogen atom to which they are attached form a 5- to 7-membered aliphatic heterocycle and
$R^3$ is selected from
(i) hydrogen,
(ii) loweralkyl,
(iii) halo-substituted loweralkyl,
(iv) -CN,
(v) $-NO_2$,
(vi) $-NH_2$,
(vii) -CHO,
(viii) tetrazolyl,
(ix) $-NHS(O)_2R^9$ or $-CH_2NHS(O)_2R^9$ or $-NHC(O)R^9$ or $-CH_2NHS(O)_2R^9$ wherein $R^9$ is selected from lower alkyl and halo-substituted lower alkyl,
(x) $-COOR^{10}$ or $-CH_2COOR^{10}$ wherein $R^{10}$ is hydrogen or a carboxy- protecting group,
(xi) $-C(O)NR^{11}R^{12}$ or $-CH_2C(O)NR^{11}R^{12}$ or $-NHC(O)NR^{11}R^{12}$ or $-CH_2NHC(O)NR^{11}R^{12}$ wherein $R^{11}$ and $R^{12}$ are independently selected from hydrogen, lower alkyl, hydroxy, lower alkoxy, hydroxy- substituted lower alkyl, loweralkoxy-substituted lower alkyl and loweralkoxy-substituted loweralkoxy, or
$R^{11}$ and $R^{12}$ taken together with the nitrogen atom to which they are attached form a 5- to 7-membered aliphatic heterocycle;
(xii) $-CH_2OR^{13}$ wherein $R^{13}$ is selected from hydrogen, lower alkyl and $-C(O)R^{14}$ wherein $R^{14}$ is hydrogen, lower alkyl or aryl;
(xiii) $-CH_2NR^{15}R^{15*}$ wherein $R^{15}$ is selected from hydrogen, lower alkyl, $-C(O)R^{16}$, $-C(O)NR^{16}R^{16*}$ and $-S(O)_2R^{17}$ wherein
$R^{16}$ is selected from hydrogen, lower alkyl and aryl and
$R^{17}$ is selected from lower alkyl and halo-substituted lower alkyl and wherein $R^{15*}$ and $R^{16*}$ are independently selected from hydrogen, loweralkyl, hydroxy and lower alkoxy;
(xiv) $-SO_3H$ or $-CH_2SO_3H$ and
(xv) $-SO_2NR^{11}R^{12}$ or $-CH_2SO_2NR^{11}R^{12}$ wherein $R^{11}$ and $R^{12}$ are defined as above;
or a pharmaceutically acceptable salt or prodrug thereof.

**7.** A pharmaceutical composition for blocking the interaction of angiotensin II with angiotensin II receptors comprising a pharmaceutically acceptable carrier and a therapeutically effective amount of a compound of claim 6.

**8.** A pharmaceutical composition for treating hypertension, edema, congestive heart failure or to prevent atherosclerosis comprising a pharmaceutically acceptable carrier and a therapeutically effective amount of a compound of claim 6.

**9.** Use of a compound of Claim 6 for manufacturing a medicament for blocking the interaction of angiotensin II with angiotensin II receptors in a human or other mammal in need.

**10.** Use of a compound of Claim 6 for manufacturing a medicament for treating hypertension, edema, congestive heart failure or to prevent atherosclerosis in a human or other mammal in need.

**11.** A process for the preparation of a compound of Claim 6 comprising reacting a compound of the formula:

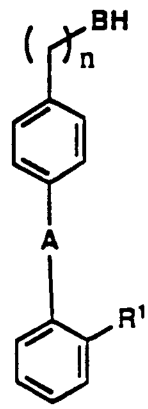

with a compound of the formula: